# EUROPEAN PATENT APPLICATION

(11) **EP 3 527 219 A1**
(43) Date of publication of application: **21.08.2019**
(21) Application number: 19151143.5
(22) Date of filing: 23.10.2013
(51) Int. Cl.: A61K 38/18, A61K 38/00, A61P 7/06

(54) **METHODS FOR TREATING ANEMIA**

(30) Priority: 24.10.2012 US 201261718128 P
(62) Divisional of application: 13849960.3
(71) Applicant: Celgene Corporation, Summit, NJ 07901 (US)
(72) Inventor: SUNG, Victoria, San Francisco, CA 94117 (US); CHOPRA, Rajesh, London SW15 1LE (GB); HERMINE, Olivier, 91120 Palaiseau (FR); CRUZ MOURA, Ivan, 75015 Paris (FR); DUSSIOT, Michael, 92320 Chatillon (FR); TROVATI MACIEL, Thiago, 75020 Paris (FR); FRICOT, Aurelie, 93100 Montreuil (FR)
(74) Representative: Jones Day

(57) **Abstract**

Provided herein is an antagonist of GDF11 for use in a method for the treatment of anemia, ineffective erythropoiesis, beta thalassemia, or for increasing Orthochromatic Erythroblasts (Ery-C) in a patient, wherein the methods comprise administration of antagonists of Growth differentiation factor 11 (GDF 11; also known as bone morphogenetic protein 11 (BMP11)) to a subject in need of the treatment.

## Description

This application claims priority to U.S. provisional patent application No. 61/718,128, filed October 24, 2012 the disclosure of which is herein incorporated by reference in its entirety.

### 1. INTRODUCTION

Provided herein are methods for the treatment of anemia, wherein the methods comprise administration of antagonists of growth differentiation factor 11 (GDF11; also known as bone morphogenetic protein 11 (BMP11)) to a subject in need of the treatment.

### 2. BACKGROUND

Anemia is a decrease in number of red blood cells or a less than normal quantity or function of hemoglobin in the blood. Anemia is the most common disorder of the blood.

Anemia can be caused by ineffective erythropoiesis. Ineffective erythropoiesis is present if active erythropoiesis takes place but mature red blood cells fail to develop at the proper rate. Progenitor cells undergo apoptosis before the stage of mature red blood cells is reached. Anemia can also involve a decreased oxygen-binding ability of the hemoglobin.

Thalassemia is a form of ineffective erythropoiesis. In thalassemia, ineffective erythropoiesis is characterized by apoptosis of the maturing nucleated erythroid cells. Specifically beta-thalassemia is a disease characterized by defects in Hemoglobin (or Hgb) synthesis leading to impaired erythrocyte maturation and production. The decrease is primarily thought to be due to abnormally accelerated erythroid differentiation and apoptosis at the late basophilic/polychromatic erythroblast stage of red blood cell differentiation, resulting in an overall decrease in mature red blood cell production. beta-thalassemia is characterized by a hypercellular bone marrow compartment where abnormal erythroblasts accumulate and undergo apoptosis, resulting in systemic anemia.

The transforming growth factor beta (TGF-beta) family, which includes TGF-beta, activins, bone morphogenic proteins (BMPs) and growth and differentiation factors (GDFs), includes secreted proteins known to regulate numerous cellular processes during both development and tissue homeostasis. TGF-beta1, activin A, BMP-2 and BMP-4 have all been associated with the regulation of erythropoiesis in various model systems. TGF-beta1 both inhibits and promotes erythroid differentiation depending on the context, activin A was shown to be a pro-erythroid differentiation agent, while BMP-4 has been implicated in stress erythropoiesis and recovery from acute anemia in murine models. BMP-2 acts on early erythroid cells to increase colony formation in samples obtained from mobilized peripheral blood or bone marrow CD34+ cells. Abnormally high levels of some of these growth factors have been associated with various hematologic diseases. For example, high levels of GDF-15 are not usually a feature of normal erythropoiesis but in conditions of ineffective erythropoiesis, GDF-15 expression is elevated.

The TGF-beta superfamily consists of more than 30 proteins and they signal through only a limited number of receptors and signaling pathways, pointing to inherent promiscuity and redundancy in their actions. Furthermore, in any given tissue, several different ligands may be found and signaling presumably occurs via an overlapping subset of receptors complicating the ability to associate particular ligands to their function. GDF11 is a member of the GDF subfamily and shares about 90% amino acid homology with GDF8, also known as myostatin. Both can bind the activin type IIA and B receptors and activate the Smad 2/3 signaling pathway. GDF11 plays a big role in development, taking part in the formation of muscle, cartilage, bone, kidney, and nervous system while in adult tissues, GDF11 was detected in the pancreas, intestine, kidney, skeletal muscle, brain and dental pulp. Low amounts of GDF-11 can be also found in the circulation. To this date, however, there is no evidence describing a role for GDF-11 in erythropoiesis.

Type II receptors for activins can also bind GDF11. Two related type II receptors, ActRIIa and ActRIIb, have been identified (Mathews and Vale, 1991, Cell 65:973-982; Attisano et al., 1992, Cell 68: 97-108). Besides GDF11, ActRIIa and ActRIIb can biochemically interact with several other TGF-beta family proteins, including BMP7, Nodal, GDF8, and activin (Yamashita et al., 1995, J. Cell Biol. 130:217-226; Lee and McPherron, 2001, Proc. Natl. Acad. Sci. 98:9306-9311; Yeo and Whitman, 2001, Mol. Cell 7: 949-957; Oh et al., 2002, Genes Dev. 16:2749-54). ALK4 is the primary type I receptor for activins, particularly for activin A, and ALK-7 may serve as a receptor for activins as well, particularly for activin B.

A humanized fusion-protein consisting of the extracellular domain (ECD) of activin-receptor type IIA (ActRIIA) and the human IgG1 Fc domain binds with high-affinity to activin-A and other TGFbeta superfamily ligands, blocking signaling through the endogenous ActRIIA-receptor. Activin-A is also known as an erythroid-differentiation-factor (EDF) affecting late stages of RBC-maturation (Murata, 1988). ActRII inhibitors have been described for increasing red blood cell levels (e.g., patent application publication Nos. 20110038831; 20100204092; 20100068215; 20100028332; 20100028331; and 20090163417).

### 3. SUMMARY

In certain embodiments, provided herein are methods for treating anemia, ineffective erythropoiesis, beta thalassemia, or increasing Orthochromatic Erythroblasts (Ery-C) in a patient, wherein the method comprises administering an antagonist of GDF11. In certain embodiments, the patient has elevated GDF11 levels in bone marrow, spleen, liver, serum, or plasma relative to a healthy individual and/or relative to the GDF11 levels in the patient prior to onset of the anemia.

In certain embodiments, if the antagonist of GDF11 is an ActRIIA polypeptide, the ActRIIA polypeptide binds preferentially to GDF11. If the antagonist of GDF11 is an ActRIIB polypeptide, the ActRIIB polypeptide binds preferentially to GDF11.

In certain embodiments, the antagonist of GDF11 reduces expression of GDF11, reduces GDF11 activity, or reduces GDF11 protein levels. In certain embodiments, the antagonist of GDF11 is an anti GDF11 antibody. In certain embodiments, the antagonist of GDF11 is a truncated receptor that binds GDF11. In certain embodiments, the antagonist of GDF11 comprises a mutated extracellular domain of ActRIIA, wherein the mutated extracellular domain of ActRIIA has an increased affinity to GDF11 relative to Activin A as compared to the wild type of ActRIIA.

In certain embodiments, a method that is provided herein further comprises monitoring GDF11 levels. In certain embodiments, the method further comprises
a. Determining GDF11 levels; and
b. Adjusting the dose of the antagonist of GDF11 wherein, if GDF11 is increased over normal, the dose of antagonist of GDF11 is increased and wherein, if GDF11 is decreased below normal, the dose of antagonist of GDF11 is decreased. In certain embodiments, the GDF11 levels are determined as GDF11 mRNA levels, GDF11 protein levels, or GDF11 protein activity levels.

In certain embodiments, administering of a GDF11 antagonist results in a decreased cell count of late basophilic and polychromatic erythroblasts in the patient. In certain embodiments, the antagonist of GDF11 reduces GDF11 expression, GDF11 activity, and/or GDF11 protein levels.

### 4. BRIEF DESCRIPTION OF THE FIGURES

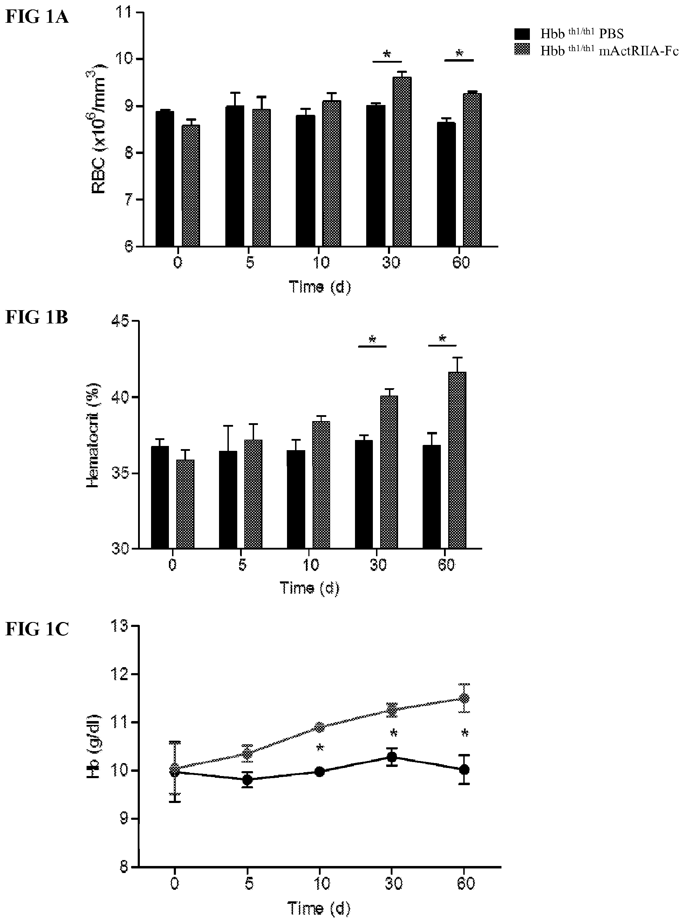

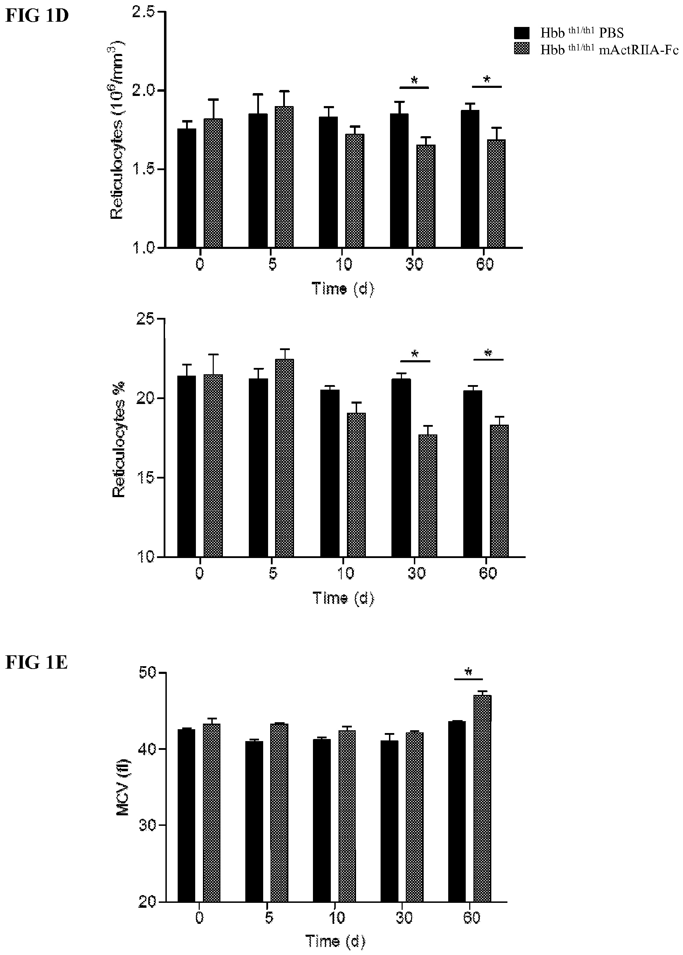

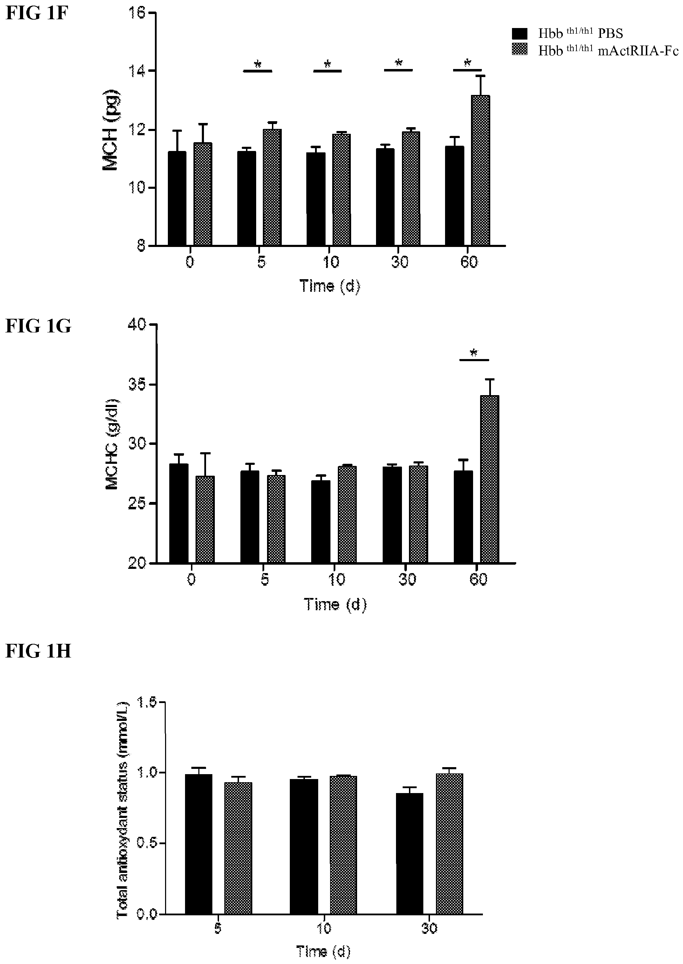

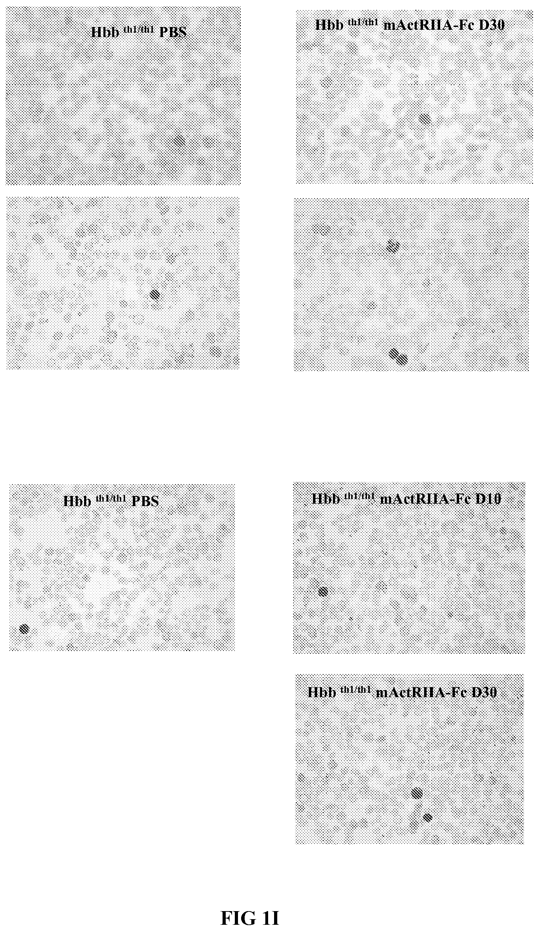

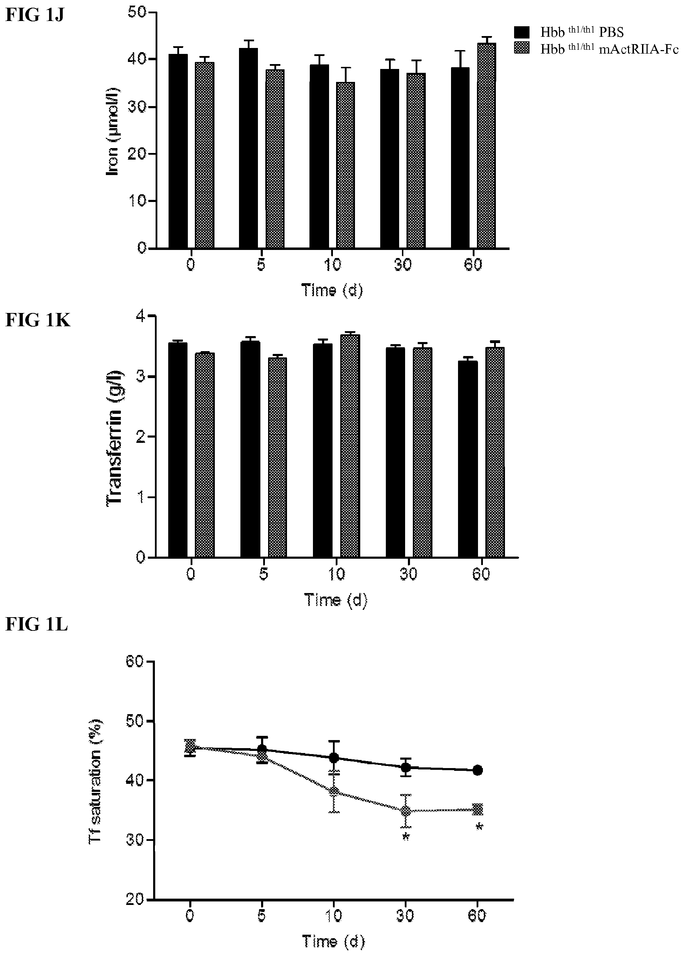

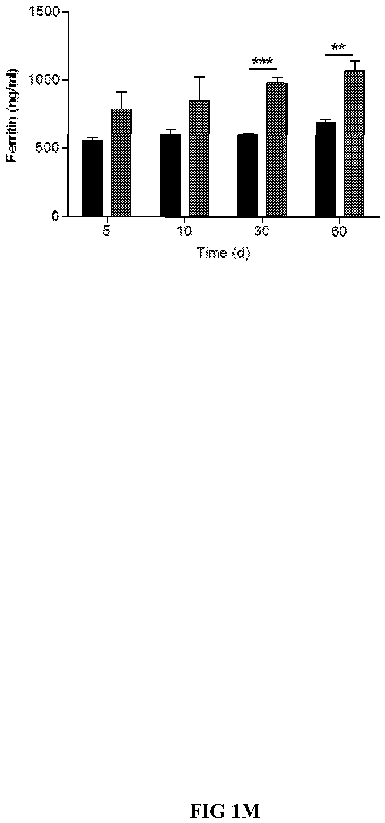

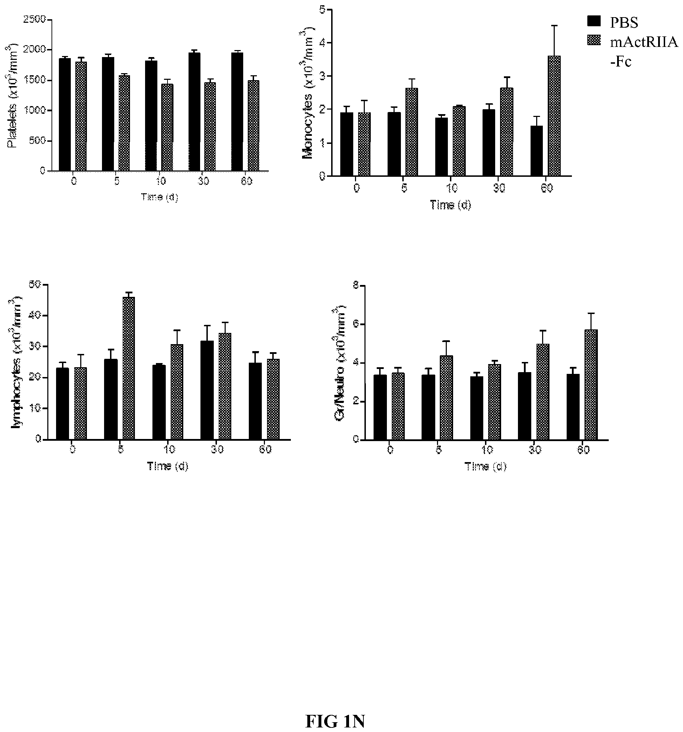

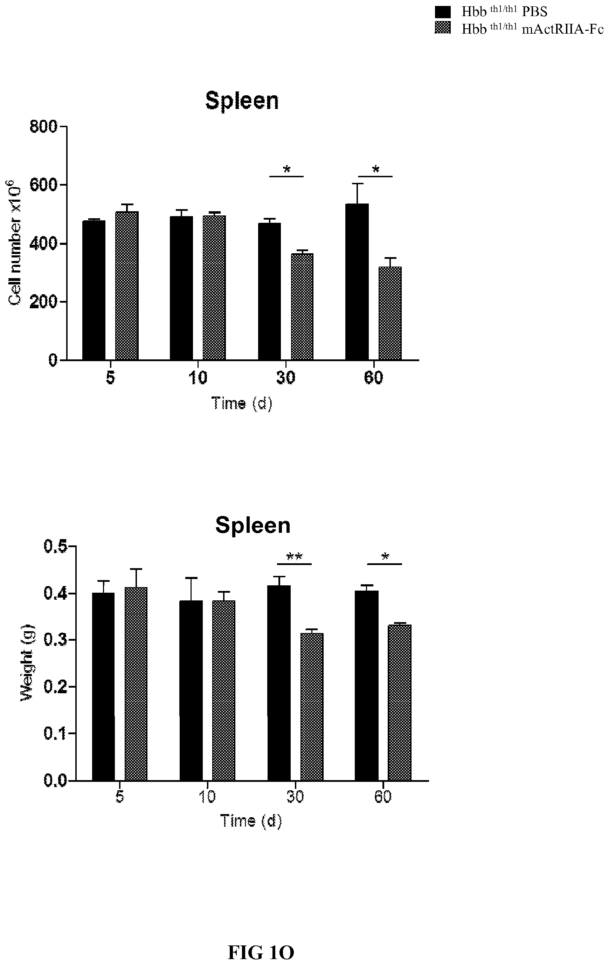

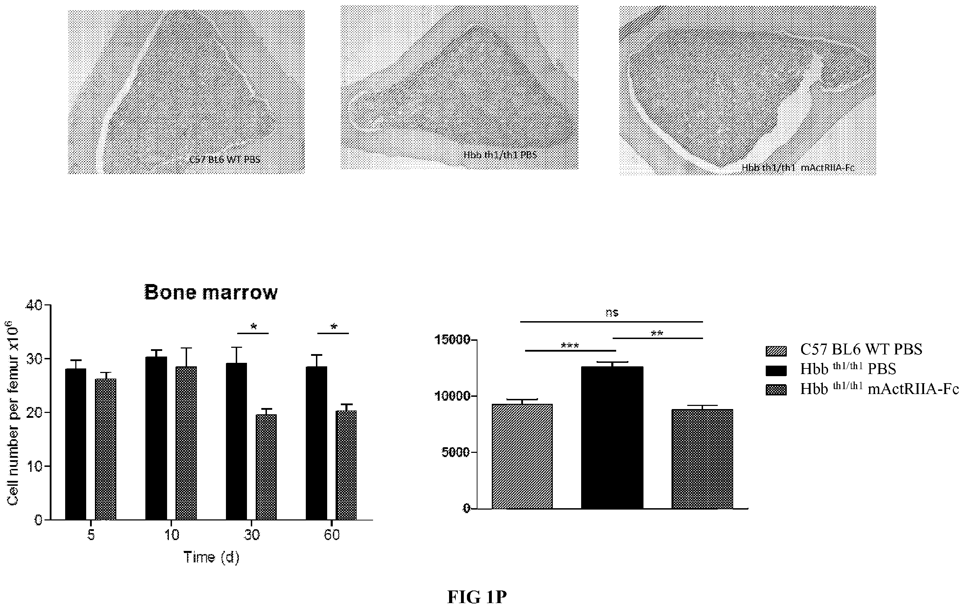

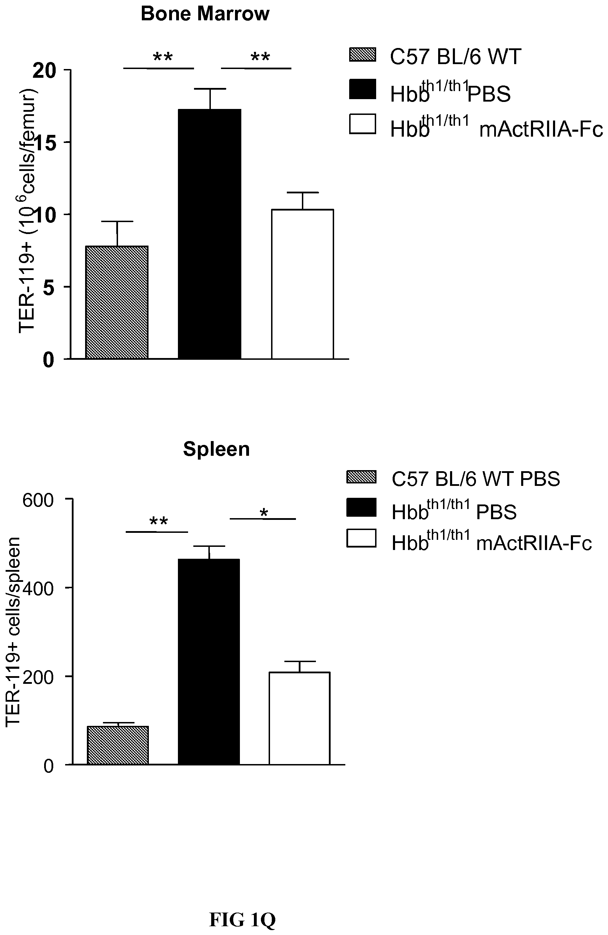

FIG. 1 illustrates that the murine counterpart of SEQ ID NO 7 (mActRIIA-Fc) ameliorates hematological parameters in β-thalassemic mice. Hbb^{th1/th1} mice (Skow LC et al., Cell 34:1043-52, 1983) were treated with PBS or mActRIIA-Fc (10 mg/Kg body weight twice a week) for 60 days. Hematological parameters were evaluated on days 5, 10, 30 and 60. Evaluation of (A) red blood cell counts, (B) hematocrit and (C) hemoglobin was associated with a decreased (D) reticulocytosis. Analysis of circulating red blood cells (RBC) parameters also show that (E) mean corpuscular volume (MCV), (F) mean corpuscular hemoglobin (MCH) and (G) MCH concentration (MCHC) increased in mice treated with mActRIIA-Fc. (H) Total antioxidant status. (I) Morphological analysis showed a reduction in anisocytosis, poikilocytosis and target cells. (J) Systemic iron levels, (K) transferrin synthesis, (L) transferrin and (M) ferritin levels saturation were also evaluated on thalassemic mice. (N) Inflammatory cell counts were also assessed. Effects of mActRIIA-Fc on splenomegaly in thalassemic mice evaluated by (O) spleen weight and total cell numbers. (P) Bone marrow erythroblasts numbers and expansion (eosin/hematoxylin staining) were also decreased in mice treated with mActRIIA-Fc. (Q) Bone marrow and spleen erythroblasts were quantified by flow cytometry by TER119 staining. * p<0.05, N=3-5 for each independent experiment.

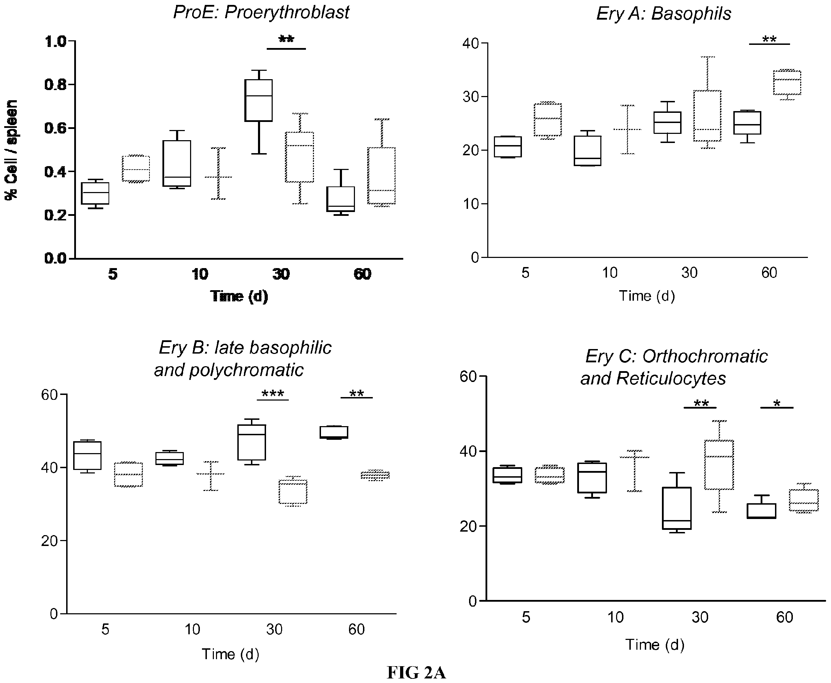

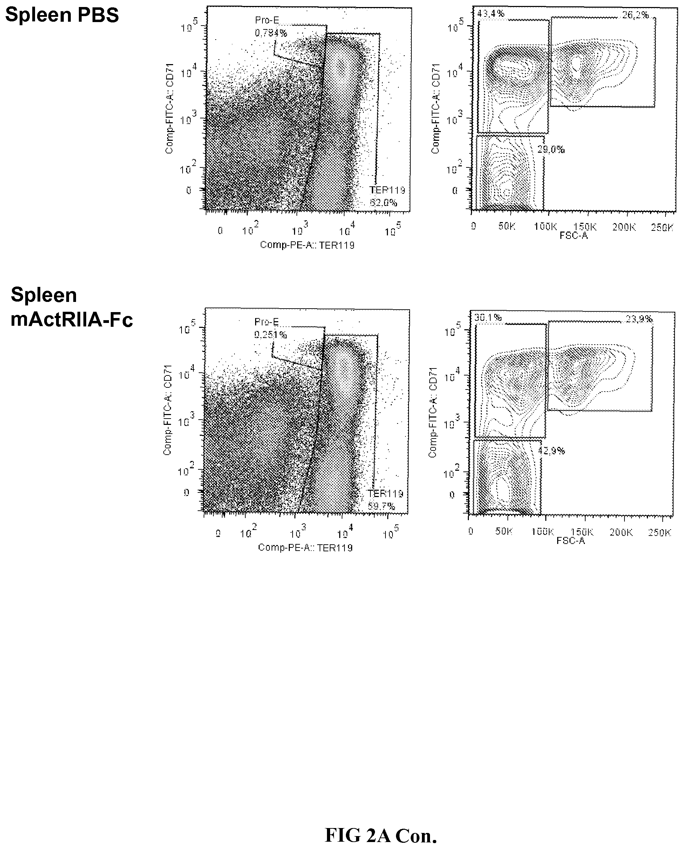

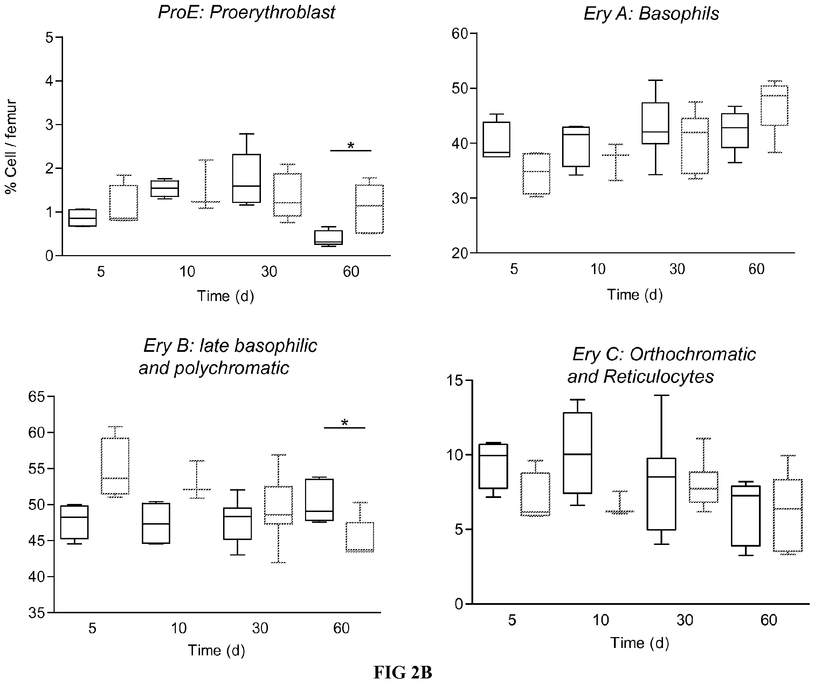

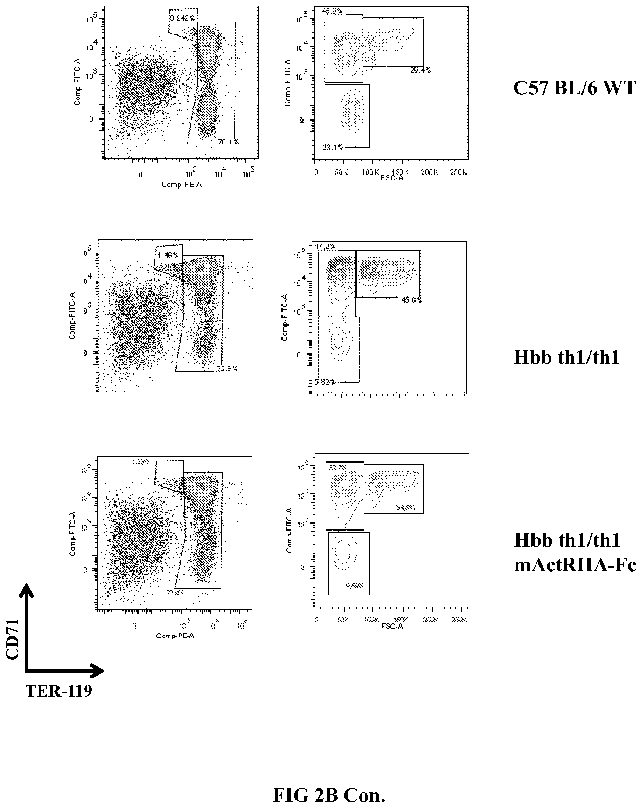

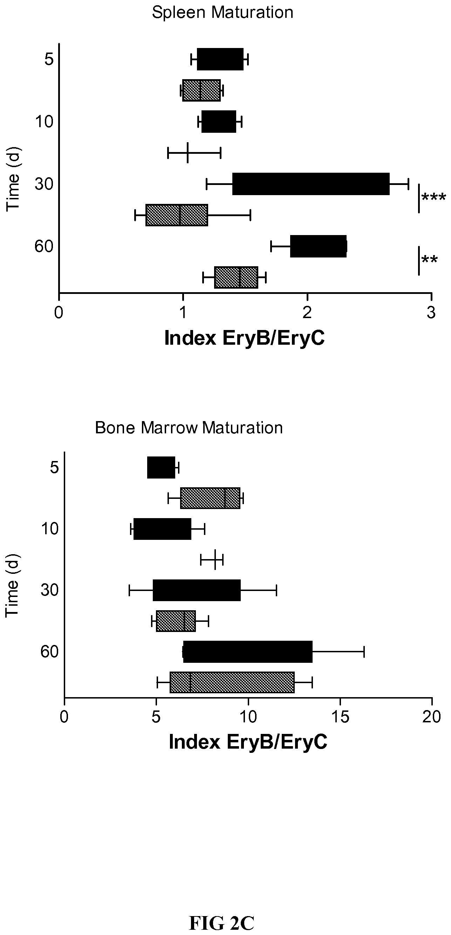

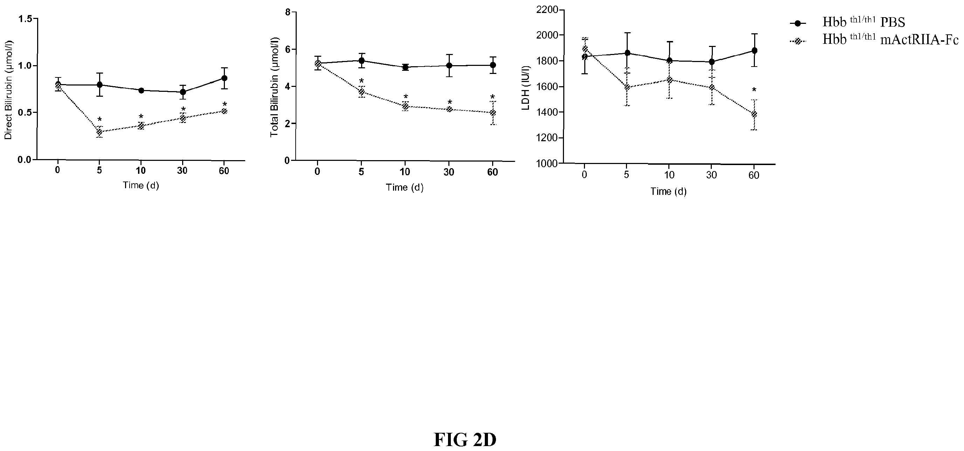

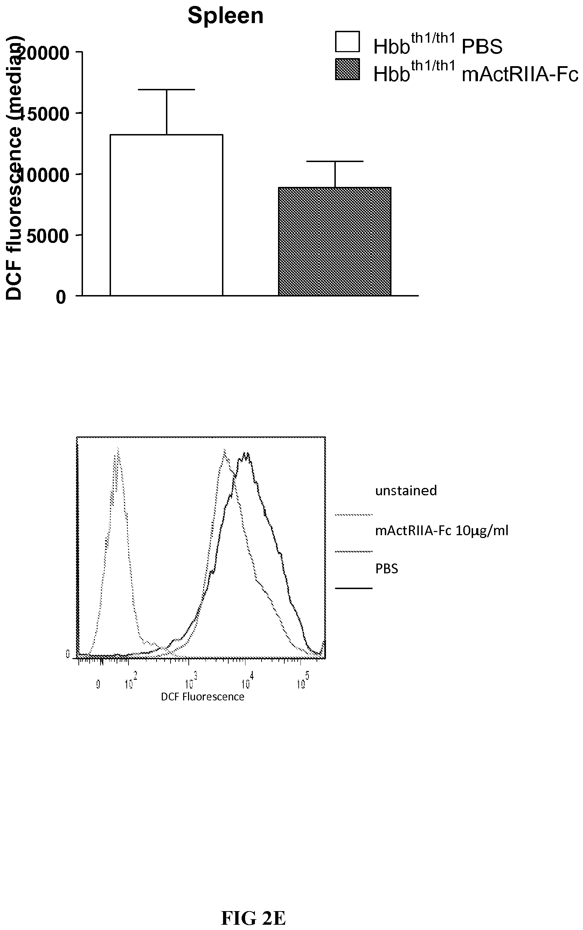

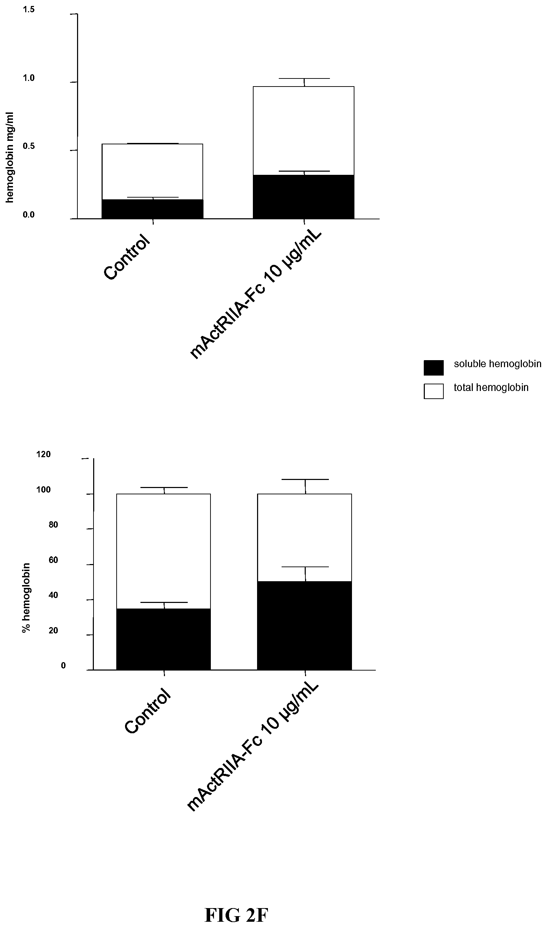

FIG. 2 illustrates that mActRIIA-Fc decreases ineffective erythropoiesis in thalassemic mice. (A-C) Bone marrow and spleen were harvested and erythroblast differentiation was evaluated by flow cytometry by CD71/TER119 staining and FSC/SSC distribution. (D) Analysis of total and direct bilirubin levels. (E) Reactive oxygen species (ROS) generation on primary pro-erythroblast differentiation was evaluated by flow cytometry using dichlorodihydrofluorescein. (F) Analysis of hemoglobin solubility on primary thalassemic pro-erythroblasts treated for 48 hours with mActRIIA-Fc or PBS. *p<0.05, **p<0.01, ***p<0.005, N=3-5 for each independent experiment.

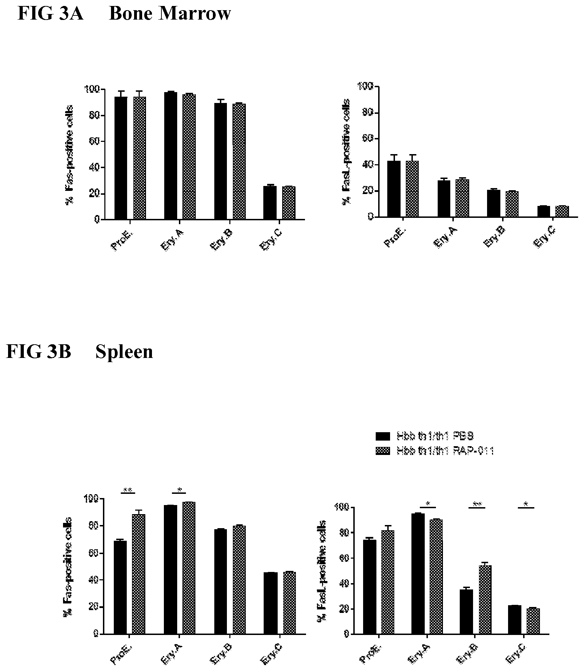

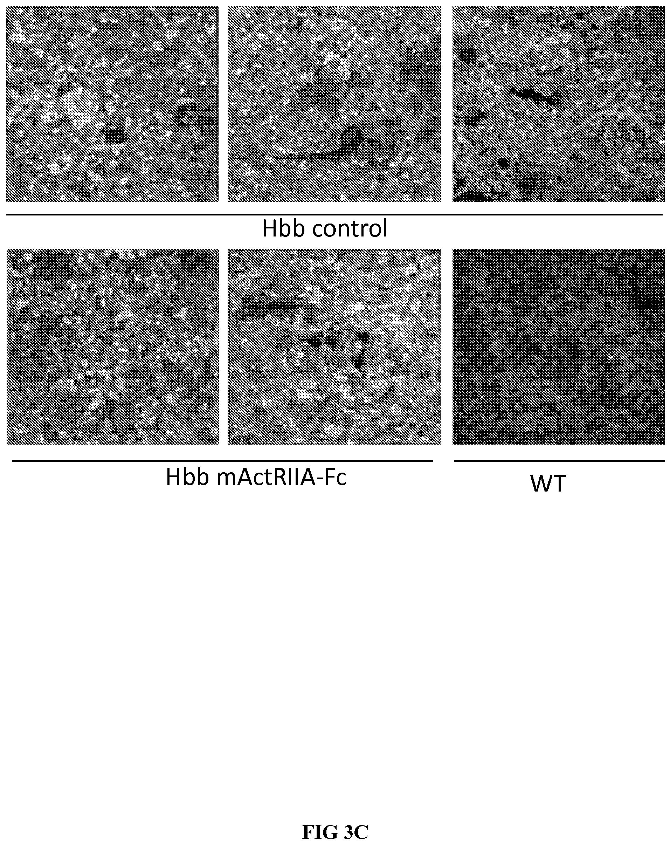

FIG. 3 illustrates the effect of mActRIIA-Fc on the apoptosis of erythroblasts from thalassemic mice. Expression of Fas-L on the bone marrow (A) and spleen (B) erythroblasts from mice treated with mActRIIA-Fc or PBS. (C) Tunel staining of erythroblasts was increased in mActRIIA-Fc-treated mice.

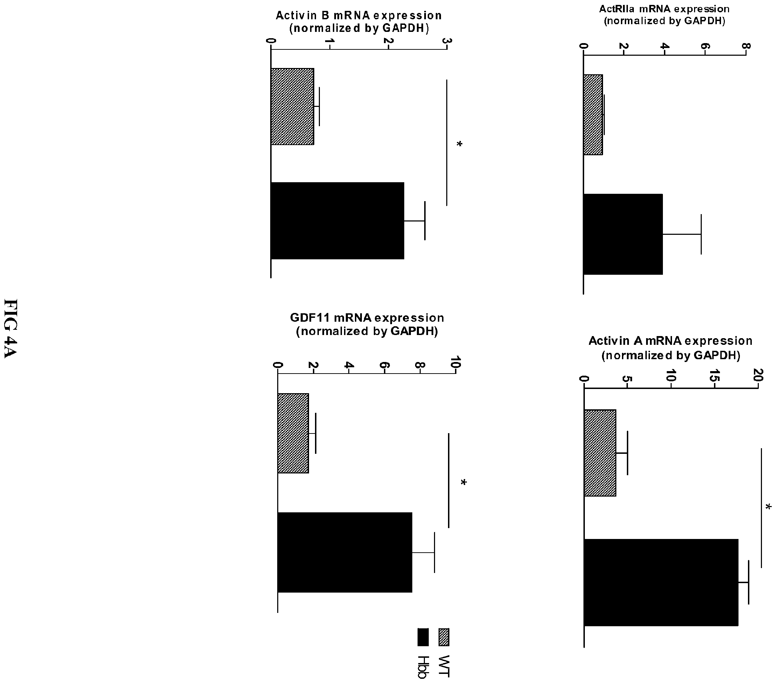

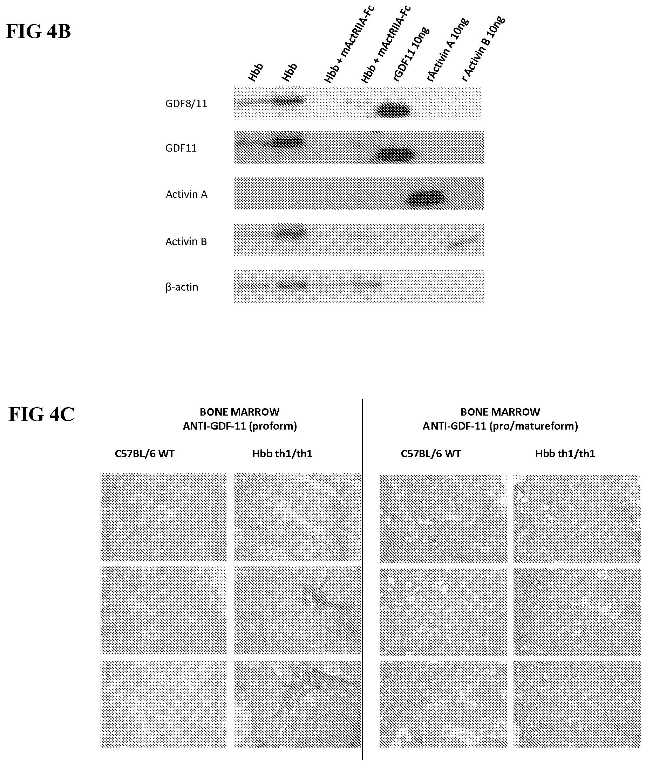

FIG. 4 illustrates the expression of ActRIIA ligands in the spleen of thalassemic mice. (A) mRNA expression levels of ActRII, activin A, activin B and GDF11 were increased in mActRIIA-Fc-treated animals. (B) Western blot analysis of GDF11 protein levels, which were decreased in mActRIIA-Fc-treated animals. (C) Immunohistochemical staining of bone marrow for GDF11 showed no change between wildtype and mice treated with mActRIIA-Fc.

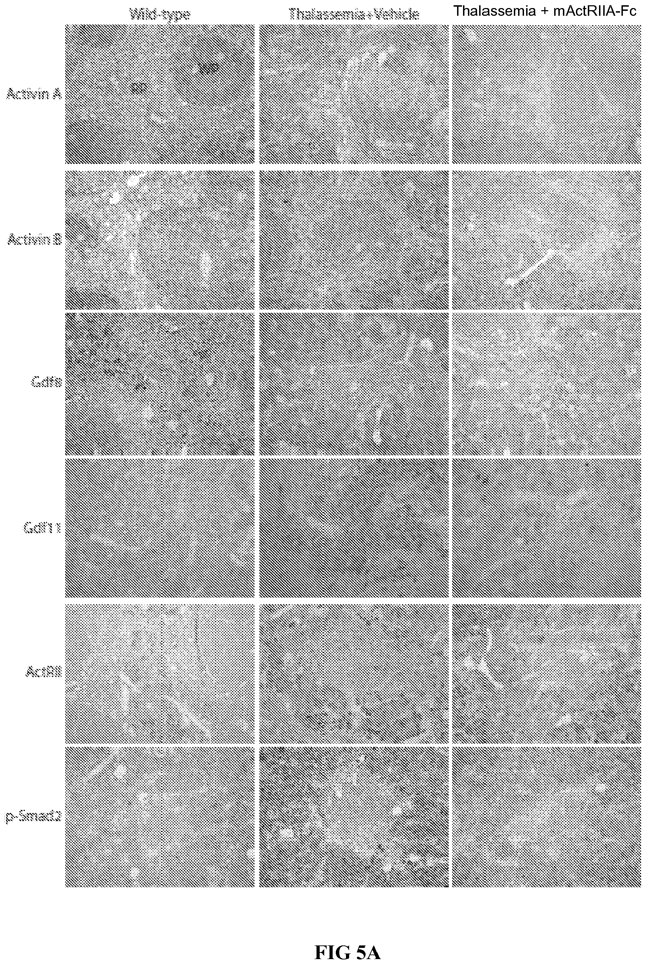

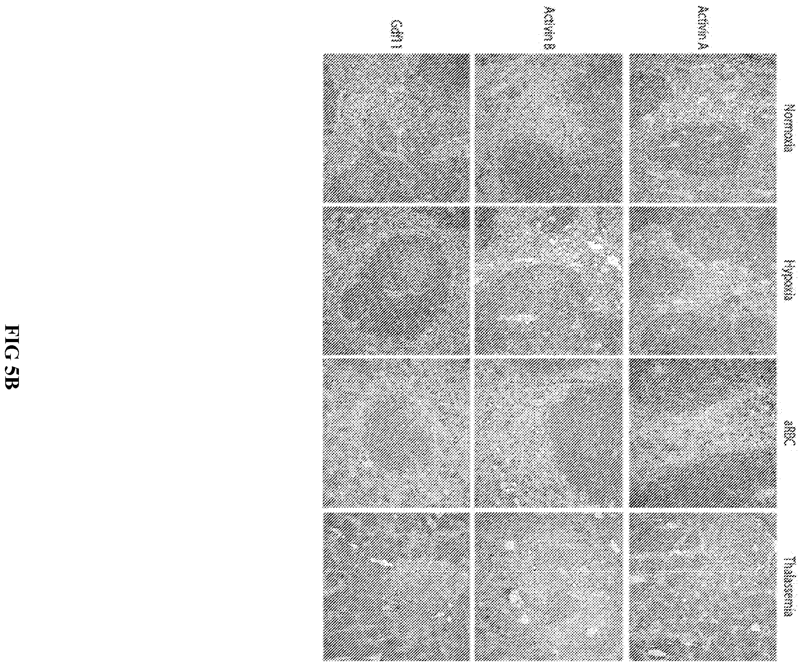

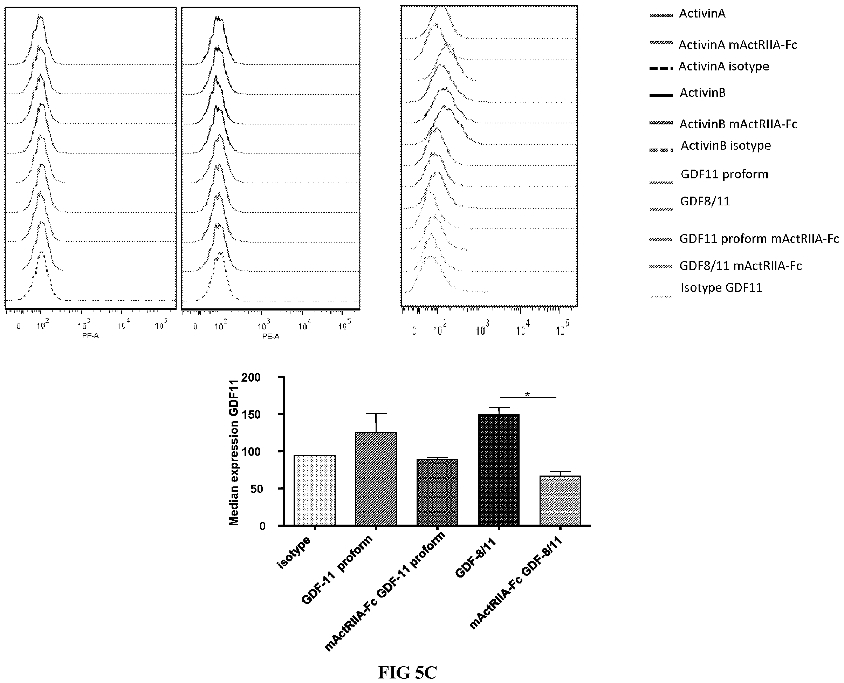

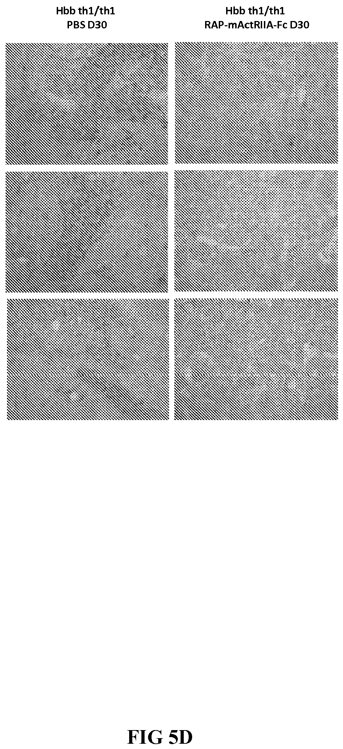

FIG. 5 illustrates the effect of mActRIIA-Fc on GDF11 expression in primary thalassemic proerythroblasts. (A) Immunohistochemical analysis of activin/GDF signaling pathway on thalassemic mice treated with PBS or mActRIIA-Fc for 30 days, showing increased levels of GDF11, ActRII and p-Smad2 on thalassemic mice. (B) Immunohistochemical analysis of activin A, activin B and GDF11 on thalassemic mice, compared to other models of anemia. (C) FACS analysis of primary thalassemic pro-erythroblasts treated with PBS or mActRIIA-Fc for 48 hours using specific antibodies against activin A, activin B, GDF11 propeptide and GDF8/GDF11 cleaved peptide. Quantification of GDF11 staining is demonstrated on the graph bars. (D) Immunohistochemical analysis of GDF11 proform expression in the spleens of thalassemic mice treated with PBS or mActRIIA-Fc. *p<0.05, N=4.

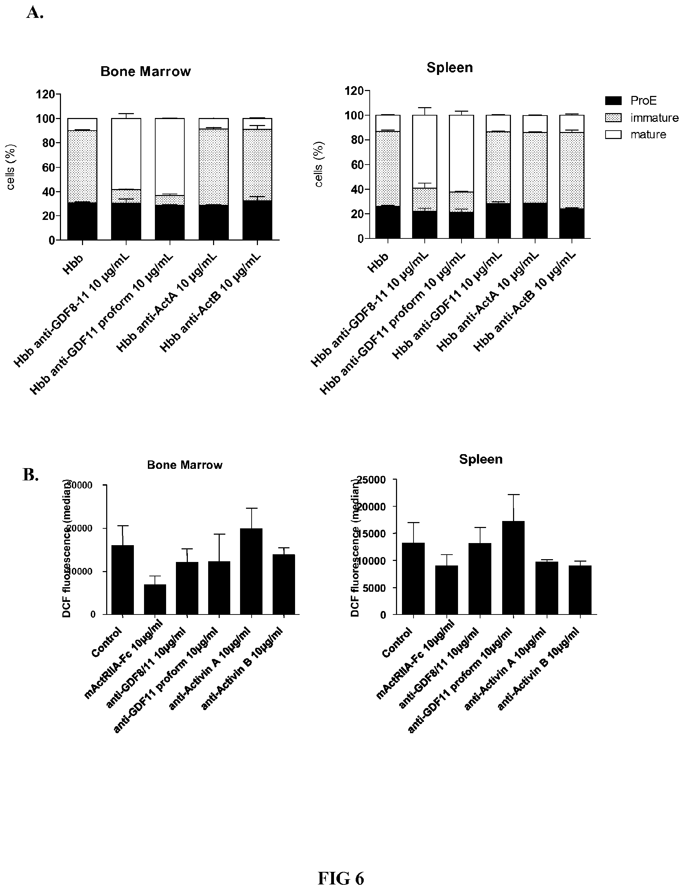

FIG. 6 illustrates that inhibition of GDF11 reduces ineffective erythropoiesis in thalassemic mice. (A) Bone marrow and spleen were harvested and primary pro-erythroblast differentiation was evaluated by flow cytometry by CD71/TER119 staining and FSC/SSC distribution. (B) ROS generation on primary pro-erythroblast differentiation was evaluated by flow cytometry using dichlorodihydrofluorescein. *p<0.05, N=4.

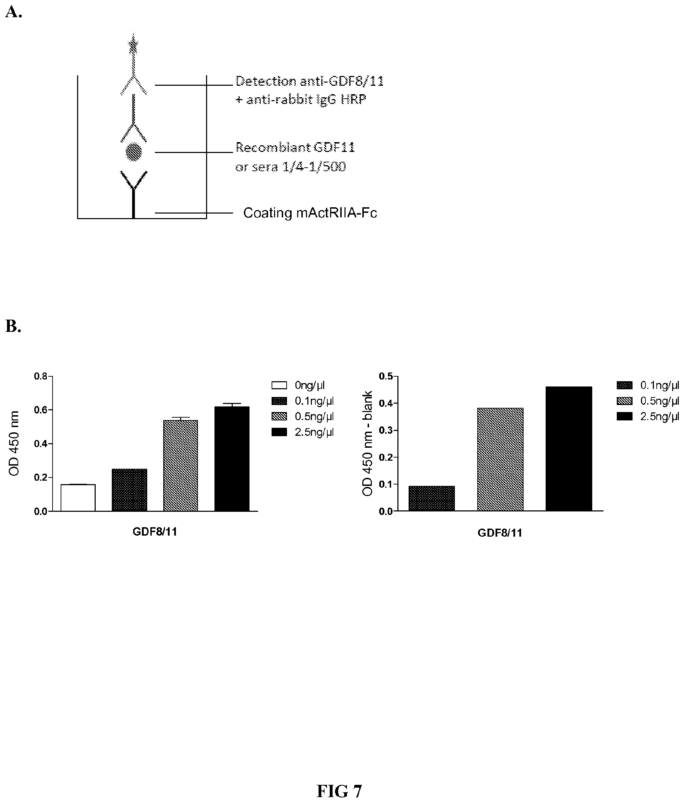

FIG. 7 illustrates a sandwich ELISA assay to detect GDF11 in serum. (A) Schematic of assay. (B) Plates were coated with 5 µg/mL of mActRIIA-Fc and increasing doses of recombinant GDF11 (0 ng/µl, 0.1 ng/µl, 0.5 ng/µl, 2.5 ng/µl) or control sera (1/4 to 1/500 dilution) were added to plates coated with mActRIIA-Fc, the plates washed and bound protein detected with anti-GDF8/11 antibodies, followed by detection using a horseradish peroxidase-coupled anti-rabbit IgG. GDF11 protein bound the plates in a dose-dependent manner.

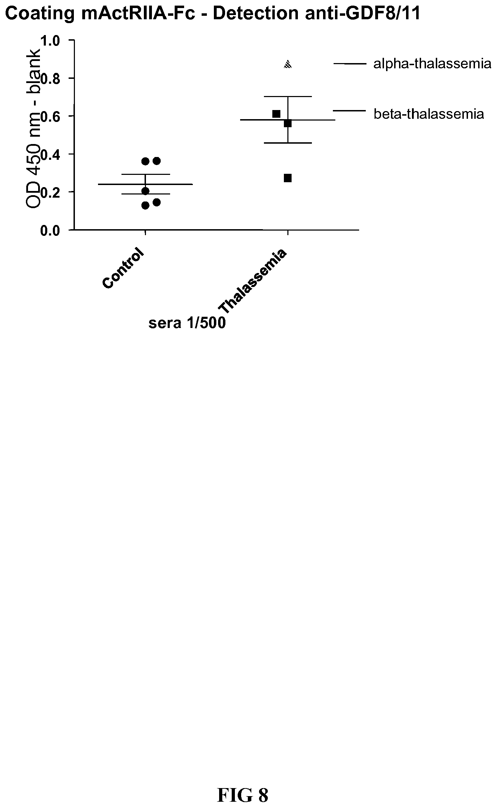

FIG. 8 illustrates the detection of increased levels of GDF11 in the serum of β-thalassemic patients. Sera were obtained from patients presenting thalassemia and healthy controls.

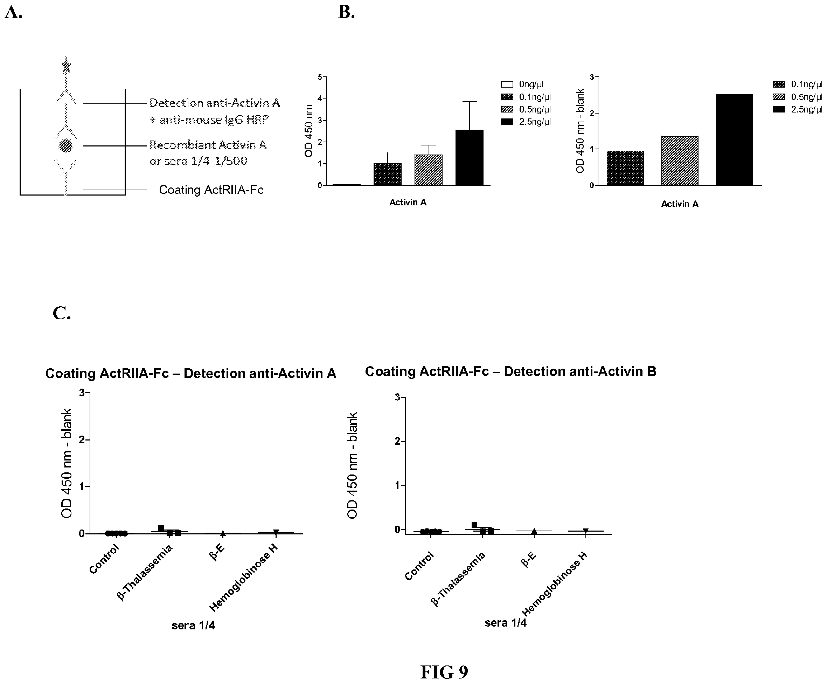

FIG. 9 illustrates a sandwich ELISA assay to detect activin A in serum. (A) Schematic of assay. (B) Plates were coated with 5 µg/mL of ActRIIA-Fc (SEQ ID NO. 7) and increasing doses of recombinant activin A (0 ng/µl, 0.1 ng/µl, 0.5 ng/µl, 2.5 ng/µl) or control sera (1/4 to 1/500 dilution) were added to ActRIIA-Fc plates, the plates washed and bound protein detected with anti-activin A antibodies, followed by detection using a horseradish peroxidase-coupled anti-rabbit IgG. Activin A protein bound the plates in a dose-dependent manner. (C) Detection of levels of activin A in the serum of β-thalassemic patients. Sera were obtained from patients presenting thalassemia and healthy controls. There was no change in the serum levels of activin A in thalassemic patients.

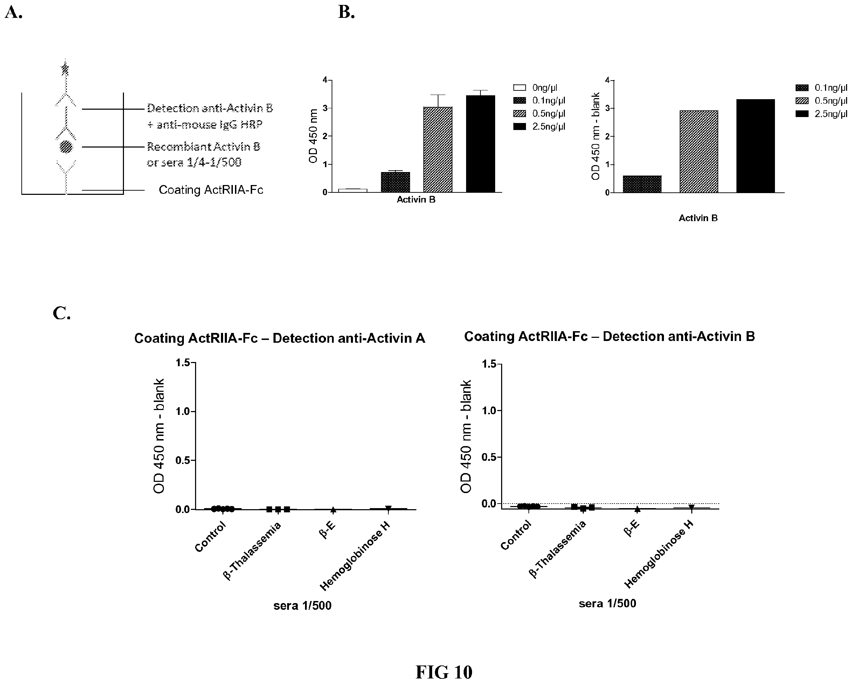

FIG 10 illustrates a sandwich ELISA assay to detect activin B in serum. (A) Schematic of assay. (B) Plates were coated with 5 µg/mL of ActRIIA-Fc and increasing doses of recombinant activin B (0 ng/µl, 0.1 ng/µl, 0.5 ng/µl, 2.5 ng/µl) or control sera (1/4 to 1/500 dilution) were added to ActRIIA-Fc plates, the plates washed and bound protein detected with anti-activin B antibodies, followed by detection using a horseradish peroxidase-coupled anti-rabbit IgG. Activin B protein bound the plates in a dose-dependent manner. (C) Detection of levels of activin B in the serum of β-thalassemic patients. Sera were obtained from patients presenting thalassemia and healthy controls. There was no change in the serum levels of activin B in thalassemic patients.

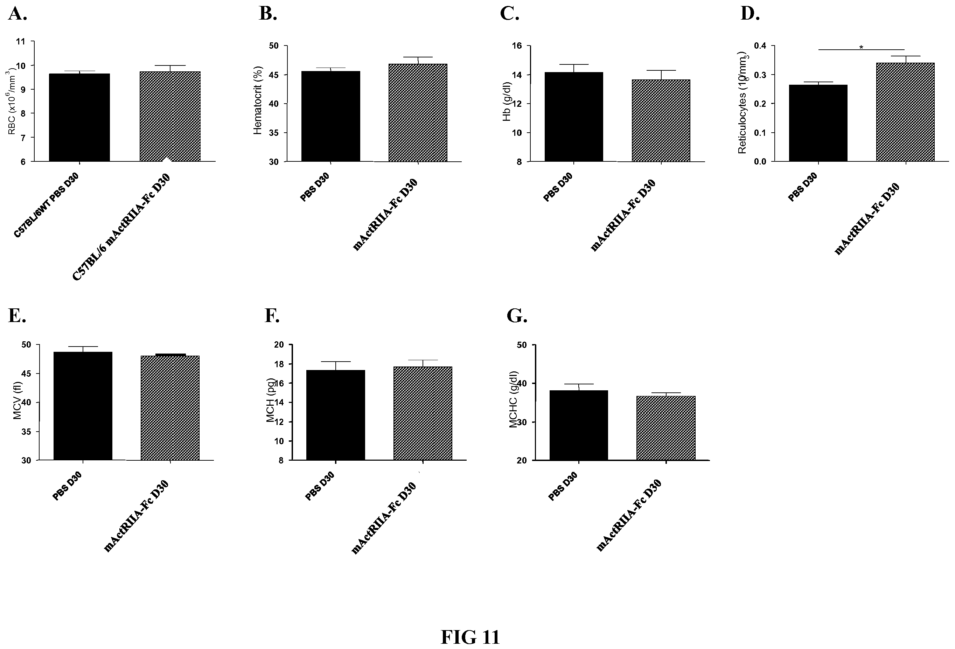

FIG. 11 illustrates that administration of mActRIIA-Fc to C57BL/6 wildtype mice does not change their hematological parameters. Evaluation of (A) red blood cell counts, (B) hematocrit, (C) hemoglobin showed no association with mActRIIA-Fc. There was a slight decrease (D) reticulocytosis. mActRIIA-Fc did not change red blood cells (RBC) parameters such as (E) mean corpuscular volume (MCV), (F) mean corpuscular hemoglobin (MCH) or (G) MCH concentration (MCHC). *p<0.05, N=3-5 for each independent experiment.

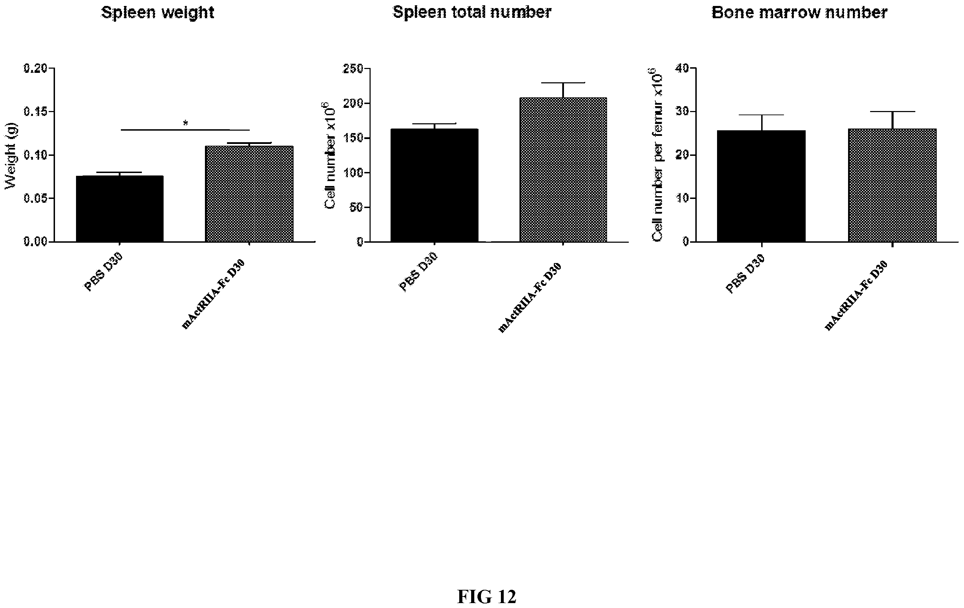

FIG. 12 illustrates that administration of mActRIIA-Fc to C57BL/6 wildtype mice had no effect on the number of spleen and bone marrow cells of the mice.

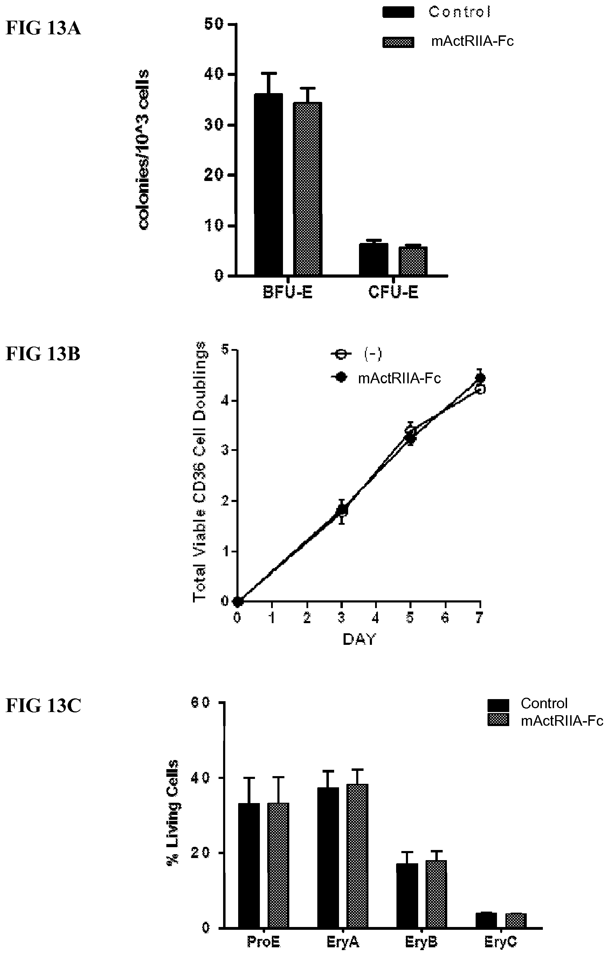

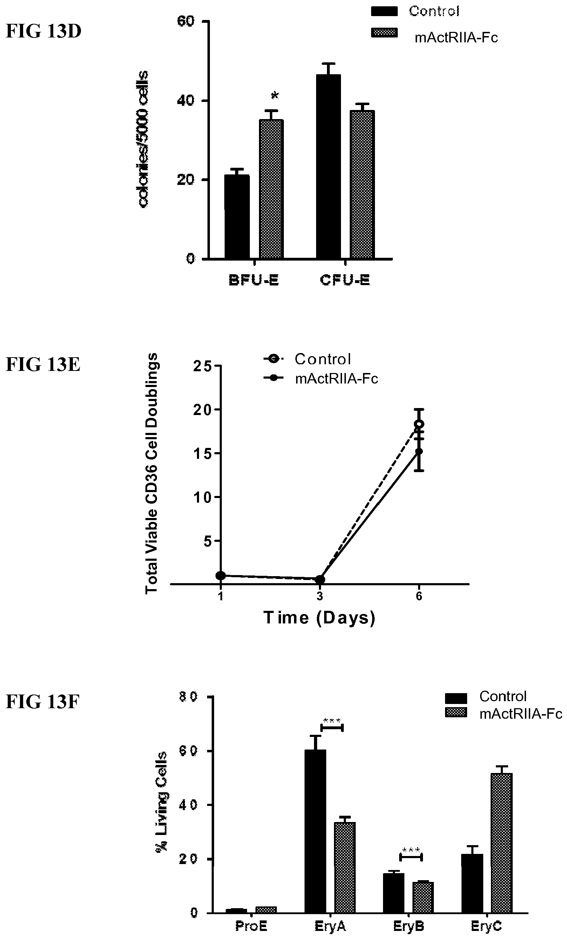

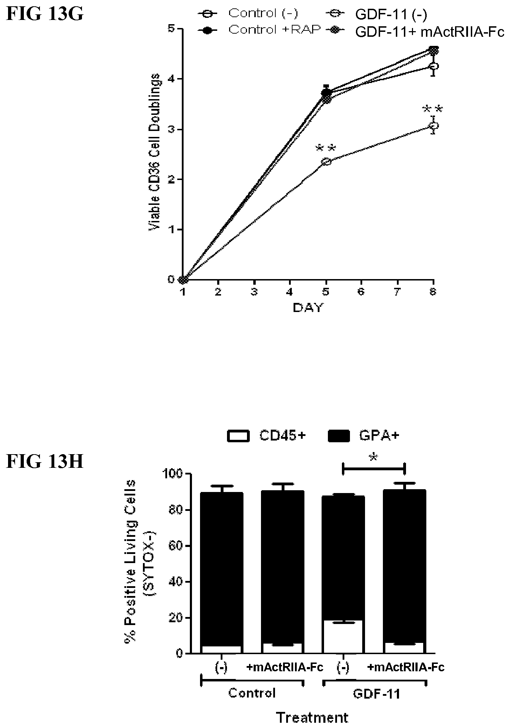

FIG. 13 illustrates that mActRIIA-Fc stimulates erythroid differentiation through inhibition of GDF11. (A-C) Erythroid differentiation of CD34+/CD36+ cells was performed by culturing in media containing EPO, +/-50µg/mL of mActRIIA-Fc; (A) erythroid progenitors, (B) cell proliferation and (C) erythoid precursors were analyzed. (D-F) Erythroid differentiation of CD34+/CD36+ cells when co-cultured with bone marrow (BM) cells in media containing EPO, +/-50µg/mL of mActRIIA-Fc; (D) erythroid progenitors, (E) cell proliferation and (F) erythoid precursors were analyzed. (G-H) Erythroid differentiation of CD36+ cells was performed by culturing in media containing EPO, +/- 200ng/mL of GDF11, +/- 100ug/mL of mActRIIA-Fc; (G) cell proliferation and (H) percentage of erythroid precursors GPA+ were analyzed

### 5. DETAILED DESCRIPTION

### 5.1 OVERVIEW

Provided herein, in one aspect, is a method for the treatment of anemia wherein the method comprises administering an antagonist of growth differentiation factor 11 (GDF11; also known as bone morphogenetic protein 11 (BMP-11)) to a patient in need of treatment. In certain embodiments, the method comprises administering to an individual in need thereof, *e.g.,* an individual having anemia, a therapeutically effective amount of a GDF11 antagonist. In certain specific embodiments, the GDF11 antagonist is not an ActRII receptor or derivative of an ActRII receptor, e.g., is not an ActRIIA-Fc fusion protein or an ActRIIB-Fc fusion protein. The antagonist of GDF11 can act at any level of GDF11, *i*.*e*., it can reduce or eliminate GDF11 expression, reduce GDF11 stability (e.g., by increasing GDF11 degradation), or antagonize GDF11 activity (e.g., by preventing GDF11 binding to its receptor). More detailed description of antagonists of GDF11 can be found in Section 5.3 below.

In certain embodiments, provided herein is a method for increasing red blood cell levels in a patient with an anemia (see, e.g., Section 5.2 for types of anemia to be treated with the methods described herein). The physiological outcome of the methods provided herein can be described as follows. In particular, increase of the following parameters indicate the treatment of the anemia in a patient. In certain embodiments, the red blood cell levels are increased by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. In certain embodiments, the hemoglobin levels are increased by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. In certain embodiments, the Hematocrit levels are increased by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. In certain embodiments, the colony forming units levels are increased by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. In certain embodiments, the mean cell volume is increased by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. In certain embodiments, the mean cell hemoglobin is increased by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. In certain embodiments, the mean corpuscular hemoglobin concentration is increased by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%.

The physiological outcome of the methods provided herein can also be described as follows. In particular, decrease of the following parameters indicate the treatment of the ineffective erythropoiesis in a patient. In certain embodiments, the proportion of reticulocytes in the blood is reduced by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. In certain embodiments, the proportion of reticulocytes in the blood is reduced by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. In certain embodiments, spleen weight or spleen total cell number is reduced by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. In certain embodiments, bone marrow cell number per femur is reduced by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. In certain embodiments, bone marrow erythroblast number is reduced by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%.

### 5.2 ANEMIA TO BE TREATED

In certain embodiments, a patient to be treated with the methods provided herein is diagnosed with anemia. In certain more specific embodiments, the anemia is caused by ineffective erythropoiesis. In certain embodiments, provided herein is a method for treating an inherited bone marrow failure syndrome (such as, but not limited to, Amegakaryocytic Thrombocytopenia, Diamond-Blackfan Anemia, Dyskeratosis Congenita, Fanconi Anemia, Pearson Syndrome, Severe Congenital Neutropenia, Shwachman-Diamond Syndrome, Thrombocytopenia Absent Radii), in particular, an inherited bone marrow failure syndrome that affects red blood cells. More specifically, provided herein are methods for treating an inherited bone marrow failure syndrome that specifically affects red blood cells. In certain embodiments, provided herein is a method for treating an anemia wherein the subject is unresponsive to administration of erythropoietin. In certain embodiments, provided herein is a method for treating an anemia wherein the subject is unresponsive to administration of iron, vitamin B-12, and/or folate. In certain embodiments, provided herein is a method for treating an anemia resulting from erythroid progenitors and precursors being highly sensitive to death by apoptosis.

In certain even more specific embodiments, a patient to be treated with the methods provided herein is diagnosed with beta-thalassemia. Beta-Thalassemia is a disease characterized by a defect in hemoglobin synthesis leading to impaired erythrocyte maturation and production. The decrease is primarily thought to be due to abnormally accelerated erythroid differentiation and apoptosis at the late basophilic/polychromatic erythroblast stage of red blood cell differentiation, resulting in an overall decrease in mature red blood cell production. The disease is characterized by a hypercellular bone marrow compartment where abnormal erythroblasts accumulate and undergo apoptosis, resulting in systemic anemia.

In certain embodiments, a patient to be treated with the methods provided herein has increased levels of GDF11 expression and/or activity in the spleen and/or the bone marrow and/or serum and/or plasma and/or liver. In certain embodiments, the GDF11 levels of the patient are compared relative to levels of GDF11 expression and/or activity in the same person prior to the anemia (if sample or data is available) or relative to levels of GDF11 expression and/or activity in a human without anemia. In certain embodiments, GDF11 expression and/or activity levels are measured / compared from spleen, bone marrow, serum, plasma, and/or liver. In certain embodiments, GDF11 mRNA levels in a patient to be treated with the methods provided herein are elevated by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, 100%, 125%, 150%, 175%, 200%, 250%, or at least 500%. In certain embodiments, GDF11 protein levels in a patient to be treated with the methods provided herein are elevated by at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, 100%, 125%, 150%, 175%, 200%, 250%, or at least 500%.

In certain embodiments, a patient to be treated with the methods provided herein is not responsive to treatment with erythropoietin. In certain embodiments, the methods provided herein further comprise co-administration of EPO, wherein the EPO can be administered prior to, concurrently with, or subsequent to the administration of the antagonist of GDF11.

In certain embodiments, a patient to be treated with the methods provided herein has hemoglobin levels of less than 13 g/dl, less than 12.5 g/dl, less than 12 g/dl, less than 11.5 g/dl, less than 11 g/dl, less than 10.5 g/dl, less than 10 g/dl, less than 9.5 g/dl, or less than 9 g/dl.

In certain embodiments, the anemia to be treated with the methods provided herein is a chemotherapy-induced anemia.

### 5.3 ANTAGONISTS OF GDF11

GDF11 antagonists that can be used with the methods provided herein are described below. In certain embodiments, the GDF11 antagonists that can be used with the methods provided herein target the expression of GDF11. In other embodiments, the GDF11 antagonists that can be used with the methods provided herein target the activity of GDF11. In other embodiments, the GDF11 antagonists that can be used with the methods provided herein target the protein stability of GDF11 (e.g., increases degradation). The suitability of GDF11 antagonists that can be used with the methods provided herein can be demonstrated, *e.g*., using the assays described in Section 5.4 below.

Accordingly, GDF11 antagonists for use in the methods provided herein, for example, downregulate/reduce, neutralize, block, inhibit and/or ameliorate GDF11-expression and/or GDF11-mediated biological effects. In one embodiment, GDF11 antagonists are capable of inhibiting the activity, signal transduction, receptor binding, expression, processing or secretion of GDF11. Thus, such a GDF11 antagonist reduces the activity of GDF11 relative to GDF11 activity in the absence of the antagonist. In certain embodiments, the activity of the GDF11 protein is reduced by at least about 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or by at least 100%. A GDF11 antagonist may also, in one embodiment, affect the stability of a GDF11 protein. In another embodiment, a GDF11 antagonist may affect the conversion of a GDF11 precursor protein into the mature GDF11 form. In another embodiment, GDF11 antagonists can interfere with the binding of GDF11 to a cognate receptor (e.g., ActRII receptor) and/or inhibit intracellular signaling by a GDF11 cognate receptor.

GDF11 antagonists include GDF11 "traps" (e.g., inhibitory GDF11 receptor polypeptides (e.g., ActRII polypeptides)), anti-GDF11 antibodies, GDF11 propeptides, GDF11 analogs (e.g., dominant negative polypeptides, peptidomimetics), other GDF11-binding proteins (e.g., follistatin, follistatin-related gene, follistatin domain-containing proteins), synthetic small molecule inhibitors, polynucleotides that inhibit GDF11 expression (e.g., antisense, interfering RNA (RNAi), and triplexing molecules and ribozymes) and the like. In other embodiments, GDF11 antagonists also include antibodies to GDF11 receptors (e.g., ActRIIB and ActRIIA). In certain specific embodiments, an antagonist of GDF11 binds to a GDF11 receptor (e.g., ActRIIB and ActRIIA) and prevents binding of GDF11 to its receptor (e.g., by steric hindrance) but does not (or to a lesser degree) affect binding of other ligands (such as Activin A).

In certain embodiments, a GDF11 antagonist for use with the methods provided herein is not an ActRIIA polypeptide and/or an ActRIIB polypeptide. In certain embodiments, a GDF11 antagonist for use with the methods provided herein is not an inhibitor of ActRIIA and/or an inhibitor ActRIIB polypeptide. In certain embodiments, a GDF11 antagonist for use with the methods provided herein does not comprise a ligand-binding portion of ActRIIA and/or a ligand-binding portion of ActRIIB.

In certain embodiments, a GDF11 antagonist binds to a GDF11 polypeptide sequence, fragment or variant thereof. In one embodiment, the GDF11 antagonists bind to non-mammalian (e.g., chicken, fish) GDF11 proteins. In another embodiment, the GDF11 antagonists bind to a mammalian (e.g., human, mouse, rat, dog, cat, pig, gerbil, hamster, cow, horse, goat, sheep, rabbit, camel, cynomologous, chimpanzee, marmoset, rhesus) GDF11 protein. A number of GDF11 molecules have been described and homologs are well known in the art. In yet another embodiment, the GDF11 antagonists bind to a human GDF11 protein having the polypeptide sequence of SEQ ID NO:50 or SEQ ID NO:52.

Like other TGF-beta family members, GDF11 is synthesized as a preproprotein comprised of a signal sequence, an amino-terminal propetide and a carboxy-terminus consisting of disulfide-linked fragments. The propeptide is proteolytically cleaved from the rest of the molecule, leaving the bioactive carboxy-terminal dimer as the mature receptor-binding form. The propeptide functions as an inhibitor of the mature GDF11 dimer, binding to and inhibiting its activity. The full-length, unprocessed precursor protein, i.e., GDF11 preproprotein can have the amino acid sequence of SEQ ID NO:48, the GDF propeptide can have the amino acid sequence of SEQ ID NO:50, and the mature form of GDF11 has the amino acid sequence of SEQ ID NO:52.

### 5.3.1 ANTI-GDF11 ANTIBODIES

In certain embodiments, antibodies against GDF11 can be used with the methods provided herein. In certain embodiments, antibodies against GDF11 bind immunospecifically to GDF11. In particular, in certain embodiments, such anti-GDFl 1 antibodies bind to GDF11 with higher binding affinity than to any other protein. In specific embodiments, anti-GDFl 1 antibodies bind to GDF11 with higher affinity than to GDF8. In a specific embodiment, anti-GDF11 antibodies do not cross-react with GDF8. In specific embodiments, anti-GDFl 1 antibodies bind to GDF11 with higher affinity than to Activin A. In a specific embodiment, anti-GDF11 antibodies do not cross-react with Activin A.

By using GDF11 as an immunogen, anti-protein/anti-peptide antisera or monoclonal antibodies can be made by standard protocols (see, for example, Antibodies: A Laboratory Manual ed. by Harlow and Lane (Cold Spring Harbor Press: 1988)). A mammal, such as a mouse, a hamster or rabbit can be immunized with an immunogenic form of GDF11, an antigenic fragment which is capable of eliciting an antibody response, or a fusion protein. Techniques for conferring immunogenicity on a protein or peptide include conjugation to carriers or other techniques well known in the art. An immunogenic portion of GDF11 can be administered in the presence of adjuvant. The progress of immunization can be monitored by detection of antibody titers in plasma or serum. Standard ELISA or other immunoassays can be used with the immunogen as antigen to assess the levels of antibodies.

Following immunization of an animal with an antigenic preparation of GDF11, antisera can be obtained and, if desired, polyclonal antibodies can be isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique (Kozbar et al., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with GDF11 and monoclonal antibodies isolated from a culture comprising such hybridoma cells.

The term "antibody" as used herein is intended to include fragments thereof which are also specifically reactive with a subject polypeptide. Antibodies can be fragmented using conventional techniques and the fragments screened for utility in the same manner as described above for whole antibodies. For example, F(ab)₂ fragments can be generated by treating antibody with pepsin. The resulting F(ab)₂ fragment can be treated to reduce disulfide bridges to produce Fab fragments. An antibody is further intended to include bispecific, single-chain, chimeric, humanized and fully human molecules having affinity for an ActRII receptor or activin polypeptide conferred by at least one CDR region of the antibody. The term "antibody" also includes Fab, Fab', and F(ab') fragments.

In certain embodiments, the antibody is a recombinant antibody, which term encompasses any antibody generated in part by techniques of molecular biology, including CDR-grafted or chimeric antibodies, human or other antibodies assembled from library-selected antibody domains, single chain antibodies and single domain antibodies (e.g., human V_{H} proteins or camelid V_{HH} proteins). In certain embodiments, an antibody can be a monoclonal antibody. Each monoclonal antibody typically recognizes a single epitope and is an antibody obtained from a population of homogenous, or substantially homogenous, antibodies. The term "monoclonal" is not limited by any particular method of production. For example, a method for generating a monoclonal antibody that binds specifically to GDF11 may comprise administering to a mouse an amount of an immunogenic composition comprising the antigen polypeptide effective to stimulate a detectable immune response, obtaining antibody-producing cells (e.g., cells from the spleen) from the mouse and fusing the antibody-producing cells with myeloma cells to obtain antibody-producing hybridomas, and testing the antibody-producing hybridomas to identify a hybridoma that produces a monoclonal antibody that binds specifically to the antigen. Once obtained, a hybridoma can be propagated in a cell culture, optionally in culture conditions where the hybridoma-derived cells produce the monoclonal antibody that binds specifically to the antigen. The monoclonal antibody may be purified from the cell culture.

The adjective "specifically reactive with" as used in reference to an antibody is intended to mean, as is generally understood in the art, that the antibody is sufficiently selective between the antigen of interest (e.g., a GDF11 polypeptide) and other antigens that are not of interest that the antibody is useful for, at minimum, detecting the presence of the antigen of interest in a particular type of biological sample. In certain methods employing the antibody, such as therapeutic applications, a higher degree of specificity in binding may be desirable. Monoclonal antibodies generally have a greater tendency (as compared to polyclonal antibodies) to discriminate effectively between the desired antigens and cross-reacting polypeptides. One characteristic that influences the specificity of an antibody:antigen interaction is the affinity of the antibody for the antigen. Although the desired specificity may be reached with a range of different affinities, generally antibodies will have an affinity (a dissociation constant) of about 10⁻⁶, 10⁻⁷, 10⁻⁸, 10⁻⁹ or 10⁻¹⁰ or less.

In addition, the techniques used to screen antibodies in order to identify a desirable antibody may influence the properties of the antibody obtained. For example, if an antibody is to be used for binding an antigen in solution, it may be desirable to test solution binding. A variety of different techniques are available for testing interaction between antibodies and antigens to identify particularly desirable antibodies. Such techniques include ELISAs, surface plasmon resonance binding assays (e.g., the BIACORE binding assay, Biacore AB, Uppsala, Sweden), sandwich assays (e.g., the paramagnetic bead system of IGEN International, Inc., Gaithersburg, Md.), Western blots, immunoprecipitation assays, and immunohistochemistry.

In one embodiment, the anti-GDFl 1 antibodies bind GDF11 with high affinity. Thus, in one embodiment, the anti-GDFl 1 antibodies have an IC50 of less than or equal to about 40 nM, 30 nM, 25 nM, 20 nM, or 10 nM. In another embodiment, anti-GDFl 1 antibodies that specifically bind GDF11 have an IC50 value of less than or equal to about 5 nM, 4 nM, 3 nM, or 1 nM in an in vitro assay for binding to GDF11 (such as a BIACORE assay or ELISA assay). In one embodiment, the anti-GDFl 1 antibodies for use in the methods have a strong binding affinity (Kd) for GDF11. Thus the anti-GDFl 1 antibodies have a Kd of less than about 4.2x10⁻⁹ M or 4.0x10⁻⁹ M, less than about 4.6x10⁻¹⁰ M, 4.0x10⁻¹⁰ M or 2x10⁻¹⁰ M or less than about 8x10^{- 11} M, 7x10⁻¹¹ M, 5x10⁻¹² M or 1.4x10⁻¹² M. Alternatively, the anti-GDF antibodies for use in the methods described herein have a Kd for GDF11 no greater than about 4.2x10⁻⁹ M or 4.0x10⁻⁹ M, no greater than about 4.6x10⁻¹⁰ M, 4.0x10⁻¹⁰ M or 2x10⁻¹⁰ M or no greater than about 8x10⁻¹¹ M, 7x10⁻¹¹ M, 5x10⁻¹² M or 1.4x10⁻¹² M. The Kd of the anti-GDFl 1 antibodies can be determined by methods well-known in the art (using, e.g., the BIACORE™ system).

In another embodiment, the anti-GDFl 1 antibodies preferentially bind GDF11 over myostatin/GDF8. Accordingly, in one embodiment, the anti-GDFl 1 antibodies for use in the methods described herein bind GDF11 with at least a 1.5 fold or higher, at least a 2-fold or higher, at least a 2.5 fold or higher, at least a 3-fold or higher, at least a 3.5-fold or higher, or at least a 4-fold or higher preference for GDF11 compared to GDF-8. In another embodiment, the anti-GDFl 1 antibodies preferentially inhibit GDF11 activity over that of myostatin / GDF8. Examples of anti-GDF8/11 antibodies are disclosed, for example, in several published patents and applications, for example, US Patent No. 8,066,995; 7,320,789 (murine monoclonal antibody JA-16, ATCC Deposit No. PTA-4236); US Patent No. 7,655,763 (e.g., human monoclonal antibodies Myo29 (Stamulumab) (ATCC Deposit No. PTA-4741), Myo22 (ATCC Deposit No. PTA-4740), Myo28 (ATCC Deposit No. PTA-4739)); US Patent No. 7,261,893 and US Patent Application No. 20110293630 (Serial No. 13/115,170). Examples of monoclonal antibodies that can be used with the methods provided herein include antibodies from LifeSpan Biosciences Inc., Seattle, WA, with catalog numbers LS-C121127, LS-C138772, LS-C105098 (available); antibodies available from Santa Cruz Biotechnology, Inc., Santa Cruz, CA, with catalog number (X-19): sc-81952; and antibodies available from Sigma-Aldrich Co. LLC, with product number: WH0010220M3. The skilled artisan can use routine technologies to use the antigen-binding sequences of these antibodies (e.g., the CDRs) and generate humanized antibodies for treatment of anemia as disclosed herein.

### 5.3.2 GDF11 ANTAGONISTS COMPRISING ACTRII POLYPEPTIDES

In certain embodiments, GDF11 antagonists comprise part of the extracellular domain of an ActRII receptor, such as ActRIIA or ActRIIB, *e.g*., human ActRIIA or ActRIIB. More specifically, such GDF11 antagonists can be polypeptides comprising the GDF11-binding domain of ActRII, such as ActRIIA or ActRIIB. Without being bound by theory, such GDF11-binding domain comprising polypeptides sequester GDF11 and thereby prevent GDF11 signaling. These GDF11-binding domain comprising polypeptides may comprise all or a portion of the extracellular domain of an ActRII receptor (i.e., all or a portion of the extracellular domain of ActRIIA or all or a portion of the extracellular domain of ActRIIB). In specific embodiments, the extracellular domain of an ActRII receptor is soluble.

In certain embodiments, the GDF11-binding, extracellular domain of an ActRII receptor is mutated relative to the wild-type receptor such that the GDF11-binding, extracellular domain of an ActRII receptor binds with higher affinity to GDF11 than to any other TGFbeta. In particular, the GDF11-binding, extracellular domain of an ActRII receptor is mutated relative to the wild-type receptor such that the GDF11-binding, extracellular domain of an ActRII receptor binds with higher affinity to GDF11 than to Activin A. Such higher affinity can be at least 10%, 25%, 50%, 75%, 100%, 250%, 500%, or 1000% higher than the affinity to the next highest affinity ligand.

In certain embodiments, the GDF11-binding domain comprising polypeptides are linked to an Fc portion of an antibody (i.e., a conjugate comprising an activin-binding domain comprising polypeptide of an ActRII receptor and an Fc portion of an antibody is generated). Without being bound by theory, the antibody portion confers increased stability on the conjugate and/or reduces the patient's immune response against the GDF11 antagonist. In certain embodiments, the GDF11-binding domain is linked to an Fc portion of an antibody via a linker, *e.g*., a peptide linker.

Examples of such ActRII polypeptide GDF11 antagonists are disclosed in several published patents/applications. For example, ActRIIA polypeptide inhibitors as disclosed in U.S. Patent No. 7,709,605; U.S. Patent No. 8,252,900; U.S. Patent No. 7,960,343; U.S. Patent No. 7,988,973. Examples of ActRIIB polypeptide inhibitors that bind several TGF-beta ligands are known in the art and disclosed, for example, in U.S. Patent No. 8,138,143, U.S. Patent No. 8,058,229 and U.S. Patent No. 7,947,646. Examples of ActRIIB polypeptide inhibitors that specifically bind GDF8 and GDF11 are disclosed in U.S. Patent No. 7,842,663. Examples of ActRIIB antagonists that specifically bind GDF11 are disclosed in U.S. Patent No. 8,216,997. Clinical ActRII traps include AMG-745, ACE-031 and ACE-011.

### (a) ACTRIIA

As used herein, the term "ActRIIA" refers to a family of activin receptor type IIa (ActRIIA) proteins from any species and variants derived from such ActRIIA proteins by mutagenesis or other modification. Reference to ActRIIA herein is understood to be a reference to any one of the currently identified forms. Members of the ActRIIA family are generally transmembrane proteins, composed of a ligand-binding extracellular domain with a cysteine-rich region, a transmembrane domain, and a cytoplasmic domain with predicted serine/threonine kinase activity.

The term "ActRIIA polypeptide" includes polypeptides comprising any naturally occurring polypeptide of an ActRIIA family member as well as any variants thereof (including mutants, fragments, fusions, and peptidomimetic forms) that retain a useful activity. For example, ActRIIA polypeptides include polypeptides derived from the sequence of any known ActRIIA having a sequence at least about 80% identical to the sequence of an ActRIIA polypeptide, and optionally at least 85%, 90%, 95%, 97%, 98%, 99% or greater identity. Examples of ActRIIA polypeptides include human ActRIIA precursor polypeptide (SEQ ID NO: 1) and soluble human ActRIIA polypeptides (e.g., SEQ ID NOS:2, 3, 7 and 12). With respect to the ActRIIA precursor polypeptide whose amino acid sequence is depicted at SEQ ID NO:1, the signal peptide of the human ActRIIA precursor polypeptide located at amino acid positions 1 to 20; the extracellular domain is located at amino acid positions 21 to 135 and the N-linked glycosylation sites of the human ActRIIA precursor polypeptide (SEQ ID NO:1) are located at amino acid positions 43 and 56 of SEQ ID NO:1. The nucleic acid sequence encoding the human ActRIIB precursor polypeptide of SEQ ID NO:1 is disclosed as SEQ ID NO:4 (nucleotides 164-1705 of Genbank entry NM_001616). The nucleic acid sequence encoding the soluble human ActRIIA polypeptide of SEQ ID NO:2 is disclosed as SEQ ID NO:5. See Table 1 for a description of the sequences.

In specific embodiments, the ActRIIA polypeptides used in the compositions and methods described herein are soluble ActRIIA polypeptides. As used herein, the term "soluble ActRIIA polypeptide" generally refers to polypeptides comprising an extracellular domain of an ActRIIA protein, including any naturally occurring extracellular domain of an ActRIIA protein as well as any variants thereof (including mutants, fragments and peptidomimetic forms). Soluble ActRIIA polypeptides for use with the methods provided herein bind to GDF11 with higher affinity than to activin A. In certain embodiments, soluble ActRIIA polypeptides for use with the methods provided herein bind to GDF11 with higher affinity than to any other member of the TGF-beta superfamily. Native or altered ActRIIA proteins may be given added specificity for GDF11 by coupling them with a second, GDF11-selective binding agent.

In specific embodiments, an ActRIIA polypeptide for use with the methods provided herein is not a polypeptide with the amino acid sequence of SEQ ID NOS:2, 3, 6, 7, 12 and 13 but is at least 80%, 85%, 90%, 95%, 96%, 97%, 98%, or 99% identical to any one of these amino acid sequences.

For synthesis of the ActRIIA polypeptide, the following leader sequences can be used: honey bee mellitin leader sequence (SEQ ID NO:8), the tissue plasminogen activator (TPA) leader (SEQ ID NO:9) or the native ActRIIA leader (SEQ ID NO:10).

In certain embodiments, the GDF11 antagonists used in the compositions and methods described herein comprise a conjugate/fusion protein comprising a GDF11-binding domain of ActRIIA linked to an Fc portion of an antibody. In certain embodiments, the activin-binding domain is linked to an Fc portion of an antibody via a linker, *e.g.,* a peptide linker. Optionally, the Fc domain has one or more mutations at residues such as Asp-265, lysine 322, and Asn-434. In certain cases, the mutant Fc domain having one or more of these mutations (e.g., an Asp-265 mutation) has a reduced ability to bind to the Fcγ receptor relative to a wild-type Fc domain. In other cases, the mutant Fc domain having one or more of these mutations (e.g., an Asn-434 mutation) has an increased ability to bind to the MHC class I- related Fc-receptor (FcRN) relative to a wild-type Fc domain.

In certain embodiments, the GDF11-binding domain of ActRIIA used in the compositions and methods described herein comprise a truncated form of an extracellular domain of ActRIIA. The truncation can be at the carboxy terminus and/or the amino terminus of the ActRIIA polypeptide. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 amino acids long relative to the mature ActRIIB polypeptide extracellular domain. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 N-terminal amino acids of the mature ActRIIA polypeptide extracellular domain. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 C-terminal amino acids of the mature ActRIIA polypeptide extracellular domain. For example, truncated forms of ActRIIA include polypeptides with amino acids 20-119; 20-128; 20-129; 20-130; 20-131; 20-132; 20-133; 20-134; 20-131; 21-131; 22-131; 23-131; 24-131; and 25-131, wherein the amino acid positions refer to the amino acid positions in SEQ ID NO:1.

In certain embodiments, the GDF11-binding domain of ActRIIA used in the compositions and methods described herein comprise an extracellular domain of ActRIIA with one or more amino acid substitutions, additions, and/or deletions. In certain embodiments, the inhibitors of ActRIIA used in the compositions and methods described herein comprise a truncated form of an ActRIIA extracellular domain that also carries an amino acid substitution.

In a specific embodiment, the GDF11 inhibitor to be used in the compositions and methods described herein is a fusion protein between the extracellular domain of the human ActRIIA receptor and the Fc portion of IgG1. In another specific embodiment, the GDF11 inhibitor to be used in the compositions and methods described herein is a fusion protein between a truncated extracellular domain of the human ActRIIA receptor and the Fc portion of IgG1. In another specific embodiment, the GDF11 inhibitor to be used in the compositions and methods described herein is a fusion protein between a truncated extracellular domain of the human ActRIIA receptor and the Fc portion of IgG1, wherein the truncated extracellular domain of the human ActRIIA receptor possesses one or more amino acid substitutions.

Functionally active fragments of ActRIIA polypeptides can be obtained, for example, by screening polypeptides recombinantly produced from the corresponding fragment of the nucleic acid encoding an ActRIIA polypeptide. In addition, fragments can be chemically synthesized using techniques known in the art such as conventional Merrifield solid phase f-Moc or t-Boc chemistry. The fragments can be produced (recombinantly or by chemical synthesis) and tested to identify those peptidyl fragments that can function as antagonists (inhibitors) of ActRIIA protein or signaling mediated by activin.

In addition, functionally active variants of ActRIIA polypeptides can be obtained, for example, by screening libraries of modified polypeptides recombinantly produced from the corresponding mutagenized nucleic acids encoding an ActRIIA polypeptide. The variants can be produced and tested to identify those that can function as antagonists (inhibitors) of GDF11 signaling or binding to ActRIIA. In certain embodiments, a functional variant (i.e., with GDF11-binding activity) of the ActRIIA polypeptides comprises an amino acid sequence that is at least 75% identical to an amino acid sequence selected from SEQ ID NOS:2 or 3. In certain cases, the functional variant has an amino acid sequence at least 80%, 85%, 90%, 95%, 97%, 98%, 99% or 100% identical to an amino acid sequence selected from SEQ ID NOS:2 or 3.

There are many ways by which the library of potential homologs can be generated from a degenerate oligonucleotide sequence. Chemical synthesis of a degenerate gene sequence can be carried out in an automatic DNA synthesizer, and the synthetic genes then be ligated into an appropriate vector for expression. The synthesis of degenerate oligonucleotides is well known in the art (see for example, Narang, S A (1983) Tetrahedron 39:3; Itakura et al., (1981) Recombinant DNA, Proc. 3rd Cleveland Sympos. Macromolecules, ed. AG Walton, Amsterdam: Elsevier pp 273-289; Itakura et al., (1984) Annu. Rev. Biochem. 53:323; Itakura et al., (1984) Science 198:1056; Ike et al., (1983) Nucleic Acid Res. 11:477). Such techniques have been employed in the directed evolution of other proteins (see, for example, Scott et al., (1990) Science 249:386-390; Roberts et al., (1992) PNAS USA 89:2429-2433; Devlin et al., (1990) Science 249: 404-406; Cwirla et al., (1990) PNAS USA 87: 6378-6382; as well as U.S. Pat. Nos. 5,223,409, 5,198,346, and 5,096,815).

Alternatively, other forms of mutagenesis can be utilized to generate a combinatorial library. For example, ActRIIA polypeptide variants can be generated and isolated from a library by screening using, for example, alanine scanning mutagenesis and the like (Ruf et al., (1994) Biochemistry 33:1565-1572; Wang et al., (1994) J. Biol. Chem. 269:3095-3099; Balint et al., (1993) Gene 137:109-118; Grodberg et al., (1993) Eur. J. Biochem. 218:597-601; Nagashima et al., (1993) J. Biol. Chem. 268:2888-2892; Lowman et al., (1991) Biochemistry 30:10832-10838; and Cunningham et al., (1989) Science 244:1081-1085), by linker scanning mutagenesis (Gustin et al., (1993) Virology 193:653-660; Brown et al., (1992) Mol. Cell Biol. 12:2644-2652; McKnight et al., (1982) Science 232:316); by saturation mutagenesis (Meyers et al., (1986) Science 232:613); by PCR mutagenesis (Leung et al., (1989) Method Cell Mol Biol 1:11-19); or by random mutagenesis, including chemical mutagenesis, etc. (Miller et al., (1992) A Short Course in Bacterial Genetics, CSHL Press, Cold Spring Harbor, N.Y.; and Greener et al., (1994) Strategies in Mol Biol 7:32-34). Linker scanning mutagenesis, particularly in a combinatorial setting, is an attractive method for identifying truncated (bioactive) forms of ActRIIA polypeptides.

A wide range of techniques are known in the art for screening gene products of combinatorial libraries made by point mutations and truncations, and, for that matter, for screening cDNA libraries for gene products having a certain property. Such techniques will be generally adaptable for rapid screening of the gene libraries generated by the combinatorial mutagenesis of ActRIIA polypeptides. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Preferred assays include GDF11 binding assays and activin-mediated cell signaling assays.

In certain aspects, functional variants or modified forms of the ActRIIA polypeptides include fusion proteins having at least a portion of the ActRIIA polypeptides and one or more fusion domains. Well known examples of such fusion domains include, but are not limited to, polyhistidine, Glu-Glu, glutathione S transferase (GST), thioredoxin, protein A, protein G, an immunoglobulin heavy chain constant region (Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt-conjugated resins are used. Many of such matrices are available in "kit" form, such as the Pharmacia GST purification system and the QIAexpress.TM. system (Qiagen) useful with (HIS₆) fusion partners. As another example, a fusion domain may be selected so as to facilitate detection of the ActRIIA polypeptides. Examples of such detection domains include the various fluorescent proteins (e.g., GFP) as well as "epitope tags," which are usually short peptide sequences for which a specific antibody is available. Well known epitope tags for which specific monoclonal antibodies are readily available include FLAG, influenza virus hemagglutinin (HA), and c-myc tags. In some cases, the fusion domains have a protease cleavage site, such as for Factor Xa or Thrombin, which allows the relevant protease to partially digest the fusion proteins and thereby liberate the recombinant proteins therefrom. The liberated proteins can then be isolated from the fusion domain by subsequent chromatographic separation. In certain embodiments, an ActRIIA polypeptide is fused with a domain that stabilizes the ActRIIA polypeptide in vivo (a "stabilizer" domain). By "stabilizing" is meant anything that increases serum half life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Likewise, fusions to human serum albumin can confer desirable properties. Other types of fusion domains that may be selected include multimerizing (e.g., dimerizing, tetramerizing) domains and functional domains (that confer an additional biological function, such as further stimulation of bone growth or muscle growth, as desired).

It is understood that different elements of the fusion proteins may be arranged in any manner that is consistent with the desired functionality. For example, an ActRIIA polypeptide may be placed C-terminal to a heterologous domain, or, alternatively, a heterologous domain may be placed C-terminal to an ActRIIA polypeptide. The ActRIIA polypeptide domain and the heterologous domain need not be adjacent in a fusion protein, and additional domains or amino acid sequences may be included C- or N-terminal to either domain or between the domains.

In certain embodiments, the ActRIIA polypeptides used in the methods and compositions described herein contain one or more modifications that are capable of stabilizing the ActRIIA polypeptides. For example, such modifications enhance the in vitro half life of the ActRIIA polypeptides, enhance circulatory half life of the ActRIIA polypeptides or reduce proteolytic degradation of the ActRIIA polypeptides. Such stabilizing modifications include, but are not limited to, fusion proteins (including, for example, fusion proteins comprising an ActRIIA polypeptide and a stabilizer domain), modifications of a glycosylation site (including, for example, addition of a glycosylation site to an ActRIIA polypeptide), and modifications of carbohydrate moiety (including, for example, removal of carbohydrate moieties from an ActRIIA polypeptide). In the case of fusion proteins, an ActRIIA polypeptide is fused to a stabilizer domain such as an IgG molecule (e.g., an Fc domain). As used herein, the term "stabilizer domain" not only refers to a fusion domain (e.g., Fc) as in the case of fusion proteins, but also includes nonproteinaceous modifications such as a carbohydrate moiety, or nonproteinaceous polymer, such as polyethylene glycol.

In certain embodiments, isolated and/or purified forms of the ActRIIA polypeptides, which are isolated from, or otherwise substantially free of, other proteins can be used with the methods and compositions described herein. ActRIIA polypeptides will generally be produced by expression from recombinant nucleic acids.

In certain aspects, isolated and/or recombinant nucleic acids can be used to generate any of the ActRIIA polypeptides (e.g., soluble ActRIIA polypeptides), including fragments, functional variants and fusion proteins used in the methods and compositions disclosed herein.

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures, see Molecular Cloning A Laboratory Manual, 3rd Ed., ed. by Sambrook, Fritsch and Maniatis (Cold Spring Harbor Laboratory Press, 2001). In some instances, it may be desirable to express the recombinant polypeptides by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the .beta.-gal containing pBlueBac III).

Vectors can be designed for production of the subject ActRIIA polypeptides in CHO cells, such as a Pcmv-Script vector (Stratagene, La Jolla, Calif.), pcDNA4 vectors (Invitrogen, Carlsbad, Calif.) and pCI-neo vectors (Promega, Madison, Wis.). As will be apparent, the subject gene constructs can be used to cause expression of the subject ActRIIA polypeptides in cells propagated in culture, e.g., to produce proteins, including fusion proteins or variant proteins, for purification.

Accordingly, provided herein are methods of producing the ActRIIA polypeptides. For example, a host cell transfected with an expression vector encoding an ActRIIA polypeptide can be cultured under appropriate conditions to allow expression of the ActRIIA polypeptide to occur. The ActRIIA polypeptide may be secreted and isolated from a mixture of cells and medium containing the ActRIIA polypeptide. Alternatively, the ActRIIA polypeptide may be retained cytoplasmically or in a membrane fraction and the cells harvested, lysed and the protein isolated. A cell culture includes host cells, media and other byproducts. Suitable media for cell culture are well known in the art. The subject ActRIIA polypeptides can be isolated from cell culture medium, host cells, or both, using techniques known in the art for purifying proteins, including ion-exchange chromatography, gel filtration chromatography, ultrafiltration, electrophoresis, immunoaffinity purification with antibodies specific for particular epitopes of the ActRIIA polypeptides and affinity purification with an agent that binds to a domain fused to the ActRIIA polypeptide (e.g., a protein A column may be used to purify an ActRIIA-Fc fusion). In one embodiment, the ActRIIA polypeptide is a fusion protein containing a domain which facilitates its purification. In one embodiment, purification is achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. As demonstrated herein, ActRIIA-hFc protein was purified to a purity of >98% as determined by size exclusion chromatography and >95% as determined by SDS PAGE. This level of purity was sufficient to achieve desirable effects on bone in mice and an acceptable safety profile in mice, rats and non-human primates.

In another embodiment, a fusion gene coding for a purification leader sequence, such as a poly-(His)/enterokinase cleavage site sequence at the N-terminus of the desired portion of the recombinant ActRIIA polypeptide, can allow purification of the expressed fusion protein by affinity chromatography using a Ni²⁺ metal resin. The purification leader sequence can then be subsequently removed by treatment with enterokinase to provide the purified ActRIIA polypeptide (e.g., see Hochuli et al., (1987) J. Chromatography 411:177; and Janknecht et al., PNAS USA 88:8972).

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence (see, for example, Current Protocols in Molecular Biology, eds. Ausubel et al., John Wiley & Sons: 1992).

ActRIIA-Fc fusion protein can be expressed in stably transfected CHO-DUKX B11 cells from a pAID4 vector (SV40 ori/enhancer, CMV promoter), using a tissue plasminogen leader sequence of SEQ ID NO:9. The Fc portion is a human IgG1 Fc sequence, as shown in SEQ ID NO:7. In certain embodiments, upon expression, the protein contained has, on average, between about 1.5 and 2.5 moles of sialic acid per molecule of ActRIIA-Fc fusion protein.

In certain embodiments, the long serum half-life of an ActRIIA-Fc fusion can be 25-32 days in human patients. Additionally, the CHO cell expressed material can have a higher affinity for activin B ligand than that reported for an ActRIIA-hFc fusion protein expressed in human 293 cells (del Re et al., J Biol Chem. 2004 Dec 17;279(51):53126-35). Additionally, without being bound by theory, the use of the TPA leader sequence provided greater production than other leader sequences and, unlike ActRIIA-Fc expressed with a native leader, may provide a highly pure N-terminal sequence. Use of the native leader sequence may result in two major species of ActRIIA-Fc, each having a different N-terminal sequence.

### (b) ACTRIIB

As used herein, the term "ActRIIB" refers to a family of activin receptor type IIb (ActRIIB) proteins from any species and variants derived from such ActRIIB proteins by mutagenesis or other modification. Reference to ActRIIB herein is understood to be a reference to any one of the currently identified forms. Members of the ActRIIB family are generally transmembrane proteins, composed of a ligand-binding extracellular domain with a cysteine-rich region, a transmembrane domain, and a cytoplasmic domain with predicted serine/threonine kinase activity.

The term "ActRIIB polypeptide" includes polypeptides comprising any naturally occurring polypeptide of an ActRIIB family member as well as any variants thereof (including mutants, fragments, fusions, and peptidomimetic forms) that retain a useful activity. For example, ActRIIB polypeptides include polypeptides derived from the sequence of any known ActRIIB having a sequence at least about 80% identical to the sequence of an ActRIIB polypeptide, and optionally at least 85%, 90%, 95%, 97%, 98%, 99% or greater identity. Examples of ActRIIB polypeptides include human ActRIIB precursor polypeptide (SEQ ID NOS:16 or 28) and soluble human ActRIIB polypeptides. The numbering of amino acids for all of the ActRIIB-related polypeptides described herein is based on the amino acid numbering of SEQ ID NOS:16 or 28. With respect to the ActRIIB precursor polypeptide whose amino acid sequence is depicted at SEQ ID NOS:16 and 28, the signal peptide of the human ActRIIB precursor polypeptide located at amino acid positions 1-18; the extracellular domain is located at amino acid positions 19 to 134 and the N-linked glycosylation sites of the human ActRIIB precursor polypeptide are located at amino acid positions 42 and 65. The nucleic acid sequence encoding the human ActRIIB precursor polypeptide of SEQ ID NO:16 is disclosed as SEQ ID NO:19. See Table 1 for a description of the sequences.

In specific embodiments, the ActRIIB polypeptides used in the compositions and methods described herein are soluble ActRIIB polypeptides. As used herein, the term "soluble ActRIIB polypeptide" generally refers to polypeptides comprising an extracellular domain of an ActRIIB protein, including any naturally occurring extracellular domain of an ActRIIB protein as well as any variants thereof (including mutants, fragments and peptidomimetic forms). Soluble ActRIIB polypeptides for use with the methods provided herein bind to GDF11 with higher affinity than to activin A. In certain embodiments, soluble ActRIIB polypeptides for use with the methods provided herein bind to GDF11 with higher affinity than to any other member of the TGF-beta superfamily. Native or altered ActRIIB proteins may be given added specificity for GDF11 by coupling them with a second, GDF11-selective binding agent.

In specific embodiments, an ActRIIB polypeptide for use with the methods provided herein is not a polypeptide with the amino acid sequence of SEQ ID NOS:17, 18, 23 to 26, 27, 29, 30, 31, 32, 33, 36, 37, 42, and 43 but is at least 80%, 85%, 90%, 95%, 96%, 97%, 98%, or 99% identical to any one of these amino acid sequences. Methods for modifying ActRIIB polypeptides are described, *e.g*., in US Application Publication Nos. 2009/0005308 and 2010/0068215, and international patent application publication nos. WO 2006/012627 and WO 2010/019261 (the disclosures of these references are incorporated herein in their entireties).

In certain embodiments, an ActRIIB polypeptide for use with the methods provided herein has an arginine at position 64 of the ActRIIB precursor amino acid sequence.

It has been shown that a deletion of the proline knot at the C-terminus of the extracellular domain of ActRIIB reduces the affinity of the receptor for activin (see, e.g., Attisano et al., Cell, 1992, 68(1):97-108). An ActRIIB-Fc fusion protein containing amino acids 20-119 of SEQ ID NO: 28 (i.e., SEQ ID NO:32), "ActRIIB(20-119)-Fc" has reduced binding to GDF-11 and activin relative to an ActRIIB-Fc fusion protein containing amino acids 20-134 of SEQ ID NO: 28 (i.e., SEQ ID NO:31), "ActRIIB(20-134)-Fc", which includes the proline knot region and the complete juxtamembrane domain. However, an ActRIIB-Fc fusion protein containing amino acids 20-129 of SEQ ID NO: 28, "ActRIIB(20-129)-Fc" retains similar but somewhat reduced activity relative to the non-truncated extracellular domain of ActRIIB, even though the proline knot region is disrupted. Thus, ActRIIB polypeptides comprising extracellular domains that stop at amino acid 134, 133, 132, 131, 130 and 129 of SEQ ID NO: 28 (or SEQ ID NO:16) are all expected to be active, but constructs stopping at amino acid 134 or 133 may be most active. Similarly, mutations at any of residues 129-134 are not expected to alter ligand binding affinity by large margins, as indicated by the fact that mutations of P129 and P130 of SEQ ID NO:28 do not substantially decrease ligand binding. Therefore, the ActRIIB polypeptides used in accordance with the methods and compositions described herein may end as early as amino acid 109 (i.e., the final cysteine) of SEQ ID NO:28 (or SEQ ID NO:16), however, forms ending at or between amino acid positions 109 and 119 of SEQ ID NO:28 (or SEQ ID NO:16) are expected to have reduced ligand binding ability.

In certain embodiments, amino acid 29 of SEQ ID NO:16 and SEQ ID NO:28 represents the initial cysteine in the ActRIIB precursor sequence. It is expected that an ActRIIB polypeptide beginning at amino acid 29 of the N-terminus of SEQ ID NO:16 or SEQ ID NO:28, or before these amino acid positions, will retain ligand binding activity. An alanine to asparagine mutation at position 24 of SEQ ID NO:16 or SEQ ID NO:28 introduces an N-linked glycosylation sequence without substantially affecting ligand binding. This confirms that mutations in the region between the signal cleavage peptide and the cysteine cross-linked region, corresponding to amino acids 20-29 of SEQ ID NO:16 or SEQ ID NO:28, are well tolerated. In particular, ActRIIB polypeptides beginning at amino acid position 20, 21, 22, 23 and 24 of SEQ ID NO:16 or SEQ ID NO:28 will retain activity, and ActRIIB polypeptides beginning at amino acid positions 25, 26, 27, 28 and 29 of SEQ ID NO:16 or SEQ ID NO:28 are also expected to retain activity. An ActRIIB polypeptide beginning at amino acid position 22, 23, 24 or 25 of SEQ ID NO:16 or SEQ ID NO:28 will have the most activity.

In certain embodiments, the active portions (i.e., ActRIIB polypeptides) of the ActRIIB precursor protein (i.e., SEQ ID NO:16 or SEQ ID NO:28) to be used in accordance with the methods and compositions described herein will generally comprise amino acids 29-109 of SEQ ID NO:16 or SEQ ID NO:28, and such ActRIIB polypeptides may, for example, begin at a residue corresponding to any one of amino acids 19-29 of SEQ ID NO:16 or SEQ ID NO:28 and end at a position corresponding to any one of amino acids 109-134 of SEQ ID NO:16 or SEQ ID NO:28. Specific examples of ActRIIB polypeptides encompassed herein include those that begin at an amino acid position from 19-29, 20-29 or 21-29 of SEQ ID NO:16 or SEQ ID NO:28 and end at an amino acid position from 119-134, 119-133 or 129-134, 129-133 of SEQ ID NO:16 or SEQ ID NO:28. Other specific examples of ActRIIB polypeptides encompassed herein include those that begin at an amino acid position from 20-24 (or 21-24, or 22-25) of SEQ ID NO:16 or SEQ ID NO:28 and end at an amino acid position from 109-134 (or 109-133), 119-134 (or 119-133) or 129-134 (or 129-133) of SEQ ID NO:16 or SEQ ID NO:28. Variant ActRIIB polypeptides falling within these ranges are also contemplated, particularly those having at least 80%, 85%, 90%, 91%, 92%, 93%, 94%, 95%, 96%, 97%, 98%, or 99% sequence identity or sequence homology to the corresponding portion of SEQ ID NO:16 or SEQ ID NO:28.

For example, truncated forms of ActRIIB include polypeptides with amino acids 20-119; 20-128; 20-129; 20-130; 20-131; 20-132; 20-133; 20-134; 20-131; 21-131; 22-131; 23-131; 24-131; and 25-131, wherein the amino acid positions refer to the amino acid positions in SEQ ID NO:16 or SEQ ID NO:28.

Additional exemplary truncated forms of ActRIIB include (i) polypeptides beginning at amino acids at any of amino acids 21-29 of SEQ ID NO:16 or SEQ ID NO:28 (optionally beginning at 22-25 of SEQ ID NO:16 or SEQ ID NO:28) and ending at any of amino acids 109-134 of SEQ ID NO:16 or SEQ ID NO:28; (ii) polypeptides beginning at any of amino acids 20-29 of SEQ ID NO:16 or SEQ ID NO:28 (optionally beginning at 22-25 of SEQ ID NO:16 or SEQ ID NO:28) and ending at any of amino acids 109-133 of SEQ ID NO:16 or SEQ ID NO:28; (iii) polypeptides beginning at any of amino acids 20-24 of SEQ ID NO:16 or SEQ ID NO:28 (optionally beginning at 22-25 of SEQ ID NO:16 or SEQ ID NO:28) and ending at any of amino acids 109-133 of SEQ ID NO:16 or SEQ ID NO:28; (iv) polypeptides beginning at any of amino acids 21-24 of SEQ ID NO:16 or SEQ ID NO:28 and ending at any of amino acids 109-134 of SEQ ID NO:16 or SEQ ID NO:28; (v) polypeptides beginning at any of amino acids 20-24 of SEQ ID NO:16 or SEQ ID NO:28 and ending at any of amino acids 118-133 of SEQ ID NO:16 or SEQ ID NO:28; (vi) polypeptides beginning at any of amino acids 21-24 of SEQ ID NO:16 or SEQ ID NO:28 and ending at any of amino acids 118-134 of SEQ ID NO:16 or SEQ ID NO:28; (vii) polypeptides beginning at any of amino acids 20-24 of SEQ ID NO:16 or SEQ ID NO:28 and ending at any of amino acids 128-133 of SEQ ID NO:16 or SEQ ID NO:28; (viii) polypeptides beginning at any of amino acids 20-24 of SEQ ID NO:16 or SEQ ID NO:28 and ending at any of amino acids 128-133 of SEQ ID NO:16 or SEQ ID NO:28; (ix) polypeptides beginning at any of amino acids 21-29 of SEQ ID NO:16 or SEQ ID NO:28 and ending at any of amino acids 118-134 of SEQ ID NO:16 or SEQ ID NO:28; (x) polypeptides beginning at any of amino acids 20-29 of SEQ ID NO:16 or SEQ ID NO:28 and ending at any of amino acids 118-133 of SEQ ID NO:16 or SEQ ID NO:28; (xi) polypeptides beginning at any of amino acids 21-29 of SEQ ID NO:16 or SEQ ID NO:28 and ending at any of amino acids 128-134 of SEQ ID NO:16 or SEQ ID NO:28; and (xii) polypeptides beginning at any of amino acids 20-29 of SEQ ID NO:16 or SEQ ID NO:28 and ending at any of amino acids 128-133 of SEQ ID NO:16 or SEQ ID NO:28. In a specific embodiment, an ActRIIB polypeptides comprises, consists essentially of, or consists of, an amino acid sequence beginning at amino acid position 25 of SEQ ID NO:16 or SEQ ID NO:28 and ending at amino acid position 131 of SEQ ID NO:16 or SEQ ID NO:28. In another specific embodiment, an ActRIIB polypeptide consists of, or consists essentially of, the amino acid sequence of SEQ ID NO:17, 18, 23, 26, 27, 29, 30, 31, 32, 33, 36, 37, 42, or 43.

For synthesis of the ActRIIB polypeptide, the following leader sequences can be used: honey bee mellitin leader sequence (SEQ ID NO: 8), the tissue plasminogen activator (TPA) leader (SEQ ID NO: 9) or the native ActRIIB leader (SEQ ID NO: 10).

In certain embodiments, the GDF11 antagonists used in the compositions and methods described herein comprise a conjugate/fusion protein comprising a GDF11-binding domain of ActRIIB linked to an Fc portion of an antibody. In certain embodiments, the activin-binding domain is linked to an Fc portion of an antibody via a linker, *e.g*., a peptide linker. Optionally, the Fc domain has one or more mutations at residues such as Asp-265, lysine 322, and Asn-434. In certain cases, the mutant Fc domain having one or more of these mutations (e.g., an Asp-265 mutation) has a reduced ability to bind to the Fcγ receptor relative to a wild-type Fc domain. In other cases, the mutant Fc domain having one or more of these mutations (e.g., an Asn-434 mutation) has an increased ability to bind to the MHC class I- related Fc-receptor (FcRN) relative to a wild-type Fc domain.

In certain embodiments, the GDF11-binding domain of ActRIIB used in the compositions and methods described herein comprise a truncated form of an extracellular domain of ActRIIB. The truncation can be at the carboxy terminus and/or the amino terminus of the ActRIIB polypeptide. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 amino acids long relative to the mature ActRIIB polypeptide extracellular domain. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 N-terminal amino acids of the mature ActRIIB polypeptide extracellular domain. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 C-terminal amino acids of the mature ActRIIB polypeptide extracellular domain.

In certain embodiments, the GDF11-binding domain of ActRIIB used in the compositions and methods described herein comprise an extracellular domain of ActRIIB with one or more amino acid substitutions, additions, and/or deletions. In certain embodiments, the inhibitors of ActRIIB used in the compositions and methods described herein comprise a truncated form of an ActRIIB extracellular domain that also carries an amino acid substitution.

In a specific embodiment, the GDF11 inhibitor to be used in the compositions and methods described herein is a fusion protein between the extracellular domain of the human ActRIIB receptor and the Fc portion of IgG1. In another specific embodiment, the GDF11 inhibitor to be used in the compositions and methods described herein is a fusion protein between a truncated extracellular domain of the human ActRIIB receptor and the Fc portion of IgG1. In another specific embodiment, the GDF11 inhibitor to be used in the compositions and methods described herein is a fusion protein between a truncated extracellular domain of the human ActRIIB receptor and the Fc portion of IgG1, wherein the truncated extracellular domain of the human ActRIIB receptor possesses one or more amino acid substitutions.

Functionally active fragments of ActRIIB polypeptides can be obtained, for example, by screening polypeptides recombinantly produced from the corresponding fragment of the nucleic acid encoding an ActRIIB polypeptide. In addition, fragments can be chemically synthesized using techniques known in the art such as conventional Merrifield solid phase f-Moc or t-Boc chemistry. The fragments can be produced (recombinantly or by chemical synthesis) and tested to identify those peptidyl fragments that can function as antagonists (inhibitors) of ActRIIB protein or signaling mediated by activin.

In addition, functionally active variants of ActRIIB polypeptides can be obtained, for example, by screening libraries of modified polypeptides recombinantly produced from the corresponding mutagenized nucleic acids encoding an ActRIIB polypeptide. The variants can be produced and tested to identify those that can function as antagonists (inhibitors) of GDF11 signaling or binding to ActRIIB. In certain embodiments, a functional variant (i.e., with GDF11-binding activity) of the ActRIIB polypeptides comprises an amino acid sequence that is at least 75% identical to the extracellular domain of ActRIIB. In certain cases, the functional variant has an amino acid sequence at least 80%, 85%, 90%, 95%, 97%, 98%, 99% or 100% identical to an amino acid sequence selected from SEQ ID NOs: to the extracellular domain of ActRIIB.

There are many ways by which the library of potential homologs can be generated from a degenerate oligonucleotide sequence. Chemical synthesis of a degenerate gene sequence can be carried out in an automatic DNA synthesizer, and the synthetic genes then be ligated into an appropriate vector for expression. The synthesis of degenerate oligonucleotides is well known in the art (see for example, Narang, S A (1983) Tetrahedron 39:3; Itakura et al., (1981) Recombinant DNA, Proc. 3rd Cleveland Sympos. Macromolecules, ed. AG Walton, Amsterdam: Elsevier pp 273-289; Itakura et al., (1984) Annu. Rev. Biochem. 53:323; Itakura et al., (1984) Science 198:1056; Ike et al., (1983) Nucleic Acid Res. 11:477). Such techniques have been employed in the directed evolution of other proteins (see, for example, Scott et al., (1990) Science 249:386-390; Roberts et al., (1992) PNAS USA 89:2429-2433; Devlin et al., (1990) Science 249: 404-406; Cwirla et al., (1990) PNAS USA 87: 6378-6382; as well as U.S. Pat. Nos. 5,223,409, 5,198,346, and 5,096,815).

Alternatively, other forms of mutagenesis can be utilized to generate a combinatorial library. For example, ActRIIB polypeptide variants can be generated and isolated from a library by screening using, for example, alanine scanning mutagenesis and the like (Ruf et al., (1994) Biochemistry 33:1565-1572; Wang et al., (1994) J. Biol. Chem. 269:3095-3099; Balint et al., (1993) Gene 137:109-118; Grodberg et al., (1993) Eur. J. Biochem. 218:597-601; Nagashima et al., (1993) J. Biol. Chem. 268:2888-2892; Lowman et al., (1991) Biochemistry 30:10832-10838; and Cunningham et al., (1989) Science 244:1081-1085), by linker scanning mutagenesis (Gustin et al., (1993) Virology 193:653-660; Brown et al., (1992) Mol. Cell Biol. 12:2644-2652; McKnight et al., (1982) Science 232:316); by saturation mutagenesis (Meyers et al., (1986) Science 232:613); by PCR mutagenesis (Leung et al., (1989) Method Cell Mol Biol 1:11-19); or by random mutagenesis, including chemical mutagenesis, etc. (Miller et al., (1992) A Short Course in Bacterial Genetics, CSHL Press, Cold Spring Harbor, N.Y.; and Greener et al., (1994) Strategies in Mol Biol 7:32-34). Linker scanning mutagenesis, particularly in a combinatorial setting, is an attractive method for identifying truncated (bioactive) forms of ActRIIB polypeptides.

A wide range of techniques are known in the art for screening gene products of combinatorial libraries made by point mutations and truncations, and, for that matter, for screening cDNA libraries for gene products having a certain property. Such techniques will be generally adaptable for rapid screening of the gene libraries generated by the combinatorial mutagenesis of ActRIIB polypeptides. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Preferred assays include GDF11 binding assays and activin-mediated cell signaling assays.

In certain aspects, functional variants or modified forms of the ActRIIB polypeptides include fusion proteins having at least a portion of the ActRIIB polypeptides and one or more fusion domains. Well known examples of such fusion domains include, but are not limited to, polyhistidine, Glu-Glu, glutathione S transferase (GST), thioredoxin, protein A, protein G, an immunoglobulin heavy chain constant region (Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt-conjugated resins are used. Many of such matrices are available in "kit" form, such as the Pharmacia GST purification system and the QIAexpress.TM. system (Qiagen) useful with (HIS₆) fusion partners. As another example, a fusion domain may be selected so as to facilitate detection of the ActRIIB polypeptides. Examples of such detection domains include the various fluorescent proteins (e.g., GFP) as well as "epitope tags," which are usually short peptide sequences for which a specific antibody is available. Well known epitope tags for which specific monoclonal antibodies are readily available include FLAG, influenza virus hemagglutinin (HA), and c-myc tags. In some cases, the fusion domains have a protease cleavage site, such as for Factor Xa or Thrombin, which allows the relevant protease to partially digest the fusion proteins and thereby liberate the recombinant proteins therefrom. The liberated proteins can then be isolated from the fusion domain by subsequent chromatographic separation. In certain embodiments, an ActRIIB polypeptide is fused with a domain that stabilizes the ActRIIB polypeptide in vivo (a "stabilizer" domain). By "stabilizing" is meant anything that increases serum half life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Likewise, fusions to human serum albumin can confer desirable properties. Other types of fusion domains that may be selected include multimerizing (e.g., dimerizing, tetramerizing) domains and functional domains (that confer an additional biological function, such as further stimulation of bone growth or muscle growth, as desired).

It is understood that different elements of the fusion proteins may be arranged in any manner that is consistent with the desired functionality. For example, an ActRIIB polypeptide may be placed C-terminal to a heterologous domain, or, alternatively, a heterologous domain may be placed C-terminal to an ActRIIB polypeptide. The ActRIIB polypeptide domain and the heterologous domain need not be adjacent in a fusion protein, and additional domains or amino acid sequences may be included C- or N-terminal to either domain or between the domains.

In certain embodiments, the ActRIIB polypeptides used in the methods and compositions described herein contain one or more modifications that are capable of stabilizing the ActRIIB polypeptides. For example, such modifications enhance the in vitro half life of the ActRIIB polypeptides, enhance circulatory half life of the ActRIIB polypeptides or reduce proteolytic degradation of the ActRIIB polypeptides. Such stabilizing modifications include, but are not limited to, fusion proteins (including, for example, fusion proteins comprising an ActRIIB polypeptide and a stabilizer domain), modifications of a glycosylation site (including, for example, addition of a glycosylation site to an ActRIIB polypeptide), and modifications of carbohydrate moiety (including, for example, removal of carbohydrate moieties from an ActRIIB polypeptide). In the case of fusion proteins, an ActRIIB polypeptide is fused to a stabilizer domain such as an IgG molecule (e.g., an Fc domain). As used herein, the term "stabilizer domain" not only refers to a fusion domain (e.g., Fc) as in the case of fusion proteins, but also includes nonproteinaceous modifications such as a carbohydrate moiety, or nonproteinaceous polymer, such as polyethylene glycol.

In certain embodiments, isolated and/or purified forms of the ActRIIB polypeptides, which are isolated from, or otherwise substantially free of, other proteins can be used with the methods and compositions described herein. ActRIIB polypeptides will generally be produced by expression from recombinant nucleic acids.

In certain aspects, isolated and/or recombinant nucleic acids can be used to generate any of the ActRIIB polypeptides (e.g., soluble ActRIIB polypeptides), including fragments, functional variants and fusion proteins used in the methods and compositions described herein. ActRII polypeptides can be expressed as discussed above for ActRIIA polypeptides.

In certain embodiments, the long serum half-life of an ActRIIB-Fc fusion can be 25-32 days in human patients. Additionally, the CHO cell expressed material can have a higher affinity for activin B ligand than that reported for an ActRIIB-hFc fusion protein expressed in human 293 cells. Additionally, without being bound by theory, the use of the TPA leader sequence provided greater production than other leader sequences and, unlike ActRIIB-Fc expressed with a native leader, may provide a highly pure N-terminal sequence. Use of the native leader sequence may result in two major species of ActRIIB-Fc, each having a different N-terminal sequence.

Any of the ActRIIB polypeptides disclosed herein may be produced as a homodimer. Any of the ActRIIB polypeptides disclosed herein may be formulated as a fusion protein having a heterologous portion that comprises a constant region from an IgG heavy chain, such as an Fc domain. Any of the ActRIIB polypeptides disclosed herein may comprise an acidic amino acid at the position corresponding to position 79 of SEQ ID NO:16 or SEQ ID NO:28, optionally in combination with one or more additional amino acid substitutions, deletions or insertions relative to SEQ ID NO:16 or SEQ ID NO:28.

In specific embodiments, the inhibitors of ActRIIB used with the methods provided herein comprise an extracellular domain of ActRIIB with one or more amino acid substitutions/mutations. Such an amino acid substitution/mutation can be, for example, an exchange from the leucine at amino acid position 79 of SEQ ID NO:16 or SEQ ID NO:28 to an acidic amino acid, such as aspartic acid or glutamic acid. For example, position L79 of SEQ ID NO:16 or SEQ ID NO:28 may be altered in ActRIIB extracellular domain polypeptides to confer altered activin-myostatin (GDF-11) binding properties. L79A and L79P mutations reduce GDF-11 binding to a greater extent than activin binding. L79E and L79D mutations retain GDF-11 binding, while demonstrating greatly reduced activin binding. In certain embodiments, an ActRIIB polypeptide to be used with the methods provided herein has the L79E or L79D mutation and additional mutation such that the resulting molecule retains or has an increased binding affinity to GDF11. In certain embodiments, an ActRIIB polypeptide to be used with the methods provided herein does not have the L79E or L79D mutation but is otherwise modified while retaining or having increased binding affinity to GDF11.

It has been demonstrated that the ligand binding pocket of ActRIIB is defined by residues Y31, N33, N35, L38 through T41, E47, E50, Q53 through K55, L57, H58, Y60, S62, K74, W78 through N83, Y85, R87, A92, and E94 through F101 of SEQ ID NO:16 or SEQ ID NO:28. At these positions, it is expected that conservative mutations will be tolerated, although a K74A mutation is well-tolerated, as are R40A, K55A, F82A and mutations at position L79. R40 is a K in Xenopus, indicating that basic amino acids at this position will be tolerated. Q53 is R in bovine ActRIIB and K in Xenopus ActRIIB, and therefore amino acids including R, K, Q, N and H will be tolerated at this position. Thus, a general formula for an ActRIIB polypeptide for use in the methods and compositions described herein is one that comprises amino acids 29-109 of SEQ ID NO:16 or SEQ ID NO:28, but optionally beginning at an amino acid position ranging from 20-24 or 22-25 of SEQ ID NO:16 or SEQ ID NO:28 and ending at an amino acid position ranging from 129-134 of SEQ ID NO:16 or SEQ ID NO:28, and comprising no more than 1, 2, 5, or 15 conservative amino acid changes in the ligand binding pocket, and zero, one or more non-conservative alterations at amino acid positions 40, 53, 55, 74, 79 and/or 82 of SEQ ID NO:16 or SEQ ID NO:28 in the ligand binding pocket. Such an ActRIIB polypeptide may retain greater than 80%, 90%, 95% or 99% sequence identity or sequence homology to the sequence of amino acids 29-109 of SEQ ID NO:16 or SEQ ID NO:28. Sites outside the binding pocket, at which variability may be particularly well tolerated, include the amino and carboxy termini of the extracellular domain of ActRIIB, and positions 42-46 and 65-73. An asparagine to alanine alteration at position 65 of SEQ ID NO:16 or SEQ ID NO:28 (N65A) actually improves ligand binding in the A64 background, and is thus expected to have no detrimental effect on ligand binding in the R64 background. This change probably eliminates glycosylation at N65 in the A64 background, thus demonstrating that a significant change in this region is likely to be tolerated. While an R64A change is poorly tolerated, R64K is well-tolerated, and thus another basic residue, such as H may be tolerated at position 64.

Thus, in certain embodiments, to generate an ActRIIB polypeptide for use with the methods provided herein an amino acid substitution, deletion, or addition is introduced at one or more of the amino acids in the ligand binding pocket of ActRIIB and the resulting molecule is tested for its binding affinity to GDF11. In certain more specific embodiments, the resulting molecule is tested for its binding affinity to GDF11 relative to other members of the TGF beta superfamily. In certain more specific embodiments, the resulting molecule is tested for its binding affinity to GDF11 relative to Activin A.

In specific embodiments, an ActRIIB polypeptide for use with the methods provided herein has a mutation in the ligand binding domain at the positively-charged amino acid residue Asp (D80). In a specific embodiment, the D80 residue is changed to an amino acid residue selected from the group consisting of: an uncharged amino acid residue, a negative amino acid residue, and a hydrophobic amino acid residue.

### 5.3.3 GDF11 PROPEPTIDES

In certain embodiments, GDF11 propeptides are GDF11 antagonists for use in the methods described herein. Any GDF11 propeptide, fragment or analog thereof that binds to and inhibits GDF activity, signal transduction, receptor binding, expression, processing or secretion can be used in the methods described herein. For example, GDF11 propeptide antagonists can inhibit GDF11 activity by preventing the cleavage of GDF11 to its mature form, by forming a complex with a mature GDF11, or by other related mechanisms. In one embodiment, the GDF11 propeptide antagonists are a naturally occurring GDF11 propeptide of SEQ ID NO.50. GDF11 propeptide antagonists can also be any fragments, variants, analogs, homologs, muteins, or mimetics thereof the naturally occurring GDF11 propeptide (SEQ ID NO. 50) that also retain or enhance GDF11-binding ability and GDF11 inhibitory activity. Accordingly, in another embodiment, the GDF11 propeptide antagonists are a modified GDF11 polypeptide having improved pharmokinetic properties such as an increased circulatory half-life or increased protection from proteolytic degradation. Such stabilizing modifications can include fusion to other polypeptide sequences (e.g., Fc region of an IgG or albumin), linkage to a nonproteineous polymer, modification (e.g., addition) of a glycosylation site, modification (e.g., removal) of a carbohydrate moiety, and the like. Examples of such modified GDF11 propeptides are known in the art and disclosed, for example in U.S. Patent No. 7,737,116 and U.S. Patent No. 8,236,751. In one embodiment, the GDF11 propeptide antagonists bind the mature, human form of GDF11, which can be present in equilibrium as a monomer, dimer or latent complex in, for instance, human serum. In another embodiment, the GDF11 antagonists bind the homodimeric active form of GDF11.

In one embodiment, the GDF11 propeptide antagonists are produced and isolated from a biological source. In another embodiment, the GDF11 propeptide antagonists are produced recombinantly or made synthetically by methods well known in the art. In still another embodiment, the GDF11 propeptide antagonists are introduced and/or administered as polynucleotide sequences, from which the GDF11 propeptide would be translated in vivo.

### 5.3.4 GDF11 BINDING PROTEINS

In some embodiments, other GDF11 binding proteins are GDF11 antagonists for use in the methods described herein. For example, follistatin binds GDF11 with high affinity, and antagonizes GDF11 activity in vivo. Thus, in one embodiment, a follistatin GDF11 antagonist for use in the methods is a native protein produced in and isolated from a biological source, a polypeptide recombinantly produced or a synthetically made follistatin polypeptide. The inhibitory follistatin polypeptide can be any fragment, variant or mutant thereof that binds to and inhibits GDF11 activity in vitro and, preferably, in vivo. In certain embodiments, the follistatin polypeptide does not exhibit follistatin biological activity other than GDF11 binding. Specifically, in certain embodiments, the follistatin polypeptide does not exhibit binding to activin.

Genes also exist that share substantial structural and functional homology with follistatin and, consequently, also bind to and inhibit the activity of some TGF-beta family members (e.g., GDF8, bone morphogenic proteins). Thus, in other embodiments, the GDF11 antagonist is a Follistatin-related gene (FLRG), also known as Follistatin-like 3 (FSTL3) and Follistatin-related protein (FSRP). Examples of FLRG polypeptide antagonists are disclosed, for example, in U.S. Patent No. 8,236,751.

In yet another embodiment, the GDF11 antagonist are polypeptides having one or more follistatin domains and thus, bind to and inhibit the activity of GDF11. Proteins comprising a follistatin domain, have an amino acid domain characterized by cysteine rich repeats that typically span 65-90 amino acids and contain 10 conserved cysteine residues. In one embodiment, the GDF11 antagonist is the GDF11-binding protein GDF-associated serum protein-1 (GASP-1) or -2 (GASP-2). Examples of GASP-1 or GASP-2 polypeptide antagonists are disclosed, for example, in U.S. Patent No. 7,572,763.

Any GDF11-binding protein, variant or fragment thereof can be used in the methods described herein, so long as the polypeptide binds to and inhibits GDF11 biological activity. The GDF11-binding protein antagonists can be produced in a biological expression system, by a chemical method, recombinant DNA method, or by any other method know in the art. In another embodiment, the GDF11-binding protein antagonists can be introduced and/or administered as polynucleotide sequence.

### 5.3.5 GDF11 ANALOGS

GDF11 antagonists also include GDF11 analogs that bind to GDF11 and inhibit its activity. In one embodiment, antagonistic GDF11 analogs are dominant-negative GDF11 polypeptides, which encompass variants of GDF11 that bind to (dimerize with) GDF11 and inhibit its cleavage, processing and/or modification, thereby inhibiting its activity. In another embodiment, GDF11 antagonist analogs are non-peptidic. For example, non-peptide GDF11 analogs can be compounds that mimic the binding and function of GDF11 propeptides (GDF11 propeptide peptidomimetic) or mimic the binding and function of a GDF 11-binding protein (GDF11-binding protein peptidomimetic). Without being bound by theory, peptidomimetics molecules mimic elements of the secondary structure of peptides. In one embodiment, then, the GDF11 analogs bind to and inhibit GDF11 of SEQ ID NO:48 or SEQ ID NO:50.

### 5.3.6 GDF11 POLYNUCLEOTIDE ANTAGONISTS

Polynucleotides that inhibit GDF11 expression (e.g., transcription or translation) can also be useful in the methods described herein. In some embodiments, GDF11 antagonists are polynucleotide molecules that encode a GDF11 antisense molecule, an interfering RNA (RNAi), a ribonucleic encoding enzyme (ribozyme), or a triplexing molecule. GDF11 polynucletides antagonists are complementary (e.g., bind with specificity) to GDF11 target nucleotide sequence (e.g., DNA, RNA, mRNA) and can be DNA or RNA and can be coding sequence or inhibitory sequence (e.g., antisense, RNAi). In one embodiment, the GDF11 antagonist polynucleotide is an antisense GDF11 molecule. Antisense molecules are typically at least about 1 to 25 nucleotides in length and are designed to hybridize specifically to GDF11 target sequence. Specific GDF11 antisense nucleotide sequences useful for antisense molecules can be identified by methods well known in the art.

In certain embodiments, siRNA gene silencers using the sequences of the following gene silencers available from Santa Cruz Biotechnology, Inc., Santa Cruz, CA are used: catalog numbers: sc-44724, sc-44725, sc-44724-PR, sc-44725-PR; shRNA: sc-44724-SH, sc-44725-SH, sc-44724-V, and sc-44725-V.

### 5.4 ASSAYS

An antagonist of GDF-11 can be tested with various assays as set forth below. These assays can be used to test and identify GDF11 antagonists. In addition, these assays can be used to follow treatment of an anemic patient or responsiveness of a patient to treatment with a GDF11 antagonist. For example, an *in vitro* cell culture system can be created from cells of a patient to determine responsiveness of a particular patient to treatment with a GDF11 antagonist.

### 5.4.1 Red blood cell levels

RBC count is a count of the actual number of red blood cells per volume of blood and may be included as part of a standard complete blood count. Normally, males have a RBC count of between 4.7 to 6.1 million cells per microliter and females have a RBC count of between 4.2 to 5.4 million cells per microliter. However, thalassemia patients may have a RBC count lower than that normally seen. Thus, determination of the RBC count in an anemia patient, *e.g.,* a thalassemia patient, being treated in accordance with the methods provided herein allows for the determination of the efficacy of such treatment.

### 5.4.2 ERYTHROID COLONY-FORMING UNITS (CFU-E)

CFU-E can be assayed and identified, e.g., in a colony formation assay by the number and morphology of the cells and by the presence or absence of certain cell surface markers. Levels of erythroid colony-forming units can be measured using, e.g., antibody staining followed by flow cytometry analysis (FACs) to evaluate expression of markers, such as differentiation state markers, e.g., Epo receptor, c-Kit (Stem cell factor receptor), transferring receptor (CD71+), CD36 and Ter119 (glycophorin-A associated antigen) (CFU-E cells are Ter119 (glycophorin-A associated antigen)-negative (see, e.g., Terszowsky et al., 2005). Cells at the CFU-E stage express erythropoietin receptor (EpoR) and can be induced to terminally differentiate in vitro in 2-3 days in the presence of only erythropoietin in a culture media. CFU-E cells can be plated on methylcellulose and stained with diaminobenzidine reagent for hemoglobin, and then CFU-E colonies can be counted. By day 2 from the time of plating, each CFU-E colony can yield between 8 and 64 hemoglobinized cells most of which are in their end-stage of erythroid differentiation.

Colony-forming unit assays are known in the art (e.g., MesenCultTM medium, Stem Cell Technologies Inc., Vancouver British Columbia; see also Wu et al. (Wu H, Liu X, Jaenisch R, Lodish HF (1995). "Generation of committed erythroid BFU-E and CFU-E progenitors does not require erythropoietin or the erythropoietin receptor". Cell 83 (1): 59-67; Marley SB, Lewis JL, Goldman JM, Gordon MY (1996)).

### 5.4.3 ERYTHROID BURST-FORMING UNITS (BFU-E)

Similarly to CFU-e, BFU-e can be assayed and identified, e.g., in a colony formation assay by the number and morphology of the cells and by the presence or absence of certain cell surface markers. Specifically, BFU-e can be identified by the expression of several cell surface markers such as CD33, CD34 and HLA-DR, and lack of expression of glycophorin-A. For example, BFU-e assays described in Wu et al. can be utilized (Wu H, Liu X, Jaenisch R, Lodish HF (1995), "Generation of committed erythroid BFU-E and CFU-E progenitors does not require erythropoietin or the erythropoietin receptor." Cell 83 (1): 59-67).

### 5.4.4 HEMATOCRIT

A hematocrit measures the percentage of red blood cells in a given volume of whole blood and may be included as part of a standard complete blood count. The hematocrit is normally about 45% for men and about 40% for women. However, thalassemia patients typically have a hematocrit lower than that normally seen. Thus, determination of the hematocrit in a thalassemia patient being treated in accordance with the methods provided herein allows for the determination of the efficacy of such treatment.

### 5.4.5 APOPTOSIS OF ERYTHROID PROGENITORS

Apoptosis of Erythroid progenitors can be determined, *e.g*., by using Terminal deoxynucleotidyltransferase-mediated dUTP nick end-labeling (TUNEL) staining. TUNEL staining can be performed using an *in situ* Apoptosis Detection Kit (Takara Bio, Otsu, Japan).

### 5.4.6 ERYTHROID CO-CULTURE SYSTEM

To test the effect of an agent on erythroid differentiation in an in vitro environment more analogous to the in vivo environment, a co-culture system of bone marrow cells and human CD36+ cells can be used. Human CD36+ cells, which are highly enriched for erythroid progenitor cells, are co-cultured with long-term bone marrow cultures in erythropoietin-(EPO) supplemented media (2U/mL). After 6 days, the cellular output (e.g., cell type) of the culture can be assessed by, for example, flow cytometry (e.g., FACS) analysis. The numbers of erythroid cells at the various levels of erythroid differentiation (e.g., proerythroblast, basophil, late basophilic/polychromatic, orthochromatic/reticulocytes, glycoprotein A+ cells) indicate the ability of the agent being tested to modulate erythroid differentiation. The assay is conducted in the presence and absence of GDF11. Antagonists of GDF11 can then be tested for their ability to reverse the effect of GDF11.

### 5.4.7 TRANSCRIPTIONAL RESPONSE ASSAY

In certain embodiments, a transcription response assay can be used to test an antagonist of GDF11. Upon GDF11 signaling, transcription of certain genes is up- or downregulated. A cell culture system used and the transcriptional response to GDF11 can be measured (e.g., by RT-PCT). The effect of an antagonist of GDF11 on the transcriptional response is a measure of its effectiveness as an antagonist. For example, in C2C12 cells, runx2/cbfa1 is upregulated as a response to GDF11 (see, e.g., Bessa et al. 2009 Protein Expression and Purification 63:89-94). In certain embodiments, the promoter region that is known to be responsive to GDF11 signaling can be cloned upstream of a reporter gene. In this way, the assay can be simplified such that only the activity of the reporter gene need to be assayed.

### 5.5 PHARMACEUTICAL COMPOSITIONS

In certain embodiments, antagonists of GDF11 can be formulated with a pharmaceutically acceptable carrier for use with the methods provided herein. For example, antagonists of GDF11 can be administered alone or as a component of a pharmaceutical formulation (therapeutic composition). The subject compounds may be formulated for administration in any convenient way for use in human or veterinary medicine.

In certain embodiments, the therapeutic methods described herein include administering the composition systemically, or locally as an implant or device. When administered, the therapeutic composition may be in a pyrogen-free, physiologically acceptable form. Therapeutically useful agents other than the antagonist of GDF11 which may also optionally be included in the composition as described above, may be administered simultaneously or sequentially with the antagonist of GDF11.

Antagonists of GDF11 be administered parenterally. Pharmaceutical compositions suitable for parenteral administration may comprise one or more antagonists of GDF11 in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions described herein include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

Further, the composition may be encapsulated or injected in a form for delivery to a target tissue site (e.g., bone). In certain embodiments, compositions provided herein may include a matrix capable of delivering one or more antagonists of GDF11 to a target tissue site (e.g., bone), providing a structure for the developing tissue and optimally capable of being resorbed into the body. For example, the matrix may provide slow release of the antagonists of GDF11. Such matrices may be formed of materials presently in use for other implanted medical applications.

The choice of matrix material is based on biocompatibility, biodegradability, mechanical properties, cosmetic appearance and interface properties. The particular application of the subject compositions will define the appropriate formulation. Potential matrices for the compositions may be biodegradable and chemically defined calcium sulfate, tricalciumphosphate, hydroxyapatite, polylactic acid and polyanhydrides. Other potential materials are biodegradable and biologically well defined, such as bone or dermal collagen. Further matrices are comprised of pure proteins or extracellular matrix components. Other potential matrices are non-biodegradable and chemically defined, such as sintered hydroxyapatite, bioglass, aluminates, or other ceramics. Matrices may be comprised of combinations of any of the above mentioned types of material, such as polylactic acid and hydroxyapatite or collagen and tricalciumphosphate. The bioceramics may be altered in composition, such as in calcium-aluminate-phosphate and processing to alter pore size, particle size, particle shape, and biodegradability.

In certain embodiments, compositions used in the methods described herein can be administered orally, e.g., in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of an agent as an active ingredient. An agent may also be administered as a bolus, electuary or paste.

In solid dosage forms for oral administration (capsules, tablets, pills, dragees, powders, granules, and the like), one or more therapeutic compounds for use with the methods provided herein may be mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose, and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such as talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

Liquid dosage forms for oral administration include pharmaceutically acceptable emulsions, microemulsions, solutions, suspensions, syrups, and elixirs. In addition to the active ingredient, the liquid dosage forms may contain inert diluents commonly used in the art, such as water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor, and sesame oils), glycerol, tetrahydrofuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, coloring, perfuming, and preservative agents.

Suspensions, in addition to the active compounds, may contain suspending agents such as ethoxylated isostearyl alcohols, polyoxyethylene sorbitol, and sorbitan esters, microcrystalline cellulose, aluminum metahydroxide, bentonite, agar-agar and tragacanth, and mixtures thereof.

The compositions described herein may also contain adjuvants, such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption, such as aluminum monostearate and gelatin.

It is understood that the dosage regimen will be determined by the attending physician considering various factors which modify the action of the antagonists of GDF11. The various factors include, but are not limited to, physiological parameters of the blood (e.g., red blood cell levels, hematocrit, reticulocyte level, hemoglobin levels etc.) and GDF11 levels in the spleen and/or bone marrow. In certain embodiments, an anemic patient to be treated with the methods provided herein has been diagnosed to have elevated GDF11 levels in the spleen, bone marrow, liver, serum, and/or plasma (see Section 5.2). In certain embodiments, GDF11 levels in the spleen, bone marrow, liver, serum, and/or plasma are monitored to adjust the dose of the treatment with the GDF11 antagonist. For example, if a patient has initially elevated GDF11 levels and then 1, 2, 3, 4, 5, 6, 7, 8, 9, 10 days; 1, 2, 3, 4, 5, 6, 7, 8, 9, 10 weeks; 1, 2, 3, 4, 5, 6, 7, 8, 9, or 10 months; after treatment with the GDF11 antagonist started GDF11 levels in the spleen, bone marrow, liver, serum, and/or plasma are measured again and compared to the initial levels. If the GDF11 levels are reduced and close or below normal GDF11 levels, the dose with the GDF11 antagonist can be reduced.

In certain embodiments, and antagonist of GDF11 is specifically targeted to and/or administered to the spleen and/or bone marrow and/or liver of the patient.

In certain embodiments, provided herein is gene therapy for the in vivo production of an antagonist of GDF11. Such therapy would achieve its therapeutic effect by introduction of the antagonists of GDF11 polynucleotide sequences into cells or tissues having the disorders as listed above. Delivery of polynucleotide sequences can be achieved using a recombinant expression vector such as a chimeric virus or a colloidal dispersion system. Specific illustrative therapeutic delivery systems include targeted liposomes, viral vectors including adenovirus, herpes virus, vaccinia, or, preferably, an RNA virus such as a retrovirus. Preferably, the retroviral vector is a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous Sarcoma Virus (RSV). A number of additional retroviral vectors can incorporate multiple genes. All of these vectors can transfer or incorporate a gene for a selectable marker so that transduced cells can be identified and generated. Retroviral vectors can be made target-specific by attaching, for example, a sugar, a glycolipid, or a protein. Preferred targeting is accomplished by using an antibody. Those of skill in the art will recognize that specific polynucleotide sequences can be inserted into the retroviral genome or attached to a viral envelope to allow target specific delivery of the retroviral vector containing the polynucleotide that encodes the antagonist of GDF11. In one embodiment, the vector is targeted to bone marrow and/or spleen.

Another targeted delivery system for polynucleotides is a colloidal dispersion system. Colloidal dispersion systems include macromolecule complexes, nanocapsules, microspheres, beads, and lipid-based systems including oil-in-water emulsions, micelles, mixed micelles, and liposomes. In a specific embodiment, the colloidal system is a liposome. Liposomes are artificial membrane vesicles which are useful as delivery vehicles in vitro and in vivo. RNA, DNA and intact virions can be encapsulated within the aqueous interior and be delivered to cells in a biologically active form (see e.g., Fraley, et al., Trends Biochem. Sci., 6:77, 1981). Methods for efficient gene transfer using a liposome vehicle, are known in the art, see e.g., Mannino, et al., Biotechniques, 6:682, 1988. The composition of the liposome is usually a combination of phospholipids, usually in combination with steroids, especially cholesterol. Other phospholipids or other lipids may also be used. The physical characteristics of liposomes depend on pH, ionic strength, and the presence of divalent cations.

Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Illustrative phospholipids include egg phosphatidylcholine, dipalmitoylphosphatidylcholine, and distearoylphosphatidylcholine. The targeting of liposomes is also possible based on, for example, organ-specificity, cell-specificity, and organelle-specificity and is known in the art.

In certain embodiments, the antagonist of GDF11 is substantially pure in a pharmaceutical composition. Specifically, at most 20%, 10%, 5%, 2.5%, 1%, 0.1%, or at most 0.05% of the compounds in the pharmaceutical composition are compounds other than the antagonists of GDF11 and the pharmaceutical acceptable carrier.

### 6. EXAMPLES

The example presented herein demonstrates that GDF11 protein levels are elevated in thalassemia and that inhibition of GDF11 can treat anemia in a mouse model of beta-thalassemia.

### 6.1 AN ACTRIIA DECOY TREATS beta-THALASSEMIA

Beta-thalassemia is associated with ineffective erythropoiesis, accelerated erythoid differentiation and apoptosis, resulting in anemia and iron overload. The molecular mechanism underlying the effects of ineffective erythropoiesis is incompletely understood. Although members of the TGF-beta superfamily are implicated in both proliferation and differentiation of erythroid progenitor cells, the role of the numerous TGF-beta family members in the ineffective erythropoiesis seen in beta-thalassemia is unknown.

To assess the role of TGF-beta family members in the ineffective erythropoiesis of beta-thalassemia, a recombinant fusion protein that binds a number of TGF-beta superfamily ligands was used in a mouse model of human beta-thalassemia. The ActRIIA-Fc fusion protein (i.e., the murine counterpart of SEQ ID NO:7) consists of the extracellular domain of Activin Receptor IIA (ActRIIA) linked to a human immunoglobulin 1 (IgG1) Fc domain and the protein acts as a ligand trap for TGF-beta family members like activin A, activin B, growth differentiation factor-11 (GDF11) and bone morphogenetic protein-10 (BMP-10). Hbbth1/th1 is a mouse model of human beta-thalassemia, the mice having a naturally occurring deletion of the beta-major gene (Skow et al., 1983). Hbbth1/th1 mice have abnormally low hemoglobin (Hgb), hemocrit (Hct) and mean cell volume (MCV) as well as bone marrow (BM) hypercellularity and abnormally high levels of bilirubin, a by-product of hemoglobin breakdown that represents extensive red blood cell destruction.

### 6.2 MATERIALS AND METHODS

### 6.2.1 Mice

C57BL/6 were bred and housed in the pathogen-free facilities of INSERM U699. All protocols were approved by the Animal Care Committee of INSERM. The Hbbth1/th1 model was originated from a natural occurring deletion of the β-major gene (Skow LC et al; Cell 1983). Hbbth1/th1 mice constitute a model of β-thalassemia intermedia. These mice have several clinical parameters reproducing human β-thalassemia such as ineffective bone marrow erythropoiesis, precursor apoptosis, parenchymal iron distribution, decreased hepcidin expression and lower bone marrow iron levels while levels in the liver and spleen are increased.

### 6.2.2 Complete blood counts

Blood samples were collected in EDTA-coated tubes and complete blood counts were measured on a MS9-5 Blood Analyzer (Melet Schloesing Laboratories) according to the manufacturer's instructions. The selected parameters were red blood cells (RBC), hematocrit (Ht), mean corpuscular volume (MCV), hemoglobin (Hb). Reticulocyte numbers were determined with retic-count reagent (BD Biosciences Retic-Count™ Kit).

### 6.2.3 Quantitative real-time RT-PCR

RNA was extracted from erythroid progenitors using RNeasy Plus Mini Kit (Qiagen). One microgram of total RNA was used for reverse transcription using iScript reverse transcription Supermix (Bio-rad) during 30 minutes at 42°C. The enzyme was then inactivated at 85°C for 5 minutes. For qPCR, the cDNA samples were amplified in a CFX96 PCR System (Bio-rad). The PCR products were quantified using SsoFast EvaGreen Supermix (Bio-rad).

### 6.2.4 In vitro erythroblast cultures

Cells from each tissue were resuspended in serum-free "erythroid expansion media" consisting in either StemPro34 plus nutrient supplement (Life Technologies Gibco-BRL) supplemented with 2 U/mL human recombinant Epo (Roche), 100 ng/mL SCF (PeproTech), 10-6 M dexamethasone (D2915; Sigma), 40 and penicillin/streptomycin (Pen/Strep; Invitrogen). After 5 days of culture, the nonadherent cells were transferred to the differentiation medium (StemPro-34 supplemented with 1U/ml Epo and 1mg/ml ferro-transferrin (Sigma)) during two to three days supplemented or not with 10µg/ml of mActRIIA-Fc. Live and dead cell numbers were determined daily by Trypan Blue (Gibco/BRL) exclusion, and cell concentration was adjusted to 2 × 10⁶ total cells/mL daily through partial medium changes.

### 6.2.5 Methylcellulose assays

Single cell suspensions of adult mouse BM or spleen were mixed with methocult M3434 medium (Stem Cell Technologies), plated into 35 mm dishes and cultured at 37°C under a 5% CO₂ humidified atmosphere. The BFU-E colonies were scored at day 10 (in some experiments mouse BFU-E were scored from day 7 onward to day 10 and there was no difference between colony numbers at day 7 and at day 10).

### 6.2.6 Statistical analyses

Statistical analyses were performed with GraphPad Prism (version 5.0; GraphPad Software). The data are expressed as the mean ± SEM of N determinations unless noted otherwise. Student's t-test or the Mann-Whitney test were used to compare two groups, whereas multigroup comparisons were made using two-way ANOVA test followed by post-hoc analysis (Bonferroni test). Differences were considered significant at a P value less than 0.05 (*), less than 0.01 (**) or less than 0.001 (***).

### 6.2.7 Immunofluorescence analysis by flow cytometry

For bone marrow (BM) and splenocyte suspensions from mice, blocking of IgG receptors was performed with anti-FcgammaR mAb 2.4G2. Cells (1 x 106) were then stained with anti-TER-119 antibody and anti-mouse TfR1 antibody. Stained cells were further analyzed by flow cytometry (FACScalibur; Becton Dickinson) using FlowJo software (Tree Star).

### 6.2.8 Tissue collection and histology

Bone marrow and spleens were collected and fixed in 10% formalin, paraffin embedded, and sectioned at 3-6 µm for hematoxylin and eosin (H&E) staining.

### 6.2.9 Iron, ferritin, bilirubin and transferrin quantification.

Blood was drawn into heparinized tubes and centrifuged (5 min, 4°C, 1,100g). Plasma was de novo centrifuged (5 min, 4 °C, 1,100g) to remove contaminating red blood cells. Biochemical parameters were quantified with an Olympus AU400 automat according to the manufacturer's instructions.

### 6.3 RESULTS

### 6.3.1 mActRIIA-Fc ameliorates hematological parameters in thalassemic mice.

beta-thalassemia is a disease characterized by a defect in hemoglobin synthesis leading to impaired erythrocyte maturation and production. The decrease in erythrocytes is primarily thought to be due to abnormally accelerated erythroid differentiation and apoptosis at the late basophilic/polychromatic erythroblast stage of red blood cell differentiation that results in an overall decrease in mature red blood cell production. The disease is characterized by a hypercellular bone marrow compartment in which abnormal erythroblasts accumulate and undergo apoptosis, resulting in systemic anemia.

To examine the role of TGF-BETA ligands in the disease mechanism of beta-thalassemia, Hbbth1/th1 mice were subcutaneously treated with mActRIIA-Fc (the murine counterpart of SEQ ID NO:7) or PBS for 0, 5, 10, 30 or 60 days (10mg/Kg body weight) twice a week (*p <0.05, N=3-5 for each independent experiment). Compared to PBS-treated animals, treatment with mActRIIA-Fc significantly increased red blood cell counts (FIG 1A) and hemocrit (FIG 1B) and hemoglobin (FIG 1C) levels, with a concomitant decrease in reticulocyte counts (FIG ID) (from 10 days post-treatment until day 60). Analysis of circulating red blood cell (RBC) parameters also showed that mean corpuscular volume (MCV) (FIG IE), mean corpuscular hemoglobin (MCH) (FIG 1F) and MCH concentration (MCHC) (FIG 1G) all increased in all mice treated with mActRIIA-Fc, suggesting that mActRIIA-Fc improved the microcytic anemia of thalassemia and restored hemoglobin content per RBC. In addition, bone marrow and spleen cellularity and late basophilic/polychromatic erythroblasts were reduced significantly following treatment with mActRIIA-Fc. Morphological analysis of erythrocytes was evaluated by May-Grünwald (MGG) staining and showed a reduction in anisocytosis, poikilocytosis and target cells (FIG 1I) To determine the effect of mActRIIA-Fc on the anemia associated with beta-thalassemia, systemic iron levels (FIG 1J), transferrin synthesis (FIG 1K), transferrin saturation (FIG 1L) and ferritin levels (FIG 1M) were evaluated on thalassemic mice. Transferrin saturation was reduced with the induction of transferrin synthesis or decreased systemic iron levels (FIG 1L). Platelet, monocyte, lymphocyte and neutrophil levels were also assessed (FIG IN).

The effects of mActRIIA-Fc on splenomegaly in thalassemic mice was evaluated by spleen weight and measuring total spleen cell number. Spleen cell number and spleen weight were reduced in animals treated with mActRIIA-Fc compared to PBS-treated thalassemic mice (FIG 1O). Similarly, bone marrow erythroblasts numbers and expansion (as determined by eosin/hematoxylin staining) (FIG IP) were also decreased in mActRIIA-Fc-treated mice. Bone marrow and spleen were harvested and erythroblasts were quantified by flow cytometry by TER119 staining. Treatment with mActRIIA-Fc significantly reduced the number of erythrocytes in thalassemic mice (FIG 1Q), indicating that it corrected the ineffective erythropoiesis in the mice.

### 6.3.2 mActRIIA-Fc reduces ineffective erythropoiesis in thalassemic mice.

To further explore the role of TGF-BETA superfamily ligands in ineffective erythropoiesis in beta-thalassemia, spleen (FIG 2A, FIG 2C) and bone marrow (FIG 2B, FIG 2C) were harvested and erythroblast differentiation evaluated by flow cytometry by CD71/TER119 staining and forward scatter/side scatter (FSC/SSC) distribution. A time course analysis of the percent of cells at progressive stages in erythropoietic differentiation, Proerythroblast (Pro-E), Basophil erythroblasts (Ery-A), late basophilic (Ery-B) and polychromatic erythroblasts and orthochromatic erythroblasts (Ery-C), showed that mice treated with mActRIIA-Fc presented significantly decreased immature TER119/CD71 cells (late basophilic and polychromatic erythroblasts, Ery-B) in spleen with a concomitant increase in the percentage of orthochromatic erythroblasts (Ery-C) (FIG 2A). These results were in accordance with mActRIIA-Fc reduction of ineffective erythropoiesis. Although there was a decrease in TER-199+ erythroblasts and in Ery-B numbers in bone marrow from mice treated with mActRIIA-Fc (FIG 2B), there was no increase in the amount of mature erythroblasts suggesting that ineffective erythropoiesis in the bone marrow of thalassemic mice is not corrected by treatment of mice with mActRIIA-Fc.

Chronic anemia of thalassemia induces stress erythropoiesis compensatory responses. However, these responses are unproductive because of ineffective erythropoiesis. Ineffective erythropoiesis is characterized by a requirement of RBC production which cannot be compensated by an accelerated proliferation and differentiation of immature erythroblasts due to the premature cell death by apoptosis of maturing cells. Therefore, imbalanced immature/mature erythroblasts ratio is a feature of ineffective erythropoiesis of thalassemia. To further study the impact of mActRIIA-Fc on erythroid differentiation and ineffective erythropoiesis, cell suspensions from mActRIIA-Fc-treated mice and their respective controls were labeled with antibodies to TfR1 and TER119. Erythroid precursors differentiation was analyzed by flow cytometry in TER119high gate as previously described (Liu et al., Blood 2006). Accordingly, mActRIIA-Fc-treated mice presented a decreased ratio of immature/mature erythroblasts ratio in spleen indicating the correction of ineffective stress erythropoiesis. In bone marrow the immature/mature erythroblasts ratio did not differ between control and mActRIIA-Fc-treated mice further suggesting that ineffective erythropoiesis in the bone marrow of thalassemic mice was not corrected by mActRIIA-Fc treatment. These results suggest that ActRIIa ligands contribute to erythroblast differentiation but also to ineffective spleen erythropoiesis in β-thalassemia.

Bilirubin is a product of hemoglobin degradation and increased plasma bilirubin resulting from hemolysis is a feature of ineffective erythropoiesis in β-thalassemia 18. In time-course analysis, serum levels of total and direct bilirubin were decreased in thalassemic mice treated with mActRIIA-Fc from 5 days of treatment suggesting that hemolysis resulting from ineffective erythropoiesis was affected by mActRIIA-Fc administration (FIG 2D). In agreement with bilirubin values, levels of serum lactate dehydrogenase (LDH) were also reduced in animals treated with mActRIIA-Fc compared to controls after 60 days of treatment (FIG 2D), further confirming that tissue hemolysis was reduced in mice treated with mActRIIA-Fc.

Late-stage erythropoiesis is largely committed to the production of the oxygen carrier hemoglobin (Hb), a tetrameric protein composed of two α-globin and two β-globin subunits. β-thalassemia is a common inherited hemoglobinopathy characterized by impaired or absent β-globin gene production with subsequent accumulation of unpaired α-subunits. The excess of unbound free α-globin in maturing erythroid cells precipitates and leads to the production of reactive oxygen species (ROS) and cellular oxidative stress damage inducing premature death of erythroid precursors. The impact of mActRIIA-Fc on the generation of globin precipitates was further investigated. ROS generation on primary pro-erythroblast differentiation was evaluated by flow cytometry using dichlorodihydrofluorescein (FIG 2E). Analysis of hemoglobin solubility on primary thalassemic pro-erythroblasts treated for 48 hours with mActRIIA-Fc or PBS (FIG 2F).

To gain insight into the cellular mechanisms associated with treatment with mActRIIA-Fc, spleen-derived proeryhtoblasts were cultured and recovered from Hbbth1/th1 mice in the presence or in the absence of mActRIIA-Fc. A well-established in vitro model of pro-erythroblast differentiation was used (spleen precursors were cultured for 5 days in serum free stem cell expansion medium supplemented with mouse Stem Cell Factor, Epo and dexamethasone). These pro-erythroblast-enriched cultures were then differentiated in the presence of 1U/ml Epo and 1mg/ml Fe-Tf for three days. Similar to in vivo observations, treatment with mActRIIA-Fc increased total amounts of hemoglobin in thalassemic erythroblasts. However, those cells presented decreased amounts of membrane-associated precipitated hemoglobin when compared to control treated cells (FIG 2F). Accordingly, the amount of reactive oxygen species (ROS) detected on those cells decreased in cells treated with mActRIIA-Fc (FIG 2E). Therefore, treatment with mActRIIA-Fc resulted in decreased cytotoxic globin precipitation and its associated ROS production, contributing to ineffective erythropoiesis. Altogether, these data suggested that targeting ActRIIa signaling modulated erythroblast differentiation and trapping of ActRIIa ligands by mActRIIA-Fc corrected ineffective erythropoiesis by favoring the formation of mature erythroblasts and reducing membrane-associated hemoglobin precipitates.

### 6.3.3 mActRIIA-Fc modulates apoptosis in thalassemic mice.

The involvement of TGF-beta family members on the apoptotic process in beta-thalassemia-associated ineffective erythropoiesis was investigated by analyzing the expression of pro-apoptotic proteins on erythrocytes using flow cytometry. Although there were no significant changes in the expression of the apoptotic proteins in bone marrow erythroblasts (FIG 3A), analysis of spleen erythroblasts showed that Fas-L was increased on the Ery-B population of cells (FIG 3B). Multiparametric flow cytometry comparative analysis on spleen cells from mice treated with PBS and mActRIIA-Fc showed that Fas-L expression was increased on immature late basophilic and polychromatic erythroblasts (Ery-B) and decreased on orthochromatic erythroblasts (Ery-C) (FIG 3B). In contrast, Fas-L expression was not modulated on BM erythroblasts (FIG 3A). Together, these results showed that ActRIIa signaling modulated Fas/Fas-L pathway on maturing erythroblasts. Collectively, these data suggested that ActRIIa signaling induced premature cell death of maturing erythroblasts committed to ineffective erythropoiesis. Surprisingly, Fas-L expression was decreased on Ery-A and Ery-C subsets of cells showing that the impact of ActRIIa signaling was more pronounced on maturing erythroblasts (FIG 3B). The number of tunel positive cells was also increased in animals treated with mActRIIA-Fc (FIG 3C). Ineffective erythropoiesis of thalassemia is characterized by a massive apoptosis of maturing erythroblasts. Studying the impact of treatment of mActRIIA-Fc on apoptosis indicated that treatment of mice with mActRIIA-Fc presented decreased numbers of tunnel positive cells compared to their respective controls (FIG 3C), suggesting that ActRIIa signaling trough Smad-2,3 activation could control ineffective erythropoiesis by modulating apoptosis levels of maturing erythroblasts.

### 6.3.4 Activin/GDF11 ligands are overexpressed in spleen from thalassemic mice.

RNA (mRNA) expression levels of ActRII, activin A, activin B and GDF11 were evaluated in spleen from wild-type and thalassemic mice by qPCR. The mRNA levels of ActRII, activin A, activin B and GDF11 were all increased suggesting that mActRIIA-Fc could be acting through one of its ligands in its improvement of ineffective erythropoiesis (FIG 4A). Western blot analysis of protein obtained from the spleen from thalassemic mice treated with mActRIIA-Fc showed a significant decrease in GDF11 protein levels compared to PBS-treated mice (FIG 4B), further implicating GDF11 as the ActRIIA ligand responsible for ineffective erythropoiesis. Further immunohistochemical analysis revealed that GDF11 protein levels (and to a much lesser extent activin A or activin B) were greatly increased on spleen biopsies from thalassemic mice and were abrogated in animals treated with mActRIIA-Fc (FIG 3A). These results were further confirmed by immunoblotting (FIG 4B). In contrast to spleen sections, analysis of ActRIIa ligands on BM showed no accumulation of GDF11 in thalassemic mice (FIG 4B). Therefore, GDF11 overexpression in spleen section of thalassemic mice could be associated with ineffective erythropoiesis.

### 6.3.5 mActRIIA-Fc reduces the increased GDF11 expression levels seen in primary thalassemic pro-erythroblasts.

To determine which TGF-beta family members might be implicated in the treatment of beta-thalassemia with mActRIIA-Fc, immunohistochemical analysis of proteins in the activin/GDF signaling pathway was performed. Thalassemic mice were treated with PBS or mActRIIA-Fc for 30 days and spleen was harvested, fixed and stained for activin A, activin B, GDF8, GDF11, ActRII and p-Smad2 (FIG 5A). Immunohistochemical staining showed increased levels of GDF11, ActRII and p-Smad2 in thalassemic mice. To explore whether proteins in the activin/GDF signaling pathway were overexpressed in other mouse models of anemia, the expression of activin A, activin B and GDF11 in thalassemic mice was compared to normoxia, hypoxia and alphaRBC mice (FIG 5B). FACS analysis of primary thalassemic pro-erythroblasts treated with PBS or mActRIIA-Fc for 48 hours then incubated with specific antibodies against activin A, activin B, the GDF11 propeptide and the GDF8/GDF11 cleaved peptide indicated that mActRIIA-Fc treatment normalized GDF11 expression. Quantification of GDF11 staining indicated that treatment of mice with mActRIIA-Fc significantly reduced GDF11 levels (FIG 5C). This reduction in GDF11 expression upon treatment of thalassemic mice with mActRIIA-Fc was confirmed by immunohistochemical analysis of the spleens of the mice (FIG. 5D). * p<0.05, N=4. Thus, the fact that mActRIIA-Fc decreased GDF11 overexpression in thalassemic tissues was additional evidence that mActRIIA-Fc corrected ineffective erythropoiesis by targeting GDF11.

### 6.3.6 Neutralization of GDF11 restores erythroblast differentiation.

To determine if increased GDF11 expression was responsible for ineffective erythropoiesis, thalassemic pro-erythroblasts were cultured in the presence of blocking antibodies directed against activins A and B and GDF11. Anti-GDFl 1 antibodies (but not activin A and B antibodies) promoted erythropoiesis, further confirming that GDF11 negatively regulated erythropoiesis by inducing ineffective erythropoiesis as quantified by flow cytometry following CD71/TER119 staining (FIG. 6A). ROS generation on primary pro-erythroblast differentiation was evaluated by flow cytometry using dichlorodihydrofluorescein (FIG. 6B). *p<0.05, N=4. Thus, anti-GDFl 1 blocking antibodies restored cell differentiation and reduced hemoglobin aggregates in thalassemic erythroblasts.

### 6.3.7 An ActRIIA ligand-detecting assay

An assay was developed to detect, identify and quantify ActRIIA ligands present in blood serum and, specifically, their abnormal expression in diseases associated with ineffective erythropoiesis (e.g., thalassemia, myelodysplastic syndromes, chronic pernicious anemia and sickle cell anemia). The assay consists of a sandwich ELISA based on an ActRIIA-Fc coating followed by the detection of ActRIIA ligands present in serum. The ActRIIA ELISA assay can be used experimentally (for e.g., animals) or clinically (for e.g., human patients) to identify, detect and/or quantify ligand or receptor levels in blood serum in order to aid in treatment decisions and/or determine the effectiveness of a treatment designed to modulate TGF-beta ligand or receptor levels.

### 6.3.8 GDF11 levels are elevated in the serum of thalassemia patients.

The sandwich ELISA assay described above was developed to measure GDF11 levels in serum. ELISA plates were coated with 5 microg/mL of mActRIIA-Fc (FIG 7A). Increasing doses of recombinant GDF11 were added to the plates (0.1 ng/microliter, 0.5 ng/microliter, 2.5 ng/microliter). The plates were washed with PBS 0.1% Tween and bound proteins detected using anti-GDF8/11 antibodies, followed by detection using a horseradish peroxidase-coupled anti-rabbit IgG. GDF11 bound the plates coated with mActRIIA-Fc in a dose-dependent manner indicating that the assay could effectively be used to detect and quantify GDF11 levels (FIG 7B). The ELISA using mActRIIA-Fc detected as little as 100 pg/mL of recombinant GDF11.

Serum from patients having thalassemia was tested for GDF11 expression using the sandwich ELISA with mActRIIA-Fc. As shown in FIG 8, GDF11 levels were elevated 3-fold in patients with thalassemia compared to levels in healthy controls.

To determine if the levels of other ActRIIA ligands were also elevated in thalassemia patients, activin A and activin B expression levels were also measured in the serum of those patients. An ELISA assay was also developed to detect activin A and activin B. ELISA plates were coated with 5 microgram/mL of ActRIIA-Fc. Increasing doses of recombinant activin A and activin B were added to the plates (0.1 ng/microliter, 0.5 ng/microliter, 2.5 ng/microliter). The plates were washed with PBS 0.1% Tween, and bound proteins detected with anti-activin A (FIG 9A) and anti-activin B (FIG 10A) antibodies (R&D systems), followed by detection using a horseradish peroxidase-coupled anti-rabbit IgG. Both activin A (FIG 9B) and activin B (FIG 10B) bound the ActRIIA-Fc-coated plates in a dose-dependent manner indicating that the assay could effectively be used to detect and quantify both proteins. Like GDF11, activin A and activin B were also detected at a levels as low as 100 pg/mL.

The ActRIIA-Fc ELISA was used to determine the expression levels activin A and activin B in the serum of patients with thalassemia. In contrast to GDF11, neither activin A (FIG 9C), nor activin B (FIG 10C) protein levels were elevated in thalassemic patients, indicating that the ActRIIA ligand GDF11 was uniquely implicated in the thalassemia disease process.

### 6.3.9 mActRIIA-Fc does not change hematological parameters in wild-type mice.

Wild-type C57BL/6 mice were treated for 30 days with mActRIIA-Fc (10mg/Kg BW twice a week) or PBS. Evaluation of red blood cell counts (FIG 11A), hematocrit (FIG 11B), hemoglobin (FIG 11C) indicated no change in the levels of the parameters as a result of treatment of the mice with mActRIIA-Fc. Only a slight decrease in reticulocytosis was observed (FIG 11D). mActRIIA-Fc also did not alter red blood cells (RBC) parameters such as MCV (FIG 11E), MCH (FIG 11F) and MCHC (FIG 12G). *p<0.05, N=3-5 for each independent experiment.

The effect of mActRIIA-Fc on the spleen and bone marrow was also assessed in wildtype C57BL/6 mice. mActRIIA-Fc increased spleen weight in wildtype mice, but had no significant effect on spleen cell number. mActRIIA-Fc also had no effect on bone marrow cell number (FIG 12).

### 6.3.10 mACTRIIA-FC STIMULATES ERYTHROPOIETIC DIFFERENTIATION THROUGH INHIBITION OF GDF11

In order to investigate the cellular mechanism by which mActRIIA-Fc increased red blood cell parameters, a series of in vitro experiments were conducted in which no evidence was found to support direct effects of mActRIIA-Fc on human CD34+ cells as assessed in colony formation assays (FIG. 13A) and in erythroid differentiation in liquid culture (FIGs 13B and 13C). As both clinical and pharmacological findings pointed to a clear role for mActRIIA-Fc in stimulating RBC parameters, it was hypothesized that the effects of mActRIIA-Fc could be mediated by accessory cells in the bone marrow (BM) microenvironment. Human CD36+ cells, which are highly enriched for erythroid progenitors, were co-cultured with long-term BM cultures and then their erythroid differentiation was assessed following 6 days of culture in EPO (2U/mL)- supplemented media. At day 6, the output of the cultures was predominantly characterized as EryA (∼basophilic erythroblast) but with the addition of mActRIIA-Fc (50µM), a significant fraction of CD36+ cells matured into EryB/C cells (polychromatic/orthochromatic erythroblasts), suggesting that factors produced by BM accessory cells mediated erythropoietic effects of mActRIIA-Fc and that, in contrast to EPO, mActRIIA-Fc could play a role in the latter stages or erythroblast maturation (FIGs 13D - 13F). To identify cytokines that might mediate the effects of mActRIIA-Fc, CD36+ cells were treated with several ActRIIA ligands. GDF11 treatment significantly decreased proliferation of glycoprotein A positive (GPA+) cells during the differentiation process and mActRIIA-Fc effectively reversed this effect, while having no consequence on untreated cells (FIGs 13G and 13H). These data show that inhibition of GDF11 mediates the erythropoietic stimulatory effects of mActRIIA-Fc.

### 6.4 CONCLUSION

Altogether, the data demonstrate that activin/BMP signaling controls erythroblast differentiation and that targeting BMP type II/activin type II receptors can decrease ineffective erythropoiesis and improve anemia in beta-thalassemia. In particular, the data show involvement of GDF11 in beta-thalassemia-associated anemia and show that inhibiting or decreasing GDF11 levels in thalassemic patients is an effective treatment for anemia associated with ineffective erythropoiesis.

### 7. DESCRIPTION OF THE SEQUENCES

**Table 1: Sequence Information**

| SEQ ID NO: | Description | Sequence |
|---|---|---|
| 1 | human ActRIIA precursor polypeptide | MGAAAKLAFAVFLISCSSGAILGRSETQECLFFNANWEKDRTNQTGVEPC YGDKDKRRHCFATWKNISGSIEIVKQGCWLDDINCYDRTDCVEKKDSPEV YFCCCEGNMCNEKFSYFPEMEVTQPTSNPVTPKPPYYNILLYSLVPLMLI AGIVICAFWVYRHHKMAYPPVLVPTQDPGPPPPSPLLGLKPLQLLEVKAR GRFGCVWKAQLLNEYVAVKIFPIQDKQSWQNEYEVYSLPGMKHENILQFI GAEKRGTSVDVDLWLITAFHEKGSLSDFLKANVVSWNELCHIAETMARGL AYLHEDIPGLKDGHKPAISHRDIKSKNVLLKNNLTACIADFGLALKFEAG KSAGDTHGQVGTRRYMAPEVLEGAINFQRDAFLRIDMYAMGLVLWELASR CTAADGPVDEYMLPFEEEIGQHPSLEDMQEVVVHKKKRPVLRDYWQKHAG MAMLCETIEECWDHDAEARLSAGCVGERITQMQRLTNIITTEDIVTVVTM VTNVDFPPKESSL |
| 2 | human ActRIIA soluble (extracellular), processed polypeptide sequence | ILGRSETQECLFFNANWEKDRTNQTGVEPCYGDKDKRRHCFATWKNISGS IEIVKQGCWLDDINCYDRTDCVEKKDSPEVYFCCCEGNMCNEKFSYFPEM EVTQPTSNPVTPKPP |
| 3 | human ActRIIA soluble (extracellular), processed polypeptide sequence with the C-terminal 15 amino acids deleted | ILGRSETQECLFFNANWEKDRTNQTGVEPCYGDKDKRRHCFATWKNISGS IEIVKQGCWLDDINCYDRTDCVEKKDSPEVYFCCCEGNMCNEKFSYFPEM |
| 4 | nucleic acid sequence encoding human ActRIIA precursor protein | ATGGGAGCTGCTGCAAAGTTGGCGTTTGCCGTCTTTCTTATCTCCTGTTC TTCAGGTGCTATACTTGGTAGATCAGAAACTCAGGAGTGTCTTTTCTTTA ATGCTAATTGGGAAAAAGACAGAACCAATCAAACTGGTGTTGAACCGTGT TATGGTGACAAAGATAAACGGCGGCATTGTTTTGCTACCTGGAAGAATAT TTCTGGTTCCATTGAAATAGTGAAACAAGGTTGTTGGCTGGATGATATCA ACTGCTATGACAGGACTGATTGTGTAGAAAAAAAAGACAGCCCTGAAGTA |
| | | TATTTTTGTTGCTGTGAGGGCAATATGTGTAATGAAAAGTTTTCTTATTT TCCAGAGATGGAAGTCACACAGCCCACTTCAAATCCAGTTACACCTAAGC CACCCTATTACAACATCCTGCTCTATTCCTTGGTGCCACTTATGTTAATT GCGGGGATTGTCATTTGTGCATTTTGGGTGTACAGGCATCACAAGATGGC CTACCCTCCTGTACTTGTTCCAACTCAAGACCCAGGACCACCCCCACCTT CTCCATTACTAGGGTTGAAACCACTGCAGTTATTAGAAGTGAAAGCAAGG GGAAGATTTGGTTGTGTCTGGAAAGCCCAGTTGCTTAACGAATATGTGGC TGTCAAAATATTTCCAATACAGGACAAACAGTCATGGCAAAATGAATACG AAGTCTACAGTTTGCCTGGAATGAAGCATGAGAACATATTACAGTTCATT GGTGCAGAAAAACGAGGCACCAGTGTTGATGTGGATCTTTGGCTGATCAC AGCATTTCATGAAAAGGGTTCACTATCAGACTTTCTTAAGGCTAATGTGG TCTCTTGGAATGAACTGTGTCATATTGCAGAAACCATGGCTAGAGGATTG GCATATTTACATGAGGATATACCTGGCCTAAAAGATGGCCACAAACCTGC CATATCTCACAGGGACATCAAAAGTAAAAATGTGCTGTTGAAAAACAACC TGACAGCTTGCATTGCTGACTTTGGGTTGGCCTTAAAATTTGAGGCTGGC AAGTCTGCAGGCGATACCCATGGACAGGTTGGTACCCGGAGGTACATGGC TCCAGAGGTATTAGAGGGTGCTATAAACTTCGAAAGGGATGCATTTTTGA GGATAGATATGTATGCCATGGGATTAGTCCTATGGGAACTGGCTTCTCGC TGTACTGCTGCAGATGGACCTGTAGATGAATACATGTTGCCATTTGAGGA GGAAATTGGCCAGCATCCATCTCTTGAAGACATGCAGGAAGTTGTTGTGC ATAAAAAAAAGAGGCCTGTTTTAAGAGATTATTGGCAGAAACATGCTGGA ATGGCAATGCTCTGTGAAACCATTGAAGAATGTTGGGATCACGACGCAGA AGCCAGGTTATCAGCTGGATGTGTAGGTGAAAGAATTACCCAGATGCAGA GACTAACAAATATTATTACCACAGAGGACATTGTAACAGTGGTCACAATG GTGACAAATGTTGACTTTCCTCCCAAAGAATCTAGTCTATGA |
| 5 | nucleic acid sequence encoding a human ActRIIA soluble (extracellular) polypeptide | ATACTTGGTAGATCAGAAACTCAGGAGTGTCTTTTCTTTAATGCTAATTG GGAAAAAGACAGAACCAATCAAACTGGTGTTGAACCGTGTTATGGTGACA AAGATAAACGGCGGCATTGTTTTGCTACCTGGAAGAATATTTCTGGTTCC ATTGAAATAGTGAAACAAGGTTGTTGGCTGGATGATATCAACTGCTATGA CAGGACTGATTGTGTAGAAAAAAAAGACAGCCCTGAAGTATATTTTTGTT GCTGTGAGGGCAATATGTGTAATGAAAAGTTTTCTTATTTTCCAGAGATG GAAGTCACACAGCCCACTTCAAATCCAGTTACACCTAAGCCACCC |
| 6 | fusion protein comprising a soluble extracellular domain of ActRIIA fused to an Fc domain | THTCPPCPAPELLGGPSVFLFPPKPKDTLMISRTPEVTCVVVD(A)VSHE DPEVKFNWYVDGVEVHNAKTKPREEQYNSTYRVVSVLTVLHQDWLNGKEY |
| | | KCK(A)VSNKALPVPIEKTISKAKGQPREPQVYTLPPSREEMTKNQVSLT CLVKGFYPSDIAVEWESNGQPENNYKTTPPVLDSDGPFFLYSKLTVDKSR WQQGNVFSCSVMHEALHN(A)HYTQKSLSLSPGK* |
| 7 | Extracellular domain of human ActRIIA fused to a human Fc domain | ILGRSETQECLFFNANWEKDRTNQTGVEPCYGDKDKRRHCFATWKNISGS IEIVKQGCWLDDINCYDRTDCVEKKDSPEVYFCCCEGNMCNEKFSYFPEM EVTQPTSNPVTPKPPTGGGTHTCPPCPAPELLGGPSVFLFPPKPKDTLMI SRTPEVTCVVVDVSHEDPEVKFNWYVDGVEVHNAKTKPREEQYNSTYRVV SVLTVLHQDWLNGKEYKCKVSNKALPVPIEKTISKAKGQPREPQVYTLPP SREEMTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 8 | Leader sequence of Honey bee mellitin (HBML) | MKFLVNVALVFMVVYISYIYA |
| 9 | Leader sequence of Tissue Plasminogen Activator (TPA) | MDAMKRGLCCVLLLCGAVFVSP |
| 10 | Native ActRIIA leader | MGAAAKLAFAVFLISCSSGA |
| 11 | ActRIIA-hFc and ActRIIA-mFc N-terminal sequence | ILGRSETQE |
| 12 | ActRIIA-Fc Protein with deletion of the C-terminal 15 amino acids of the extracellular domain of ActRIIA | ILGRSETQECLFFNANWEKDRTNQTGVEPCYGDKDKRRHCFATWKNISGS IEIVKQGCWLDDINCYDRTDCVEKKDSPEVYFCCCEGNMCNEKFSYFPEM TGGGTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKE YKCKVSNKALPVPIEKTISKAKGQPREPQVYTLPPSREEMTKNQVSLTCL VKGFYPSDIAVEWESNGQPENNYKTTPPVLDSDGSFFLYSKLTVDKSRWQ QGNVFSCSVMHEALHNHYTQKSLSLSPGK |
| 13 | Unprocessed ActRIIA-hFc with TPA leader sequence | MDAMKRGLCCVLLLCGAVFVSPGAAILGRSETQECLFFNANWEKDRTNQT GVEPCYGDKDKRRHCFATWKNISGSIEIVKQGCWLDDINCYDRTDCVEKK DSPEVYFCCCEGNMCNEKFSYFPEMEVTQPTSNPVTPKPPTGGGTHTCPP CPAPELLGGPSVFLFPPKPKDTLMISRTPEVTCVVVDVSHEDPEVKFNWY |
| | | VDGVEVHNAKTKPREEQYNSTYRVVSVLTVLHQDWLNGKEYKCKVSNKAL PVPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVM HEALHNHYTQKSLSLSPGK |
| 14 | Nucleic acid sequence encoding Unprocessed ActRIIA-hFc with TPA leader sequence | ATGGATGCAATGAAGAGAGGGCTCTGCTGTGTGCTGCTGCTGTGTGGAGC AGTCTTCGTTTCGCCCGGCGCCGCTATACTTGGTAGATCAGAAACTCAGG AGTGTCTTTTTTAATGCTAATTGGAAAAAGACAGAACCAATCAAACTG GTGTTGAACCGTGTTATGGTGACAAAGATAAACGGCGGCATTGTTTTGCT ACCTGGAAGAATATTTCTGGTTCCATTGAATAGTGAAACAAGGTTGTTGG CTGGATGATATCAACTGCTATGACAGGACTGATTGTGTAGAAAAAAAAGA CAGCCCTGAAGTATATTTCTGTTGCTGTGAGGGCAATATGTGTAATGAAA AGTTTTCTTATTTTCCGGAGATGGAAGTCACACAGCCCACTTCAAATCCA GTTACACCTAAGCCACCCACCGGTGGTGGAACTCACACATGCCCACCGTG CCCAGCACCTGAACTCCTGGGGGGACCGTCAGTCTTCCTCTTCCCCCCAA AACCCAAGGACACCCTCATGATCTCCCGGACCCCTGAGGTCACATGCGTG GTGGTGGACGTGAGCCACGAAGACCCTGAGGTCAAGTTCAACTGGTACGT GGACGGCGTGGAGGTGCATAATGCCAAGACAAAGCCGCGGGAGGAGCAGT ACAACAGCACGTACCGTGTGGTCAGCGTCCTCACCGTCCTGCACCAGGAC TGGCTGAATGGCAAGGAGTACAAGTGCAAGGTCTCCAACAAAGCCCTCCC AGTCCCCATCGAGAAAACCATCTCCAAAGCCAAAGGGCAGCCCCGAGAAC CACAGGTGTACACCCTGCCCCCATCCCGGGAGGAGATGACCAAGAACCAG GTCAGCCTGACCTGCCTGGTCAAAGGCTTCTATCCCAGCGACATCGCCGT GGAGTGGGAGAGCAATGGGCAGCCGGAGAACAACTACAAGACCACGCCTC CCGTGCTGGACTCCGACGGCTCCTTCTTCCTCTATAGCAAGCTCACCGTG GACAAGAGCAGGTGGCAGCAGGGGAACGTCTTCTCATGCTCCGTGATGCA TGAGGCTCTGCACAACCACTACACGCAGAAGAGCCTCTCCCTGTCTCCGG GTAAATGAGAATTC |
| 15 | human ActRIIB soluble (extracellular), processed polypeptide sequence with the N-terminal 6 amino acids of the EC domain deleted and the C-terminal 4 amino acids of the EC domain deleted (amino acids 25-130 of SEQ ID NO:28) and with an L79D mutation | ETRECIYYNANWELERTNQSGLERCEGEQDKRLHCYASWRNSSGTIELV KKGCWDDDFNCYDRQECVATEENPQVYFCCCEGNFCNERFTHLPEAGGP EVTYEPPP |
| | | |
| 16 | human ActRIIB precursor protein sequence (A64) | MTAPWVALALLWGSLW PGSGRGEAETRECIYY NANWELERTNQSGLER CEGEQDKRLHCYASWA NSSGTIELVKKGCWLD DFNCYDRQECVATEEN PQVYFCCCEGNFCNER FTHLPEAGGPEVTYEP PPTAPTLLTVLAYSLL PIGGLSLIVLLAFWMY RHRKPPYGHVDIHEDP GPPPPSPLVGLKPLQL LEIKARGRFGCVWKAQ LMNDFVAVKIFPLQDK QSWQSEREIFSTPGMK HENLLQFIAAEKRGSN LEVELWLITAFHDKGS LTDYLKGNIITWNELC HVAETMSRGLSYLHED VPWCRGEGHKPSIAHR DFKSKNVLLKSDLTAV LADFGLAVRFEPGKPP GDTHGQVGTRRYMAPE VLEGAINFQRDAFLRI DMYAMGLVLWELVSRC KAADGPVDEYMLPFEE EIGQHPSLEELQEVVV HKKMRPTIKDHWLKHP GLAQLCVTIEECWDHD AEARLSAGCVEERVSL IRRSVNGTTSDCLVSL VTSVTNVDLPPKESSI |
| 17 | human ActRIIB soluble (extracellular), processed polypeptide sequence (amino acids 19-134 of SEQ ID NO:16) | SGRGEAETRECIYYNANWELERTNQSGLERCEGEQDKRLHCYASWANSSG TIELVKKGCWLDDFNCYDRQECVATEENPQVYFCCCEGNFCNERFTHLPE AGGPEVTYEPPPTAPT |
| 18 | human ActRIIB soluble (extracellular), processed polypeptide sequence with the C-terminal 15 amino acids deleted (amino acids 19-119 of SEQ ID NO:16) | SGRGEAETRECIYYNANWELERTNQSGLERCEGEQDKRLHCYASWANSSG TIELVKKGCWLDDFNCYDRQECVATEENPQVYFCCCEGNFCNERFTHLPE A |
| 19 | nucleic acid sequence encoding a human ActRIIB (A64) precursor protein | ATGACGGCGCCCTGGGTGGCCCTCGCCCTCCTCTGGGGATCGCTGTGGCC CGGCTCTGGGCGTGGGGAGGCTGAGACACGGGAGTGCATCTACTACAACG CCAACTGGGAGCTGGAGCGCACCAACCAGAGCGGCCTGGAGCGCTGCGAA GGCGAGCAGGACAAGCGGCTGCACTGCTACGCCTCCTGGGCCAACAGCTC TGGCACCATCGAGCTCGTGAAGAAGGGCTGCTGGCTAGATGACTTCAACT GCTACGATAGGCAGGAGTGTGTGGCCACTGAGGAGAACCCCCAGGTGTAC TTCTGCTGCTGTGAAGGCAACTTCTGCAACGAGCGCTTCACTCATTTGCC AGAGGCTGGGGGCCCGGAAGTCACGTACGAGCCACCCCCGACAGCCCCCA CCCTGCTCACGGTGCTGGCCTACTCACTGCTGCCCATCGGGGGCCTTTCC CTCATCGTCCTGCTGGCCTTTTGGATGTACCGGCATCGCAAGCCCCCCTA CGGTCATGTGGACATCCATGAGGACCCTGGGCCTCCACCACCATCCCCTC TGGTGGGCCTGAAGCCACTGCAGCTGCTGGAGATCAAGGCTCGGGGGCGC TTTGGCTGTGTCTGGAAGGCCCAGCTCATGAATGACTTTGTAGCTGTCAA GATCTTCCCACTCCAGGACAAGCAGTCGTGGCAGAGTGAACGGGAGATCT TCAGCACACCTGGCATGAAGCACGAGAACCTGCTACAGTTCATTGCTGCC GAGAAGCGAGGCTCCAACCTCGAAGTAGAGCTGTGGCTCATCACGGCCTT CCATGACAAGGGCTCCCTCACGGATTACCTCAAGGGGAACATCATCACAT GGAACGAACTGTGTCATGTAGCAGAGACGATGTCACGAGGCCTCTCATAC CTGCATGAGGATGTGCCCTGGTGCCGTGGCGAGGGCCACAAGCCGTCTAT TGCCCACAGGGACTTTAAAAGTAAGAATGTATTGCTGAAGAGCGACCTCA CAGCCGTGCTGGCTGACTTTGGCTTGGCTGTTCGATTTGAGCCAGGGAAA CCTCCAGGGGACACCCACGGACAGGTAGGCACGAGACGGTACATGGCTCC TGAGGTGCTCGAGGGAGCCATCAACTTCCAGAGAGATGCCTTCCTGCGCA TTGACATGTATGCCATGGGGTTGGTGCTGTGGGAGCTTGTGTCTCGCTGC AAGGCTGCAGACGGACCCGTGGATGAGTACATGCTGCCCTTTGAGGAAGA GATTGGCCAGCACCCTTCGTTGGAGGAGCTGCAGGAGGTGGTGGTGCACA AGAAGATGAGGCCCACCATTAAAGATCACTGGTTGAAACACCCGGGCCTG GCCCAGCTTTGTGTGACCATCGAGGAGTGCTGGGACCATGATGCAGAGGC TCGCTTGTCCGCGGGCTGTGTGGAGGAGCGGGTGTCCCTGATTCGGAGGT CGGTCAACGGCACTACCTCGGACTGTCTCGTTTCCCTGGTGACCTCTGTC ACCAATGTGGACCTGCCCCCTAAAGAGTCAAGCATCTAA |
| 20 | fusion protein comprising a soluble extracellular domain of | SGRGEAETRECIYYNANWELERTNQSGLERCEGEQDKRLHCYASWANSSG TIELVKKGCWLDDFNCYDRQECVATEENPQVYFCCCEGNFCNERFTHLPE AGGPEVTYEPPPTAPTGGGTHTCPPCPAPELLGGPSVFLFPPKPKDTLMI |
| | ActRIIB (A64; SEQ ID NO:17) fused to an Fc domain | SRTPEVTCVVVDVSHEDPEVKFNWYVDGVEVHNAKTKPREEQYNSTYRVV SVLTVLHQDWLNGKEYKCKVSNKALPVPIEKTISKAKGQPREPQVYTLPP SREEMTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 21 | fusion protein comprising a soluble extracellular domain of ActRIIB (A64) with the C-terminal 15 amino acids deleted (SEQ ID NO:18) fused to an Fc domain | SGRGEAETRECIYYNANWELERTNQSGLERCEGEQDKRLHCYASWANSSG TIELVKKGCWLDDFNCYDRQECVATEENPQVYFCCCEGNFCNERFTHLPE AGGGTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKE YKCKVSNKALPVPIEKTISKAKGQPREPQVYTLPPSREEMTKNQVSLTCL VKGFYPSDIAVEWESNGQPENNYKTTPPVLDSDGSFFLYSKLTVDKSRWQ QGNVFSCSVMHEALHNHYTQKSLSLSPGK |
| 22 | human ActRIIB soluble (extracellular), processed polypeptide sequence with the N-terminal 6 amino acids of the EC domain deleted and the C-terminal 5 amino acids of the EC domain deleted (amino acids 25-129 of SEQ ID NO:28) and with an L79D mutation | ETRECIYYNANWELERTNQSGLERCEGEQDKRLHCYASWRNSSGTIELV KKGCWDDDFNCYDRQECVATEENPQVYFCCCEGNFCNERFTHLPEAGGP EVTYEPP |
| 23 | human ActRIIB soluble (extracellular), processed polypeptide sequence with the N-terminal 6 amino acids of the EC domain deleted and the C-terminal 3 amino acids | ETRECIYYNANWELERTNQSGLERCEGEQDKRLHCYASWRNSSGTIELV KKGCWDDDFNCYDRQECVATEENPQVYFCCCEGNFCNERFTHLPEAGGP EVTYEPPPT |
| | of the EC domain deleted (amino acids 25-131 of SEQ ID NO:28) and with an L79D mutatioN | |
| 24 | Unprocessed ActRIIB-Fc fusion protein with the N-terminal 6 amino acids of the EC domain deleted and the C-terminal 3 amino acids of the EC domain deleted (amino acids 25-131 of SEQ ID NO:28) and with an L79D mutation and with TPA leader sequence | MDAMKRGLCCVLLLCGAVFVSPGAAETRECIYYNANWELERTNQSGLERC EGEQDKRLHCYASWRNSSGTIELVKKGCWDDDFNCYDRQECVATEENPQV YFCCCEGNFCNERFTHLPEAGGPEVTYEPPPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 25 | Processed ActRIIB-Fc fusion protein with the N-terminal 6 amino acids of the EC domain deleted and the C-terminal 3 amino acids of the EC domain deleted (amino acids 25-131 of SEQ ID NO:28) and with an L79D mutation | ETRECIYYNANWELERTNQSGLERCEGEQDKRLHCYASWRNSSGTIELVK KGCWDDDFNCYDRQECVATEENPQVYFCCCEGNFCNERFTHLPEAGGPEV TYEPPPTGGGTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLTV DKSRWQQGNVFSCSVMHEALHNHYTQKSLSLSPGK* |
| 26 | human ActRIIB soluble (extracellular), processed polypeptide sequence (amino acids 20-134 of SEQ ID NO:16) | GRGEAETRECIYYNANWELERTNQSGLERCEGEQDKRLHCYASWANSSG TIELVKKGCWLDDFNCYDRQECVATEENPQVYFCCCEGNFCNERFTHLPE AGGPEVTYEPPPTAPT |
| 27 | human ActRIIB soluble (extracellular), processed polypeptide sequence with the C-terminal 15 amino acids deleted (amino acids 20-119 of SEQ ID NO:16) | GRGEAETRECIYYNANWELERTNQSGLERCEGEQDKRLHCYASWANSSG TIELVKKGCWLDDFNCYDRQECVATEENPQVYFCCCEGNFCNERFTHLPE A |
| 28 | human ActRIIB precursor protein sequence (R64) | MTAPWVALALLWGSLW PGSGRGEAETRECIYY NANWELERTNQSGLER CEGEQDKRLHCYASWR NSSGTIELVKKGCWLD DFNCYDRQECVATEEN PQVYFCCCEGNFCNER FTHLPEAGGPEVTYEP PPTAPTLLTVLAYSLL PIGGLSLIVLLAFWMY RHRKPPYGHVDIHEDP GPPPPSPLVGLKPLQL LEIKARGRFGCVWKAQ LMNDFVAVKIFPLQDK QSWQSEREIFSTPGMK HENLLQFIAAEKRGSN LEVELWLITAFHDKGS LTDYLKGNIITWNELC HVAETMSRGLSYLHED VPWCRGEGHKPSIAHR DFKSKNVLLKSDLTAV LADFGLAVRFEPGKPP GDTHGQVGTRRYMAPE VLEGAINFQRDAFLRI DMYAMGLVLWELVSRC KAADGPVDEYMLPFEE EIGQHPSLEELQEVVV HKKMRPTIKDHWLKHP GLAQLCVTIEECWDHD AEARLSAGCVEERVSL IRRSVNGTTSDCLVSL VTSVTNVDLPPKESSI |
| 29 | human ActRIIB soluble (extracellular), processed polypeptide sequence (amino acids 19-134 of SEQ ID NO:28) | SGRGEAETRECIYYNANWELERTNQSGLERCEGEQDKRLHCYASWRNSSG TIELVKKGCWLDDFNCYDRQECVATEENPQVYFCCCEGNFCNERFTHLPE AGGPEVTYEPPPTAPT |
| 30 | human ActRIIB soluble (extracellular), processed polypeptide sequence with the C-terminal 15 amino acids deleted (amino acids 19-119 of SEQ ID NO:28) | SGRGEAETRECIYYNANWELERTNQSGLERCEGEQDKRLHCYASWRNSSG TIELVKKGCWLDDFNCYDRQECVATEENPQVYFCCCEGNFCNERFTHLPE A |
| 31 | human ActRIIB soluble (extracellular), processed polypeptide sequence (amino acids 20-134 of SEQ ID NO:28) | GRGEAETRECIYYNANWELERTNQSGLERCEGEQDKRLHCYASWRNSSG TIELVKKGCWLDDFNCYDRQECVATEENPQVYFCCCEGNFCNERFTHLPE AGGPEVTYEPPPTAPT |
| 32 | human ActRIIB soluble (extracellular), processed polypeptide sequence with the C-terminal 15 amino acids deleted (amino acids 20-119 of SEQ ID NO:28) | GRGEAETRECIYYNANWELERTNQSGLERCEGEQDKRLHCYASWRNSSG TIELVKKGCWLDDFNCYDRQECVATEENPQVYFCCCEGNFCNERFTHLPE A |
| 33 | human ActRIIB soluble (extracellular), processed polypeptide sequence with the N-terminal 6 amino acids of the EC domain deleted and the C-terminal 3 amino acids of the EC domain deleted (amino acids 25-131 of SEQ ID NO:16) and with an L79D mutation | ETRECIYYNANWELERTNQSGLERCEGEQDKRLHCYASWANSSGTIELV KKGCWDDDFNCYDRQECVATEENPQVYFCCCEGNFCNERFTHLPEAGGP EVTYEPPPT |
| 34 | Unprocessed ActRIIB-Fc fusion protein with the N-terminal 6 amino acids of the EC domain deleted and the C-terminal 3 amino acids of the EC domain deleted (amino acids 25-131 of SEQ ID NO:16) and with an L79D mutation and with TPA leader sequence | MDAMKRGLCCVLLLCGAVFVSPGAAETRECIYYNANWELERTNQSGLERC EGEQDKRLHCYASWANSSGTIELVKKGCWDDDFNCYDRQECVATEENPQV YFCCCEGNFCNERFTHLPEAGGPEVTYEPPPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT |
| | | QKSLSLSPGK* |
| 35 | Processed ActRIIB-Fc fusion protein with the N-terminal 6 amino acids of the EC domain deleted and the C-terminal 3 amino acids of the EC domain deleted (amino acids 25-131 of SEQ ID NO:16) and with an L79D mutation | ETRECIYYNANWELERTNQSGLERCEGEQDKRLHCYASWANSSGTIELVK KGCWDDDFNCYDRQECVATEENPQVYFCCCEGNFCNERFTHLPEAGGPEV TYEPPPTGGGTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLTV DKSRWQQGNVFSCSVMHEALHNHYTQKSLSLSPGK* |
| 36 | human ActRIIB soluble (extracellular), processed polypeptide sequence (amino acids 20-134 of SEQ ID NO:28) with L79D mutation | GRGEAETRECIYYNANWELERTNQSGLERCEGEQDKRLHCYASWRNSSG TIELVKKGCWDDDFNCYDRQECVATEENPQVYFCCCEGNFCNERFTHLPE AGGPEVTYEPPPTAPT |
| 37 | human ActRIIB soluble (extracellular), processed polypeptide sequence (amino acids 20-134 of SEQ ID NO:16) with L79D mutation | GRGEAETRECIYYNANWELERTNQSGLERCEGEQDKRLHCYASWANSSG TIELVKKGCWDDDFNCYDRQECVATEENPQVYFCCCEGNFCNERFTHLPE AGGPEVTYEPPPTAPT |
| 38 | human ActRIIB soluble (extracellular), processed polypeptide sequence (amino acids 20-134 of SEQ ID NO:28) with L79D mutation fused to an Fc domain with a GGG linker | GRGEAETRECIYYNANWELERTNQSGLERCEGEQDKRLHCYASWRNSSG TIELVKKGCWDDDFNCYDRQECVATEENPQVYFCCCEGNFCNERFTHLPE AGGPEVTYEPPPTAPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS |
| | | KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 39 | human ActRIIB soluble (extracellular), processed polypeptide sequence (amino acids 20-134 of SEQ ID NO:16) with L79D mutation fused to an Fc domain | GRGEAETRECIYYNANWELERTNQSGLERCEGEQDKRLHCYASWANSSG TIELVKKGCWDDDFNCYDRQECVATEENPQVYFCCCEGNFCNERFTHLPE AGGPEVTYEPPPTAPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 40 | human ActRIIB soluble (extracellular), processed polypeptide sequence (amino acids 20-134 of SEQ ID NO:28) with L79D mutation fused to an Fc domain and with TPA leader sequence | MDAMKRGLCCVLLLCGAVFVSPGASGRGEAETRECIYYNANWELERTNQSG LERCEGEQDKRLHCYASWRNSSG TIELVKKGCWDDDFNCYDRQECVATEENPQVYFCCCEGNFCNERFTHLPE AGGPEVTYEPPPTAPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 41 | human ActRIIB soluble (extracellular), processed polypeptide sequence (amino acids 20-134 of SEQ ID NO:16) with L79D mutation fused to an Fc domain and with TPA leader sequence | MDAMKRGLCCVLLLCGAVFVSPGASGRGEAETRECIYYNANWELERTNQSG LERCEGEQDKRLHCYASWANSSG TIELVKKGCWDDDFNCYDRQECVATEENPQVYFCCCEGNFCNERFTHLPE AGGPEVTYEPPPTAPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 42 | human ActRIIB soluble (extracellular), processed polypeptide sequence having a variant C-terminal sequence (disclosed in WO2007/053775) | GRGEAETRECIYYNANWELERTNQSGLERCEGEQDKRLHCYASWRNSSGT IELVKKGCWLDDFNCYDRQECVATEENPQVYFCCCEGNFCNERFTHLPEA GGPEGPWASTTIPSGGPEATAAAGDQGSGALWLCLEGPAHE |
| 43 | human ActRIIB soluble (extracellular), processed polypeptide sequence having a variant C-terminal sequence (disclosed in WO2007/053775) having an L79D mutation | GRGEAETRECIYYNANWELERTNQSGLERCEGEQDKRLHCYASWRNSSGT IELVKKGCWDDDFNCYDRQECVATEENPQVYFCCCEGNFCNERFTHLPEA GGPEGPWASTTIPSGGPEATAAAGDQGSGALWLCLEGPAHE |
| 44 | human ActRIIB soluble (extracellular), processed polypeptide sequence having a variant C-terminal sequence (disclosed in WO2007/053775) having an L79D mutation fused to an Fc domain with a TGGG linker | GRGEAETRECIYYNANWELERTNQSGLERCEGEQDKRLHCYASWRNSSGT IELVKKGCWDDDFNCYDRQECVATEENPQVYFCCCEGNFCNERFTHLPEA GGPEGPWASTTIPSGGPEATAAAGDQGSGALWLCLEGPAHE TGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 45 | Nucleic Acid Sequence Encoding SEQ ID NO:24 | ATGGATGCAA TGAAGAGAGG GCTCTGCTGT GTGCTGCTGC TGTGTGGAGC AGTCTTCGTT TCGCCCGGCG CCGCCGAAAC CCGCGAATGT ATTTATTACA ATGCTAATTG GGAACTCGAA CGGACGAACC AATCCGGGCT CGAACGGTGT GAGGGGGAAC AGGATAAACG CCTCCATTGC TATGCGTCGT GGAGGAACTC CTCCGGGACG ATTGAACTGG TCAAGAAAGG GTGCTGGGAC GACGATTTCA ATTGTTATGA CCGCCAGGAA TGTGTCGCGA CCGAAGAGAA TCCGCAGGTC TATTTCTGTT GTTGCGAGGG GAATTTCTGT AATGAACGGT TTACCCACCT CCCCGAAGCC GGCGGGCCCG AGGTGACCTA TGAACCCCCG CCCACCGGTG GTGGAACTCA CACATGCCCA CCGTGCCCAG CACCTGAACT CCTGGGGGGA CCGTCAGTCT TCCTCTTCCC CCCAAAACCC |
| | | AAGGACACCC TCATGATCTC CCGGACCCCT GAGGTCACAT GCGTGGTGGT GGACGTGAGC CACGAAGACC CTGAGGTCAA GTTCAACTGG TACGTGGACG GCGTGGAGGT GCATAATGCC AAGACAAAGC CGCGGGAGGA GCAGTACAAC AGCACGTACC GTGTGGTCAG CGTCCTCACC GTCCTGCACC AGGACTGGCT GAATGGCAAG GAGTACAAGT GCAAGGTCTC CAACAAAGCC CTCCCAGCCC CCATCGAGAA AACCATCTCC AAAGCCAAAG GGCAGCCCCG AGAACCACAG GTGTACACCC TGCCCCCATC CCGGGAGGAG ATGACCAAGA ACCAGGTCAG CCTGACCTGC CTGGTCAAAG GCTTCTATCC CAGCGACATC GCCGTGGAGT GGGAGAGCAA TGGGCAGCCG GAGAACAACT ACAAGACCAC GCCTCCCGTG CTGGACTCCG ACGGCTCCTT CTTCCTCTAT AGCAAGCTCA CCGTGGACAA GAGCAGGTGG CAGCAGGGGA ACGTCTTCTC ATGCTCCGTG ATGCATGAGG CTCTGCACAA CCACTACACG CAGAAGAGCC TCTCCCTGTC CCCGGGTAAA TGA |
| 46 | fusion protein comprising a soluble extracellular domain of ActRIIB (R64; SEQ ID NO:29) fused to an Fc domain | SGRGEAETRECIYYNANWELERTNQSGLERCEGEQDKRLHCYASWRNSSG TIELVKKGCWLDDFNCYDRQECVATEENPQVYFCCCEGNFCNERFTHLPE AGGPEVTYEPPPTAPTGGGTHTCPPCPAPELLGGPSVFLFPPKPKDTLMI SRTPEVTCVVVDVSHEDPEVKFNWYVDGVEVHNAKTKPREEQYNSTYRVV SVLTVLHQDWLNGKEYKCKVSNKALPVPIEKTISKAKGQPREPQVYTLPP SREEMTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 47 | fusion protein comprising a soluble extracellular domain of ActRIIB (R64) with the C-terminal 15 amino acids deleted (SEQ ID NO:30) fused to an Fc domain | SGRGEAETRECIYYNANWELERTNQSGLERCEGEQDKRLHCYASWRNSSG TIELVKKGCWLDDFNCYDRQECVATEENPQVYFCCCEGNFCNERFTHLPE AGGGTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKE YKCKVSNKALPVPIEKTISKAKGQPREPQVYTLPPSREEMTKNQVSLTCL VKGFYPSDIAVEWESNGQPENNYKTTPPVLDSDGSFFLYSKLTVDKSRWQ QGNVFSCSVMHEALHNHYTQKSLSLSPGK |
| **48** | full-length, unprocessed precursor protein GDF11, i.e., GDF11 preproprotein | MVLAAPLLLGFLLLALELRPRGEAAEGPAAAAAAAAA AAAAGVGGERSSRPAPSVAPEPDGCPVCVWRQHSREL RLESIKSQILSKLRLKEAPNISREVVKQLLPKAPPLQQIL DLHDFQGDALQPEDFLEEDEYHATTETVISMAQETDPA VQTDGSPLCCHFHFSPKVMFTKVLKAQLWVYLRPVPR PATVYLQILRLKPLTGEGTAGGGGGGRRHIRIRSLKIEL HSRSGHWQSIDFKQVLHSWFRQPQSNWGIEINAFDPSG TDLAVTSLGPGAEGLHPFMELRVLENTKRSRRNLGLDC DEHSSESRCCRYPLTVDFEAFGWDWIIAPKRYKANYCS GQCEYMFMQKYPHTHLVQQANPRGSAGPCCTPTKMSP INMLYFNDKQQIIYGKIPGMVVDRCGCS |
| **49** | Nucleic acid sequence encoding SEQ ID NO: 48 | ATGGTGCTCGCGGCCCCGCTGCTGCTGGGCTTCCTGC TCCTCGCCCTGGAGCTGCGGCCCCGGGGGGAGGCGG CCGAGGGCCCCGCGGCGGCGGCGGCGGCGGCGGCG GCGGCGGCAGCGGCGGGGGTCGGGGGGGAGCGCTC CAGCCGGCCAGCCCCGTCCGTGGCGCCCGAGCCGGA CGGCTGCCCCGTGTGCGTTTGGCGGCAGCACAGCCG CGAGCTGCGCCTAGAGAGCATCAAGTCGCAGATCTT GAGCAAACTGCGGCTCAAGGAGGCGCCCAACATCAG CCGCGAGGTGGTGAAGCAGCTGCTGCCCAAGGCGCC GCCGCTGCAGCAGATCCTGGACCTACACGACTTCCA GGGCGACGCGCTGCAGCCCGAGGACTTCCTGGAGGA GGACGAGTACCACGCCACCACCGAGACCGTCATTAG CATGGCCCAGGAGACGGACCCAGCAGTACAGACAGA TGGCAGCCCTCTCTGCTGCCATTTTCACTTCAGCCCC AAGGTGATGTTCACAAAGGTACTGAAGGCCCAGCTG TGGGTGTACCTACGGCCTGTACCCCGCCCAGCCACA GTCTACCTGCAGATCTTGCGACTAAAACCCCTAACTG GGGAAGGGACCGCAGGGGGAGGGGGCGGAGGCCGG CGTCACATCCGTATCCGCTCACTGAAGATTGAGCTGC ACTCACGCTCAGGCCATTGGCAGAGCATCGACTTCA AGCAAGTGCTACACAGCTGGTTCCGCCAGCCACAGA GCAACTGGGGCATCGAGATCAACGCCTTTGATCCCA GTGGCACAGACCTGGCTGTCACCTCCCTGGGGCCGG GAGCCGAGGGGCTGCATCCATTCATGGAGCTTCGAG TCCTAGAGAACACAAAACGTTCCCGGCGGAACCTGG GTCTGGACTGCGACGAGCACTCAAGCGAGTCCCGCT GCTGCCGATATCCCCTCACAGTGGACTTTGAGGCTTT CGGCTGGGACTGGATCATCGCACCTAAGCGCTACAA GGCCAACTACTGCTCCGGCCAGTGCGAGTACATGTTC ATGCAAAAATATCCGCATACCCATTTGGTGCAGCAG GCCAATCCAAGAGGCTCTGCTGGGCCCTGTTGTACCC CCACCAAGATGTCCCCAATCAACATGCTCTACTTCAA TGACAAGCAGCAGATTATCTACGGCAAGATCCCTGG CATGGTGGTGGATCGCTGTGGCTGCTCT |
| **50** | GDF11 propeptide of human GDF11 protein | AEGPAAAAAAAAAAAAAGVGGERSSRPAPSVAPEPDG CPVCVWRQHSRELRLESIKSQILSKLRLKEAPNISREVV KQLLPKAPPLQQILDLHDFQGDALQPEDFLEEDEYHAT TETVISMAQETDPAVQTDGSPLCCHFHFSPKVMFTKVL KAQLWVYLRPVPRPATVYLQILRLKPLTGEGTAGGGG GGRRHIRIRSLKIELHSRSGHWQSIDFKQVLHSWFRQPQ SNWGIEINAFDPSGTDLAVTSLGPGAEGLHPFMELRVLE |
| | | NTKRSRR |
| **51** | Nucleic acid sequence encoding SEQ ID NO: 50 | GCCGAGGGCCCGCGGCGGCGGCGGCGGCGGCGGC GGCGGCGGCAGCGGCGGGGTCGGGGGGAGCGCT CCAGCCGGCCAGCCCCGTCCGTGGCGCCCGAGCCGG ACGGCTGCCCCGTGTGCGTTTGGCGGCAGCACAGCC GCGAGCTGCGCCTAGAGAGCATCAAGTCGCAGATCT TGAGCAAACTGCGGCTCAAGGAGGCGCCCAACATCA GCCGCGAGGTGGTGAAGCAGCTGCTGCCCAAGGCGC CGCCGCTGCAGCAGATCCTGGACCTACACGACTTCC AGGGCGACGCGCTGCAGCCCGAGGACTTCCTGGAGG AGGACGAGTACCACGCCACCACCGAGACCGTCATTA GCATGGCCCAGGAGACGGACCCAGCAGTACAGACAG ATGGCAGCCCTCTCTGCTGCCATTTTCACTTCAGCCC CAAGGTGATGTTCACAAAGGTACTGAAGGCCCAGCT GTGGGTGTACCTACGGCCTGTACCCCGCCCAGCCAC AGTCTACCTGCAGATCTTGCGACTAAAACCCCTAACT GGGGAAGGGACCGCAGGGGGAGGGGGCGGAGGCCG GCGTCACATCCGTATCCGCTCACTGAAGATTGAGCTG CACTCACGCTCAGGCCATTGGCAGAGCATCGACTTC AAGCAAGTGCTACACAGCTGGTTCCGCCAGCCACAG AGCAACTGGGGCATCGAGATCAACGCCTTTGATCCC AGTGGCACAGACCTGGCTGTCACCTCCCTGGGGCCG GGAGCCGAGGGGCTGCATCCATTCATGGAGCTTCGA GTCCTAGAGAACACAAAACGTTCCCGGCGG |
| **52** | Mature human GDF11 protein | NLGLDCDEHSSESRCCRYPLTVDFEAFGWDWIIAPKRY KANYCSGQCEYMFMQKYPHTHLVQQANPRGSAGPCC TPTKMSPINMLYFNDKQQIIYGKIPGMVVDRCGCS |
| **53** | Nucleic acid sequence encoding SEQ ID NO: 52 | AACCTGGGTCTGGACTGCGACGAGCACTCAAGCGAG TCCCGCTGCTGCCGATATCCCCTCACAGTGGACTTTG AGGCTTTCGGCTGGGACTGGATCATCGCACCTAAGC GCTACAAGGCCAACTACTGCTCCGGCCAGTGCGAGT ACATGTTCATGCAAAAATATCCGCATACCCATTTGGT GCAGCAGGCCAATCCAAGAGGCTCTGCTGGGCCCTG TTGTACCCCCACCAAGATGTCCCCAATCAACATGCTC TACTTCAATGACAAGCAGCAGATTATCTACGGCAAG ATCCCTGGCATGGTGGTGGATCGCTGTGGCTGCTCT |
| **54** | Extracellular domain of murine ActRIIA fused to a murine Fc domain ("mActRIIA-Fc") | Murine counterpart of SEQ ID NO: 7 |

### 8. EQUIVALENTS

Although the invention is described in detail with reference to specific embodiments thereof, it will be understood that variations which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims. Those skilled in the art will recognize, or be able to ascertain using no more than routine experimentation, many equivalents to the specific embodiments of the invention described herein. Such equivalents are intended to be encompassed by the following claims.

All publications, patents and patent applications mentioned in this specification are herein incorporated by reference into the specification to the same extent as if each individual publication, patent or patent application was specifically and individually indicated to be incorporated herein by reference in their entireties.
1. A method for treating anemia in a patient, wherein the method comprises administering an antagonist of GDF11.
2. A method for treating ineffective erythropoiesis in a patient, wherein the method comprises administering an antagonist of GDF11.
3. A method for treating beta thalassemia in a patient, wherein the method comprises administering an antagonist of GDF11.
4. A method for increasing Orthochromatic Erythroblasts (Ery-C) in a patient, wherein the method comprises administering an antagonist of GDF11.
5. The method of item 1, 2, 3, or 4, wherein the patient has elevated GDF11 levels in bone marrow, spleen, liver, serum, or plasma.
6. The method of item 1, 2, 3, or 4, wherein, if the antagonist of GDF11 is an ActRIIA polypeptide, the ActRIIA polypeptide binds preferentially to GDF11.
7. The method of item 1, 2, 3, or 4, wherein, if the antagonist of GDF11 is an ActRIIB polypeptide, the ActRIIB polypeptide binds preferentially to GDF11.
8. The method of item 1, 2, 3, or 4, wherein the antagonist of GDF11 reduces expression of GDF11, reduces GDF11 activity, or reduces GDF11 protein levels.
9. The method of item 1, 2, 3, or 4, wherein the antagonist of GDF11 is an anti GDF11 antibody.
10. The method of item 1, 2, 3, or 4, wherein the antagonist of GDF11 is a truncated receptor that binds GDF11.
11. The method of item 1, 2, 3, or 4, wherein the antagonist of GDF11 comprises a mutated extracellular domain of ActRIIA, wherein the mutated extracellular domain of ActRIIA has an increased affinity to GDF11 relative to Activin A as compared to the wild type of ActRIIA.
12. The method of item 1, 3, or 4 wherein the method further comprises monitoring GDF11 levels.
13. The method of item 1, 3, or 4 wherein the method further comprises
   a. Determining GDF11 levels; and
   b. Adjusting the dose of the antagonist of GDF11 wherein, if GDF11 is increased over normal, the dose of antagonist of GDF11 is increased and wherein, if GDF11 is decreased below normal, the dose of antagonist of GDF11 is decreased.
14. The method of item 12 or 13, wherein the GDF11 levels are determined as GDF11 mRNA levels, GDF11 protein levels, or GDF11 protein activity levels.
15. The method of item 1, 3, or 4, wherein said administering results in a decreased cell count of late basophilic and polychromatic erythroblasts in the patient.
16. The method of item 1, 3, or 4, wherein said antagonist of GDF11 reduces GDF11 expression, GDF11 activity, and/or GDF11 protein levels.

## Claims

1. An antagonist of GDF11 for use in a method for treating anemia, ineffective erythropoiesis, beta thalassemia, or for increasing Orthochromatic Erythroblasts (Ery-C) in a patient, wherein said antagonist of GDF11 does not comprise a ligand-binding portion of ActRIIA and/or a ligand-binding portion of ActRIIB.

2. The antagonist of GDF11 for use of clam 1, wherein the patient has elevated GDF11 levels in bone marrow, spleen, liver, serum, or plasma.

3. The antagonist of GDF11 for use of clam 1 or 2, wherein said antagonist of GDF11 reduces expression of GDF11, reduces GDF11 activity, or reduces GDF11 protein levels.

4. The antagonist of GDF11 for use of claim 1 or 2, wherein said antagonist of GDF11 reduces GDF11 expression, GDF11 activity, and GDF11 protein levels.

5. The antagonist of GDF11 for use of claim 1 or 2, wherein said method results in a decreased cell count of late basophilic and polychromatic erythroblasts in the patient.

6. The antagonist of GDF11 for use of any one of clams 1 to 5, wherein said antagonist of GDF11 is an anti GDF11 antibody.

7. The antagonist of GDF11 for use of any one of clams 1 to 5, wherein said antagonist of GDF11 is a truncated receptor that binds GDF11.

8. The antagonist of GDF11 for use of any one of claims 1 to 7, wherein the method further comprises monitoring GDF11 levels.

9. The antagonist of GDF11 for use of any one of claims 1 to 7, wherein the method further comprises:
a. determining GDF11 levels; and
b. adjusting the dose of the antagonist of GDF11,
wherein, if GDF11 is increased over normal, the dose of antagonist of GDF11 is increased; and
wherein, if GDF11 is decreased below normal, the dose of antagonist of GDF11 is decreased.

10. The antagonist of GDF11 for use of claim 8 or 9, wherein the GDF11 levels are determined as GDF11 mRNA levels, GDF11 protein levels, or GDF11 protein activity levels.


SEQUENCE LISTING

<110> Celgene Corporation

<120> METHODS FOR TREATING ANEMIA

<130> CD103809PCEPT1

<140> Divisional patent application based on EP 13849960.3
<141> October 23, 2013

<150> 61/718,128
<151> 2012-10-24

<160> 53

<170> FastSEQ for Windows Version 4.0

<210> 1
<211> 513
<212> PRT
<213> Homo sapiens

<220>
<223> human ActRIIA precursor polypeptide

<400> 1
Met Gly Ala Ala Ala Lys Leu Ala Phe Ala Val Phe Leu Ile Ser Cys
 1               5                  10                  15
Ser Ser Gly Ala Ile Leu Gly Arg Ser Glu Thr Gln Glu Cys Leu Phe
            20                  25                  30
Phe Asn Ala Asn Trp Glu Lys Asp Arg Thr Asn Gln Thr Gly Val Glu
        35                  40                  45
Pro Cys Tyr Gly Asp Lys Asp Lys Arg Arg His Cys Phe Ala Thr Trp
    50                  55                  60
Lys Asn Ile Ser Gly Ser Ile Glu Ile Val Lys Gln Gly Cys Trp Leu
65                  70                  75                  80
Asp Asp Ile Asn Cys Tyr Asp Arg Thr Asp Cys Val Glu Lys Lys Asp
                85                  90                  95
Ser Pro Glu Val Tyr Phe Cys Cys Cys Glu Gly Asn Met Cys Asn Glu
            100                 105                 110
Lys Phe Ser Tyr Phe Pro Glu Met Glu Val Thr Gln Pro Thr Ser Asn
        115                 120                 125
Pro Val Thr Pro Lys Pro Pro Tyr Tyr Asn Ile Leu Leu Tyr Ser Leu
    130                 135                 140
Val Pro Leu Met Leu Ile Ala Gly Ile Val Ile Cys Ala Phe Trp Val
145                 150                 155                 160
Tyr Arg His His Lys Met Ala Tyr Pro Pro Val Leu Val Pro Thr Gln
                165                 170                 175
Asp Pro Gly Pro Pro Pro Pro Ser Pro Leu Leu Gly Leu Lys Pro Leu
            180                 185                 190
Gln Leu Leu Glu Val Lys Ala Arg Gly Arg Phe Gly Cys Val Trp Lys
        195                 200                 205
Ala Gln Leu Leu Asn Glu Tyr Val Ala Val Lys Ile Phe Pro Ile Gln
    210                 215                 220
Asp Lys Gln Ser Trp Gln Asn Glu Tyr Glu Val Tyr Ser Leu Pro Gly
225                 230                 235                 240
Met Lys His Glu Asn Ile Leu Gln Phe Ile Gly Ala Glu Lys Arg Gly
                245                 250                 255
Thr Ser Val Asp Val Asp Leu Trp Leu Ile Thr Ala Phe His Glu Lys
            260                 265                 270
Gly Ser Leu Ser Asp Phe Leu Lys Ala Asn Val Val Ser Trp Asn Glu

275                 280                 285
Leu Cys His Ile Ala Glu Thr Met Ala Arg Gly Leu Ala Tyr Leu His
    290                 295                 300
Glu Asp Ile Pro Gly Leu Lys Asp Gly His Lys Pro Ala Ile Ser His
305                 310                 315                 320
Arg Asp Ile Lys Ser Lys Asn Val Leu Leu Lys Asn Asn Leu Thr Ala
                325                 330                 335
Cys Ile Ala Asp Phe Gly Leu Ala Leu Lys Phe Glu Ala Gly Lys Ser
            340                 345                 350
Ala Gly Asp Thr His Gly Gln Val Gly Thr Arg Arg Tyr Met Ala Pro
        355                 360                 365
Glu Val Leu Glu Gly Ala Ile Asn Phe Gln Arg Asp Ala Phe Leu Arg
    370                 375                 380
Ile Asp Met Tyr Ala Met Gly Leu Val Leu Trp Glu Leu Ala Ser Arg
385                 390                 395                 400
Cys Thr Ala Ala Asp Gly Pro Val Asp Glu Tyr Met Leu Pro Phe Glu
                405                 410                 415
Glu Glu Ile Gly Gln His Pro Ser Leu Glu Asp Met Gln Glu Val Val
            420                 425                 430
Val His Lys Lys Lys Arg Pro Val Leu Arg Asp Tyr Trp Gln Lys His
        435                 440                 445
Ala Gly Met Ala Met Leu Cys Glu Thr Ile Glu Glu Cys Trp Asp His
    450                 455                 460
Asp Ala Glu Ala Arg Leu Ser Ala Gly Cys Val Gly Glu Arg Ile Thr
465                 470                 475                 480
Gln Met Gln Arg Leu Thr Asn Ile Ile Thr Thr Glu Asp Ile Val Thr
                485                 490                 495
Val Val Thr Met Val Thr Asn Val Asp Phe Pro Pro Lys Glu Ser Ser
            500                 505                 510
Leu

<210> 2
<211> 115
<212> PRT
<213> Homo sapiens

<220>
<223> human ActRIIA soluble (extracellular), processed polypeptide

<400> 2
Ile Leu Gly Arg Ser Glu Thr Gln Glu Cys Leu Phe Phe Asn Ala Asn
1               5                   10                  15
Trp Glu Lys Asp Arg Thr Asn Gln Thr Gly Val Glu Pro Cys Tyr Gly
            20                  25                  30
Asp Lys Asp Lys Arg Arg His Cys Phe Ala Thr Trp Lys Asn Ile Ser
        35                  40                  45
Gly Ser Ile Glu Ile Val Lys Gln Gly Cys Trp Leu Asp Asp Ile Asn
    50                  55                  60
Cys Tyr Asp Arg Thr Asp Cys Val Glu Lys Lys Asp Ser Pro Glu Val
65                  70                  75                  80
Tyr Phe Cys Cys Cys Glu Gly Asn Met Cys Asn Glu Lys Phe Ser Tyr
                85                  90                  95
Phe Pro Glu Met Glu Val Thr Gln Pro Thr Ser Asn Pro Val Thr Pro
            100                 105                 110
Lys Pro Pro
        115

<210> 3
<211> 100
<212> PRT

<213> Artificial Sequence

<220>
<223> human ActRIIA soluble (extracellular), processed polypeptide
      with the C-terminal 15 amino acids deleted

<400> 3
Ile Leu Gly Arg Ser Glu Thr Gln Glu Cys Leu Phe Phe Asn Ala Asn
 1               5                   10                  15
Trp Glu Lys Asp Arg Thr Asn Gln Thr Gly Val Glu Pro Cys Tyr Gly
            20                  25                  30
Asp Lys Asp Lys Arg Arg His Cys Phe Ala Thr Trp Lys Asn Ile Ser
        35                  40                  45
Gly Ser Ile Glu Ile Val Lys Gln Gly Cys Trp Leu Asp Asp Ile Asn
    50                  55                  60
Cys Tyr Asp Arg Thr Asp Cys Val Glu Lys Lys Asp Ser Pro Glu Val
65                  70                  75                  80
Tyr Phe Cys Cys Cys Glu Gly Asn Met Cys Asn Glu Lys Phe Ser Tyr
                85                  90                  95
Phe Pro Glu Met
            100


<210> 4
<211> 1542
<212> DNA
<213> Homo sapiens

<220>
<223> human ActRIIA precursor

<400> 4
atgggagctg ctgcaaagtt ggcgtttgcc gtctttctta tctcctgttc ttcaggtgct 60
atacttggta gatcagaaac tcaggagtgt cttttcttta atgctaattg ggaaaaagac 120
agaaccaatc aaactggtgt tgaaccgtgt tatggtgaca aagataaacg gcggcattgt 180
tttgctacct ggaagaatat ttctggttcc attgaaatag tgaaacaagg ttgttggctg 240
gatgatatca actgctatga caggactgat tgtgtagaaa aaaaagacag ccctgaagta 300
tatttttgtt gctgtgaggg caatatgtgt aatgaaaagt tttcttattt tccagagatg 360
gaagtcacac agcccacttc aaatccagtt acacctaagc caccctatta caacatcctg 420
ctctattcct tggtgccact tatgttaatt gcggggattg tcatttgtgc attttgggtg 480
tacaggcatc acaagatggc ctaccctcct gtacttgttc caactcaaga cccaggacca 540
cccccacctt ctccattact agggttgaaa ccactgcagt tattagaagt gaaagcaagg 600
ggaagatttg gttgtgtctg gaaagcccag ttgcttaacg aatatgtggc tgtcaaaata 660
tttccaatac aggacaaaca gtcatggcaa aatgaatacg aagtctacag tttgcctgga 720
atgaagcatg agaacatatt acagttcatt ggtgcagaaa aacgaggcac cagtgttgat 780
gtggatcttt ggctgatcac agcatttcat gaaaagggtt cactatcaga ctttcttaag 840
gctaatgtgg tctcttggaa tgaactgtgt catattgcag aaaccatggc tagaggattg 900
gcatatttac atgaggatat acctggccta aaagatggcc acaaacctgc catatctcac 960
agggacatca aaagtaaaaa tgtgctgttg aaaaacaacc tgacagcttg cattgctgac 1020
tttgggttgg ccttaaaatt tgaggctggc aagtctgcag gcgataccca tggacaggtt 1080
ggtacccgga ggtacatggc tccagaggta ttagagggtg ctataaactt cgaaagggat 1140
gcatttttga ggatagatat gtatgccatg ggattagtcc tatgggaact ggcttctcgc 1200
tgtactgctg cagatggacc tgtagatgaa tacatgttgc catttgagga ggaaattggc 1260
cagcatccat ctcttgaaga catgcaggaa gttgttgtgc ataaaaaaaa gaggcctgtt 1320
ttaagagatt attggcagaa acatgctgga atggcaatgc tctgtgaaac cattgaagaa 1380
tgttgggatc acgacgcaga agccaggtta tcagctggat gtgtaggtga aagaattacc 1440
cagatgcaga gactaacaaa tattattacc acagaggaca ttgtaacagt ggtcacaatg 1500
gtgacaaatg ttgactttcc tcccaaagaa tctagtctat ga                   1542

<210> 5
<211> 345
<212> DNA
<213> Homo sapiens

<220>
<223> human ActRIIA soluble (extracellular) polypeptide

<400> 5
atacttggta gatcagaaac tcaggagtgt cttttcttta atgctaattg ggaaaaagac 60
agaaccaatc aaactggtgt tgaaccgtgt tatggtgaca aagataaacg gcggcattgt 120
tttgctacct ggaagaatat ttctggttcc attgaaatag tgaaacaagg ttgttggctg 180
gatgatatca actgctatga caggactgat tgtgtagaaa aaaaagacag ccctgaagta 240
tatttttgtt gctgtgaggg caatatgtgt aatgaaaagt tttcttattt tccagagatg 300
gaagtcacac agcccacttc aaatccagtt acacctaagc caccc 345

<210> 6
<211> 225
<212> PRT
<213> Artificial Sequence

<220>
<223> Synthetic construct - fusion protein comprising a soluble
      extracellular domain of ActRIIA fused to an Fc domain

<220>
<221> MOD_RES
<222> (43)..(43)
<223> Asp or Ala

<220>
<221> MOD_RES
<222> (100)..(100)
<223> Lys or Ala

<220>
<221> MOD_RES
<222> (212)..(212)
<223> Asn or Ala

<400> 6
Thr His Thr Cys Pro Pro Cys Pro Ala Pro Glu Leu Leu Gly Gly Pro
1               5                   10                  15
Ser Val Phe Leu Phe Pro Pro Lys Pro Lys Asp Thr Leu Met Ile Ser
            20                  25                  30
Arg Thr Pro Glu Val Thr Cys Val Val Val Xaa Val Ser His Glu Asp
        35                  40                  45
Pro Glu Val Lys Phe Asn Trp Tyr Val Asp Gly Val Glu Val His Asn
    50                  55                  60
Ala Lys Thr Lys Pro Arg Glu Glu Gln Tyr Asn Ser Thr Tyr Arg Val
65                  70                  75                  80
Val Ser Val Leu Thr Val Leu His Gln Asp Trp Leu Asn Gly Lys Glu
                85                  90                  95
Tyr Lys Cys Xaa Val Ser Asn Lys Ala Leu Pro Val Pro Ile Glu Lys
            100                 105                 110
Thr Ile Ser Lys Ala Lys Gly Gln Pro Arg Glu Pro Gln Val Tyr Thr
        115                 120                 125
Leu Pro Pro Ser Arg Glu Glu Met Thr Lys Asn Gln Val Ser Leu Thr
    130                 135                 140
Cys Leu Val Lys Gly Phe Tyr Pro Ser Asp Ile Ala Val Glu Trp Glu
145                 150                 155                 160
Ser Asn Gly Gln Pro Glu Asn Asn Tyr Lys Thr Thr Pro Pro Val Leu
                165                 170                 175
Asp Ser Asp Gly Pro Phe Phe Leu Tyr Ser Lys Leu Thr Val Asp Lys
            180                 185                 190
Ser Arg Trp Gln Gln Gly Asn Val Phe Ser Cys Ser Val Met His Glu
        195                 200                 205

Ala Leu His Xaa His Tyr Thr Gln Lys Ser Leu Ser Leu Ser Pro Gly
    210                 215                 220
Lys
225

<210> 7
<211> 344
<212> PRT
<213> Artificial Sequence

<220>
<223> Synthetic construct - Extracellular domain of human ActRIIA
fused to a human Fc domain

<400> 7
Ile Leu Gly Arg Ser Glu Thr Gln Glu Cys Leu Phe Phe Asn Ala Asn
1               5                   10                  15
Trp Glu Lys Asp Arg Thr Asn Gln Thr Gly Val Glu Pro Cys Tyr Gly
            20                  25                  30
Asp Lys Asp Lys Arg Arg His Cys Phe Ala Thr Trp Lys Asn Ile Ser
        35                  40                  45
Gly Ser Ile Glu Ile Val Lys Gln Gly Cys Trp Leu Asp Asp Ile Asn
    50                  55                  60
Cys Tyr Asp Arg Thr Asp Cys Val Glu Lys Lys Asp Ser Pro Glu Val
65                  70                  75                  80
Tyr Phe Cys Cys Cys Glu Gly Asn Met Cys Asn Glu Lys Phe Ser Tyr
                85                  90                  95
Phe Pro Glu Met Glu Val Thr Gln Pro Thr Ser Asn Pro Val Thr Pro
            100                 105                 110
Lys Pro Pro Thr Gly Gly Gly Thr His Thr Cys Pro Pro Cys Pro Ala
        115                 120                 125
Pro Glu Leu Leu Gly Gly Pro Ser Val Phe Leu Phe Pro Pro Lys Pro
    130                 135                 140
Lys Asp Thr Leu Met Ile Ser Arg Thr Pro Glu Val Thr Cys Val Val
145                 150                 155                 160
Val Asp Val Ser His Glu Asp Pro Glu Val Lys Phe Asn Trp Tyr Val
                165                 170                 175
Asp Gly Val Glu Val His Asn Ala Lys Thr Lys Pro Arg Glu Glu Gln
            180                 185                 190
Tyr Asn Ser Thr Tyr Arg Val Val Ser Val Leu Thr Val Leu His Gln
        195                 200                 205
Asp Trp Leu Asn Gly Lys Glu Tyr Lys Cys Lys Val Ser Asn Lys Ala
    210                 215                 220
Leu Pro Val Pro Ile Glu Lys Thr Ile Ser Lys Ala Lys Gly Gln Pro
225                 230                 235                 240
Arg Glu Pro Gln Val Tyr Thr Leu Pro Pro Ser Arg Glu Glu Met Thr
                245                 250                 255
Lys Asn Gln Val Ser Leu Thr Cys Leu Val Lys Gly Phe Tyr Pro Ser
            260                 265                 270
Asp Ile Ala Val Glu Trp Glu Ser Asn Gly Gln Pro Glu Asn Asn Tyr
        275                 280                 285
Lys Thr Thr Pro Pro Val Leu Asp Ser Asp Gly Ser Phe Phe Leu Tyr
    290                 295                 300
Ser Lys Leu Thr Val Asp Lys Ser Arg Trp Gln Gln Gly Asn Val Phe
305                 310                 315                 320
Ser Cys Ser Val Met His Glu Ala Leu His Asn His Tyr Thr Gln Lys
                325                 330                 335
Ser Leu Ser Leu Ser Pro Gly Lys
            340

<210> 8

<211> 21
<212> PRT
<213> Apis mellifera

<220>
<223> Leader sequence of Honey bee mellitin

<400> 8
Met Lys Phe Leu Val Asn Val Ala Leu Val Phe Met Val Val Tyr Ile
1               5                   10                  15
Ser Tyr Ile Tyr Ala
            20


<210> 9
<211> 22
<212> PRT
<213> Unknown

<220>
<223> Leader sequence of Tissue Plasminogen Activator (TPA)

<400> 9
Met Asp Ala Met Lys Arg Gly Leu Cys Cys Val Leu Leu Leu Cys Gly
1               5                   10                  15
Ala Val Phe Val Ser Pro
            20


<210> 10
<211> 20
<212> PRT
<213> Unknown

<220>
<223> Native ActRIIA leader

<400> 10
Met Gly Ala Ala Ala Lys Leu Ala Phe Ala Val Phe Leu Ile Ser Cys
1               5                   10                  15
Ser Ser Gly Ala
            20


<210> 11
<211> 9
<212> PRT
<213> Artificial Sequence

<220>
<223> Synthetic construct - ActRIIA-hFc and ActRIIA-mFc N-terminal
      sequence

<400> 11
Ile Leu Gly Arg Ser Glu Thr Gln Glu
1               5


<210> 12
<211> 329
<212> PRT
<213> Artificial Sequence

<220>
<223> Synthetic construct - ActRIIA-Fc Protein with deletion of the
      C-terminal 15 amino acids of the extracellular domain of ActRIIA

<400> 12
Ile Leu Gly Arg Ser Glu Thr Gln Glu Cys Leu Phe Phe Asn Ala Asn
1               5                   10                  15
Trp Glu Lys Asp Arg Thr Asn Gln Thr Gly Val Glu Pro Cys Tyr Gly
            20                  25                  30
Asp Lys Asp Lys Arg Arg His Cys Phe Ala Thr Trp Lys Asn Ile Ser
        35                  40                  45
Gly Ser Ile Glu Ile Val Lys Gln Gly Cys Trp Leu Asp Asp Ile Asn
    50                  55                  60
Cys Tyr Asp Arg Thr Asp Cys Val Glu Lys Lys Asp Ser Pro Glu Val
65                  70                  75                  80
Tyr Phe Cys Cys Cys Glu Gly Asn Met Cys Asn Glu Lys Phe Ser Tyr
                85                  90                  95
Phe Pro Glu Met Thr Gly Gly Gly Thr His Thr Cys Pro Pro Cys Pro
            100                 105                 110
Ala Pro Glu Leu Leu Gly Gly Pro Ser Val Phe Leu Phe Pro Pro Lys
        115                 120                 125
Pro Lys Asp Thr Leu Met Ile Ser Arg Thr Pro Glu Val Thr Cys Val
    130                 135                 140
Val Val Asp Val Ser His Glu Asp Pro Glu Val Lys Phe Asn Trp Tyr
145                 150                 155                 160
Val Asp Gly Val Glu Val His Asn Ala Lys Thr Lys Pro Arg Glu Glu
                165                 170                 175
Gln Tyr Asn Ser Thr Tyr Arg Val Val Ser Val Leu Thr Val Leu His
            180                 185                 190
Gln Asp Trp Leu Asn Gly Lys Glu Tyr Lys Cys Lys Val Ser Asn Lys
        195                 200                 205
Ala Leu Pro Val Pro Ile Glu Lys Thr Ile Ser Lys Ala Lys Gly Gln
    210                 215                 220
Pro Arg Glu Pro Gln Val Tyr Thr Leu Pro Pro Ser Arg Glu Glu Met
225                 230                 235                 240
Thr Lys Asn Gln Val Ser Leu Thr Cys Leu Val Lys Gly Phe Tyr Pro
                245                 250                 255
Ser Asp Ile Ala Val Glu Trp Glu Ser Asn Gly Gln Pro Glu Asn Asn
            260                 265                 270
Tyr Lys Thr Thr Pro Pro Val Leu Asp Ser Asp Gly Ser Phe Phe Leu
        275                 280                 285
Tyr Ser Lys Leu Thr Val Asp Lys Ser Arg Trp Gln Gln Gly Asn Val
    290                 295                 300
Phe Ser Cys Ser Val Met His Glu Ala Leu His Asn His Tyr Thr Gln
305                 310                 315                 320
Lys Ser Leu Ser Leu Ser Pro Gly Lys
                325

<210> 13
<211> 369
<212> PRT
<213> Artificial Sequence

<220>
<223> Synthetic construct - Unprocessed ActRIIA-hFc with TPA
      leader sequence

<400> 13
Met Asp Ala Met Lys Arg Gly Leu Cys Cys Val Leu Leu Leu Cys Gly
1               5                   10                  15
Ala Val Phe Val Ser Pro Gly Ala Ala Ile Leu Gly Arg Ser Glu Thr
            20                  25                  30

Gln Glu Cys Leu Phe Phe Asn Ala Asn Trp Glu Lys Asp Arg Thr Asn
        35                  40                  45
Gln Thr Gly Val Glu Pro Cys Tyr Gly Asp Lys Asp Lys Arg Arg His
    50                  55                  60
Cys Phe Ala Thr Trp Lys Asn Ile Ser Gly Ser Ile Glu Ile Val Lys
65                  70                  75                  80
Gln Gly Cys Trp Leu Asp Asp Ile Asn Cys Tyr Asp Arg Thr Asp Cys
                85                  90                  95
Val Glu Lys Lys Asp Ser Pro Glu Val Tyr Phe Cys Cys Cys Glu Gly
            100                 105                 110
Asn Met Cys Asn Glu Lys Phe Ser Tyr Phe Pro Glu Met Glu Val Thr
        115                 120                 125
Gln Pro Thr Ser Asn Pro Val Thr Pro Lys Pro Pro Thr Gly Gly Gly
    130                 135                 140
Thr His Thr Cys Pro Pro Cys Pro Ala Pro Glu Leu Leu Gly Gly Pro
145                 150                 155                 160
Ser Val Phe Leu Phe Pro Pro Lys Pro Lys Asp Thr Leu Met Ile Ser
                165                 170                 175
Arg Thr Pro Glu Val Thr Cys Val Val Val Asp Val Ser His Glu Asp
            180                 185                 190
Pro Glu Val Lys Phe Asn Trp Tyr Val Asp Gly Val Glu Val His Asn
        195                 200                 205
Ala Lys Thr Lys Pro Arg Glu Glu Gln Tyr Asn Ser Thr Tyr Arg Val
    210                 215                 220
Val Ser Val Leu Thr Val Leu His Gln Asp Trp Leu Asn Gly Lys Glu
225                 230                 235                 240
Tyr Lys Cys Lys Val Ser Asn Lys Ala Leu Pro Val Pro Ile Glu Lys
                245                 250                 255
Thr Ile Ser Lys Ala Lys Gly Gln Pro Arg Glu Pro Gln Val Tyr Thr
            260                 265                 270
Leu Pro Pro Ser Arg Glu Glu Met Thr Lys Asn Gln Val Ser Leu Thr
        275                 280                 285
Cys Leu Val Lys Gly Phe Tyr Pro Ser Asp Ile Ala Val Glu Trp Glu
    290                 295                 300
Ser Asn Gly Gln Pro Glu Asn Asn Tyr Lys Thr Thr Pro Pro Val Leu
305                 310                 315                 320
Asp Ser Asp Gly Ser Phe Phe Leu Tyr Ser Lys Leu Thr Val Asp Lys
                325                 330                 335
Ser Arg Trp Gln Gln Gly Asn Val Phe Ser Cys Ser Val Met His Glu
            340                 345                 350
Ala Leu His Asn His Tyr Thr Gln Lys Ser Leu Ser Leu Ser Pro Gly
        355                 360                 365
Lys

<210> 14
<211> 1114
<212> DNA
<213> Artificial Sequence

<220>
<223> Synthetic construct - Unprocessed ActRIIA-hFc with TPA leader sequence

<400> 14
atggatgcaa tgaagagagg gctctgctgt gtgctgctgc tgtgtggagc agtcttcgtt 60
tcgcccggcg ccgctatact tggtagatca gaaactcagg agtgtctttt tttaatgcta 120
attgggaaaa agacagaacc aatcaaactg gtgttgaacc gtgttatggt gacaaagata 180
aacggcggca ttgttttgct acctggaaga atatttctgg ttccattgaa tagtgaaaca 240
aggttgttgg ctggatgata tcaactgcta tgacaggact gattgtgtag aaaaaaaaga 300
cagccctgaa gtatatttct gttgctgtga gggcaatatg tgtaatgaaa agttttctta 360
ttttccggag atggaagtca cacagcccac ttcaaatcca gttacaccta agccacccac 420
cggtggtgga actcacacat gcccaccgtg cccagcacct gaactcctgg ggggaccgtc 480

agtcttcctc ttccccccaa aacccaagga caccctcatg atctcccgga cccctgaggt 540
cacatgcgtg gtggtggacg tgagccacga agaccctgag gtcaagttca actggtacgt 600
ggacggcgtg gaggtgcata atgccaagac aaagccgcgg gaggagcagt acaacagcac 660
gtaccgtgtg gtcagcgtcc tcaccgtcct gcaccaggac tggctgaatg gcaaggagta 720
caagtgcaag gtctccaaca aagccctccc agtccccatc gagaaaacca tctccaaagc 780
caaagggcag ccccgagaac cacaggtgta caccctgccc ccatcccggg aggagatgac 840
caagaaccag gtcagcctga cctgcctggt caaaggcttc tatcccagcg acatcgccgt 900
ggagtgggag agcaatgggc agccggagaa caactacaag accacgcctc ccgtgctgga 960
ctccgacggc tccttcttcc tctatagcaa gctcaccgtg gacaagagca ggtggcagca 1020
ggggaacgtc ttctcatgct ccgtgatgca tgaggctctg cacaaccact acacgcagaa 1080
gagcctctcc ctgtctccgg gtaaatgaga attc 1114

<210> 15
<211> 106
<212> PRT
<213> Artificial Sequence

<220>
<223> Synthetic construct - human ActRIIB soluble (extracellular), processed
      polypeptide sequence with the N-terminal 6 amino acid of the EC domain
      deleted and the C-terminal 4 amino acids of the EC domain deleted

<400> 15
Glu Thr Arg Glu Cys Ile Tyr Tyr Asn Ala Asn Trp Glu Leu Glu Arg
1               5                   10                  15
Thr Asn Gln Ser Gly Leu Glu Arg Cys Glu Gly Glu Gln Asp Lys Arg
            20                  25                  30
Leu His Cys Tyr Ala Ser Trp Arg Asn Ser Ser Gly Thr Ile Glu Leu
        35                  40                  45
Val Lys Lys Gly Cys Trp Asp Asp Asp Phe Asn Cys Tyr Asp Arg Gln
    50                  55                  60
Glu Cys Val Ala Thr Glu Glu Asn Pro Gln Val Tyr Phe Cys Cys Cys
65                  70                  75                  80
Glu Gly Asn Phe Cys Asn Glu Arg Phe Thr His Leu Pro Glu Ala Gly
                85                  90                  95
Gly Pro Glu Val Thr Tyr Glu Pro Pro Pro
            100                 105

<210> 16
<211> 512
<212> PRT
<213> Homo sapiens

<220>
<223> human ActRIIB precursor protein

<400> 16
Met Thr Ala Pro Trp Val Ala Leu Ala Leu Leu Trp Gly Ser Leu Trp
1               5                   10                  15
Pro Gly Ser Gly Arg Gly Glu Ala Glu Thr Arg Glu Cys Ile Tyr Tyr
            20                  25                  30
Asn Ala Asn Trp Glu Leu Glu Arg Thr Asn Gln Ser Gly Leu Glu Arg
        35                  40                  45
Cys Glu Gly Glu Gln Asp Lys Arg Leu His Cys Tyr Ala Ser Trp Ala
    50                  55                  60
Asn Ser Ser Gly Thr Ile Glu Leu Val Lys Lys Gly Cys Trp Leu Asp
65                  70                  75                  80
Asp Phe Asn Cys Tyr Asp Arg Gln Glu Cys Val Ala Thr Glu Glu Asn
                85                  90                  95
Pro Gln Val Tyr Phe Cys Cys Cys Glu Gly Asn Phe Cys Asn Glu Arg
            100                 105                 110
Phe Thr His Leu Pro Glu Ala Gly Gly Pro Glu Val Thr Tyr Glu Pro

115                 120                 125
Pro Pro Thr Ala Pro Thr Leu Leu Thr Val Leu Ala Tyr Ser Leu Leu
    130                 135                 140
Pro Ile Gly Gly Leu Ser Leu Ile Val Leu Leu Ala Phe Trp Met Tyr
145                 150                 155                 160
Arg His Arg Lys Pro Pro Tyr Gly His Val Asp Ile His Glu Asp Pro
                165                 170                 175
Gly Pro Pro Pro Pro Ser Pro Leu Val Gly Leu Lys Pro Leu Gln Leu
            180                 185                 190
Leu Glu Ile Lys Ala Arg Gly Arg Phe Gly Cys Val Trp Lys Ala Gln
        195                 200                 205
Leu Met Asn Asp Phe Val Ala Val Lys Ile Phe Pro Leu Gln Asp Lys
    210                 215                 220
Gln Ser Trp Gln Ser Glu Arg Glu Ile Phe Ser Thr Pro Gly Met Lys
225                 230                 235                 240
His Glu Asn Leu Leu Gln Phe Ile Ala Ala Glu Lys Arg Gly Ser Asn
                245                 250                 255
Leu Glu Val Glu Leu Trp Leu Ile Thr Ala Phe His Asp Lys Gly Ser
            260                 265                 270
Leu Thr Asp Tyr Leu Lys Gly Asn Ile Ile Thr Trp Asn Glu Leu Cys
        275                 280                 285
His Val Ala Glu Thr Met Ser Arg Gly Leu Ser Tyr Leu His Glu Asp
    290                 295                 300
Val Pro Trp Cys Arg Gly Glu Gly His Lys Pro Ser Ile Ala His Arg
305                 310                 315                 320
Asp Phe Lys Ser Lys Asn Val Leu Leu Lys Ser Asp Leu Thr Ala Val
                325                 330                 335
Leu Ala Asp Phe Gly Leu Ala Val Arg Phe Glu Pro Gly Lys Pro Pro
            340                 345                 350
Gly Asp Thr His Gly Gln Val Gly Thr Arg Arg Tyr Met Ala Pro Glu
        355                 360                 365
Val Leu Glu Gly Ala Ile Asn Phe Gln Arg Asp Ala Phe Leu Arg Ile
    370                 375                 380
Asp Met Tyr Ala Met Gly Leu Val Leu Trp Glu Leu Val Ser Arg Cys
385                 390                 395                 400
Lys Ala Ala Asp Gly Pro Val Asp Glu Tyr Met Leu Pro Phe Glu Glu
                405                 410                 415
Glu Ile Gly Gln His Pro Ser Leu Glu Glu Leu Gln Glu Val Val Val
            420                 425                 430
His Lys Lys Met Arg Pro Thr Ile Lys Asp His Trp Leu Lys His Pro
        435                 440                 445
Gly Leu Ala Gln Leu Cys Val Thr Ile Glu Glu Cys Trp Asp His Asp
    450                 455                 460
Ala Glu Ala Arg Leu Ser Ala Gly Cys Val Glu Glu Arg Val Ser Leu
465                 470                 475                 480
Ile Arg Arg Ser Val Asn Gly Thr Thr Ser Asp Cys Leu Val Ser Leu
                485                 490                 495
Val Thr Ser Val Thr Asn Val Asp Leu Pro Pro Lys Glu Ser Ser Ile
            500                 505                 510


<210> 17
<211> 116
<212> PRT
<213> Homo sapiens

<220>
<223> human ActRIIB soluble (extracellular), processed polypeptide

<400> 17
Ser Gly Arg Gly Glu Ala Glu Thr Arg Glu Cys Ile Tyr Tyr Asn Ala
1               5                   10                  15
Asn Trp Glu Leu Glu Arg Thr Asn Gln Ser Gly Leu Glu Arg Cys Glu

                    20                  25                  30
Gly Glu Gln Asp Lys Arg Leu His Cys Tyr Ala Ser Trp Ala Asn Ser
        35                  40                  45
Ser Gly Thr Ile Glu Leu Val Lys Lys Gly Cys Trp Leu Asp Asp Phe
    50                  55                  60
Asn Cys Tyr Asp Arg Gln Glu Cys Val Ala Thr Glu Glu Asn Pro Gln
65                  70                  75                  80
Val Tyr Phe Cys Cys Cys Glu Gly Asn Phe Cys Asn Glu Arg Phe Thr
                85                  90                  95
His Leu Pro Glu Ala Gly Gly Pro Glu Val Thr Tyr Glu Pro Pro Pro
            100                 105                 110
Thr Ala Pro Thr
        115

<210> 18
<211> 101
<212> PRT
<213> Artificial Sequence

<220>
<223> human ActRIIB soluble (extracellular), processed polypeptide
      sequence with the C-terminal 15 amino acids deleted

<400> 18
Ser Gly Arg Gly Glu Ala Glu Thr Arg Glu Cys Ile Tyr Tyr Asn Ala
1               5                   10                  15
Asn Trp Glu Leu Glu Arg Thr Asn Gln Ser Gly Leu Glu Arg Cys Glu
            20                  25                  30
Gly Glu Gln Asp Lys Arg Leu His Cys Tyr Ala Ser Trp Ala Asn Ser
        35                  40                  45
Ser Gly Thr Ile Glu Leu Val Lys Lys Gly Cys Trp Leu Asp Asp Phe
    50                  55                  60
Asn Cys Tyr Asp Arg Gln Glu Cys Val Ala Thr Glu Glu Asn Pro Gln
65                  70                  75                  80
Val Tyr Phe Cys Cys Cys Glu Gly Asn Phe Cys Asn Glu Arg Phe Thr
                85                  90                  95
His Leu Pro Glu Ala
            100

<210> 19
<211> 1539
<212> DNA
<213> Homo sapiens

<220>
<223> human ActRIIB (A64) precursor

<400> 19
atgacggcgc cctgggtggc cctcgccctc ctctggggat cgctgtggcc cggctctggg 60
cgtggggagg ctgagacacg ggagtgcatc tactacaacg ccaactggga gctggagcgc 120
accaaccaga gcggcctgga gcgctgcgaa ggcgagcagg acaagcggct gcactgctac 180
gcctcctggg ccaacagctc tggcaccatc gagctcgtga agaagggctg ctggctagat 240
gacttcaact gctacgatag gcaggagtgt gtggccactg aggagaaccc ccaggtgtac 300
ttctgctgct gtgaaggcaa cttctgcaac gagcgcttca ctcatttgcc agaggctggg 360
ggcccggaag tcacgtacga gccacccccg acagccccca ccctgctcac ggtgctggcc 420
tactcactgc tgcccatcgg gggcctttcc ctcatcgtcc tgctggcctt ttggatgtac 480
cggcatcgca agccccccta cggtcatgtg gacatccatg aggaccctgg gcctccacca 540
ccatcccctc tggtgggcct gaagccactg cagctgctgg agatcaaggc tcgggggcgc 600
tttggctgtg tctggaaggc ccagctcatg aatgactttg tagctgtcaa gatcttccca 660
ctccaggaca agcagtcgtg gcagagtgaa cgggagatct tcagcacacc tggcatgaag 720
cacgagaacc tgctacagtt cattgctgcc gagaagcgag gctccaacct cgaagtagag 780

ctgtggctca tcacggcctt ccatgacaag ggctccctca cggattacct caaggggaac 840
atcatcacat ggaacgaact gtgtcatgta gcagagacga tgtcacgagg cctctcatac 900
ctgcatgagg atgtgccctg gtgccgtggc gagggccaca agccgtctat tgcccacagg 960
gactttaaaa gtaagaatgt attgctgaag agcgacctca cagccgtgct ggctgacttt 1020
ggcttggctg ttcgatttga gccagggaaa cctccagggg acacccacgg acaggtaggc 1080
acgagacggt acatggctcc tgaggtgctc gagggagcca tcaacttcca gagagatgcc 1140
ttcctgcgca ttgacatgta tgccatgggg ttggtgctgt gggagcttgt gtctcgctgc 1200
aaggctgcag acggacccgt ggatgagtac atgctgccct ttgaggaaga gattggccag 1260
cacccttcgt tggaggagct gcaggaggtg gtggtgcaca agaagatgag gcccaccatt 1320
aaagatcact ggttgaaaca cccgggcctg gcccagcttt gtgtgaccat cgaggagtgc 1380
tgggaccatg atgcagaggc tcgcttgtcc gcgggctgtg tggaggagcg ggtgtccctg 1440
attcggaggt cggtcaacgg cactacctcg gactgtctcg tttccctggt gacctctgtc 1500
accaatgtgg acctgccccc taaagagtca agcatctaa 1539

<210> 20
<211> 344
<212> PRT
<213> Artificial Sequence

<220>
<223> Synthetic construct - fusion protein comprising a soluble
      extracellular domain of ActRIIB fused to an Fc domain

<400> 20
Ser Gly Arg Gly Glu Ala Glu Thr Arg Glu Cys Ile Tyr Tyr Asn Ala
1               5                   10                  15
Asn Trp Glu Leu Glu Arg Thr Asn Gln Ser Gly Leu Glu Arg Cys Glu
            20                  25                  30
Gly Glu Gln Asp Lys Arg Leu His Cys Tyr Ala Ser Trp Ala Asn Ser
        35                  40                  45
Ser Gly Thr Ile Glu Leu Val Lys Lys Gly Cys Trp Leu Asp Asp Phe
    50                  55                  60
Asn Cys Tyr Asp Arg Gln Glu Cys Val Ala Thr Glu Glu Asn Pro Gln
65                  70                  75                  80
Val Tyr Phe Cys Cys Cys Glu Gly Asn Phe Cys Asn Glu Arg Phe Thr
                85                  90                  95
His Leu Pro Glu Ala Gly Gly Pro Glu Val Thr Tyr Glu Pro Pro Pro
            100                 105                 110
Thr Ala Pro Thr Gly Gly Gly Thr His Thr Cys Pro Pro Cys Pro Ala
        115                 120                 125
Pro Glu Leu Leu Gly Gly Pro Ser Val Phe Leu Phe Pro Pro Lys Pro
    130                 135                 140
Lys Asp Thr Leu Met Ile Ser Arg Thr Pro Glu Val Thr Cys Val Val
145                 150                 155                 160
Val Asp Val Ser His Glu Asp Pro Glu Val Lys Phe Asn Trp Tyr Val
                165                 170                 175
Asp Gly Val Glu Val His Asn Ala Lys Thr Lys Pro Arg Glu Glu Gln
            180                 185                 190
Tyr Asn Ser Thr Tyr Arg Val Val Ser Val Leu Thr Val Leu His Gln
        195                 200                 205
Asp Trp Leu Asn Gly Lys Glu Tyr Lys Cys Lys Val Ser Asn Lys Ala
    210                 215                 220
Leu Pro Val Pro Ile Glu Lys Thr Ile Ser Lys Ala Lys Gly Gln Pro
225                 230                 235                 240
Arg Glu Pro Gln Val Tyr Thr Leu Pro Pro Ser Arg Glu Glu Met Thr
                245                 250                 255
Lys Asn Gln Val Ser Leu Thr Cys Leu Val Lys Gly Phe Tyr Pro Ser
            260                 265                 270
Asp Ile Ala Val Glu Trp Glu Ser Asn Gly Gln Pro Glu Asn Asn Tyr
        275                 280                 285
Lys Thr Thr Pro Pro Val Leu Asp Ser Asp Gly Ser Phe Phe Leu Tyr
    290                 295                 300
Ser Lys Leu Thr Val Asp Lys Ser Arg Trp Gln Gln Gly Asn Val Phe

305                 310                 315                 320
Ser Cys Ser Val Met His Glu Ala Leu His Asn His Tyr Thr Gln Lys
                325                 330                 335
Ser Leu Ser Leu Ser Pro Gly Lys
            340

<210> 21
<211> 329
<212> PRT
<213> Artificial Sequence

<220>
<223> Synthetic construct - fusion protein comprising a soluble
      extracellular domain of ActRIIB (A64) with the C-terminal 15
      amino acids deleted fused to an Fc domain

<400> 21
Ser Gly Arg Gly Glu Ala Glu Thr Arg Glu Cys Ile Tyr Tyr Asn Ala
1               5                   10                  15
Asn Trp Glu Leu Glu Arg Thr Asn Gln Ser Gly Leu Glu Arg Cys Glu
            20                  25                  30
Gly Glu Gln Asp Lys Arg Leu His Cys Tyr Ala Ser Trp Ala Asn Ser
        35                  40                  45
Ser Gly Thr Ile Glu Leu Val Lys Lys Gly Cys Trp Leu Asp Asp Phe
    50                  55                  60
Asn Cys Tyr Asp Arg Gln Glu Cys Val Ala Thr Glu Glu Asn Pro Gln
65                  70                  75                  80
Val Tyr Phe Cys Cys Cys Glu Gly Asn Phe Cys Asn Glu Arg Phe Thr
                85                  90                  95
His Leu Pro Glu Ala Gly Gly Gly Thr His Thr Cys Pro Pro Cys Pro
            100                 105                 110
Ala Pro Glu Leu Leu Gly Gly Pro Ser Val Phe Leu Phe Pro Pro Lys
        115                 120                 125
Pro Lys Asp Thr Leu Met Ile Ser Arg Thr Pro Glu Val Thr Cys Val
    130                 135                 140
Val Val Asp Val Ser His Glu Asp Pro Glu Val Lys Phe Asn Trp Tyr
145                 150                 155                 160
Val Asp Gly Val Glu Val His Asn Ala Lys Thr Lys Pro Arg Glu Glu
                165                 170                 175
Gln Tyr Asn Ser Thr Tyr Arg Val Val Ser Val Leu Thr Val Leu His
            180                 185                 190
Gln Asp Trp Leu Asn Gly Lys Glu Tyr Lys Cys Lys Val Ser Asn Lys
        195                 200                 205
Ala Leu Pro Val Pro Ile Glu Lys Thr Ile Ser Lys Ala Lys Gly Gln
    210                 215                 220
Pro Arg Glu Pro Gln Val Tyr Thr Leu Pro Pro Ser Arg Glu Glu Met
225                 230                 235                 240
Thr Lys Asn Gln Val Ser Leu Thr Cys Leu Val Lys Gly Phe Tyr Pro
                245                 250                 255
Ser Asp Ile Ala Val Glu Trp Glu Ser Asn Gly Gln Pro Glu Asn Asn
            260                 265                 270
Tyr Lys Thr Thr Pro Pro Val Leu Asp Ser Asp Gly Ser Phe Phe Leu
        275                 280                 285
Tyr Ser Lys Leu Thr Val Asp Lys Ser Arg Trp Gln Gln Gly Asn Val
    290                 295                 300
Phe Ser Cys Ser Val Met His Glu Ala Leu His Asn His Tyr Thr Gln
305                 310                 315                 320
Lys Ser Leu Ser Leu Ser Pro Gly Lys
                325

<210> 22

<211> 105
<212> PRT
<213> Artificial Sequence

<220>
<223> human ActRIIB soluble (extracellular), processed polypeptide
      with the N-terminal 6 amino acids of the EC domain
      deleted and the C-terminal 5 amino acids of the EC domain
      deleted and with an L79D mutation

<400> 22
Glu Thr Arg Glu Cys Ile Tyr Tyr Asn Ala Asn Trp Glu Leu Glu Arg
1               5                   10                  15
Thr Asn Gln Ser Gly Leu Glu Arg Cys Glu Gly Glu Gln Asp Lys Arg
            20                  25                  30
Leu His Cys Tyr Ala Ser Trp Arg Asn Ser Ser Gly Thr Ile Glu Leu
        35                  40                  45
Val Lys Lys Gly Cys Trp Asp Asp Asp Phe Asn Cys Tyr Asp Arg Gln
    50                  55                  60
Glu Cys Val Ala Thr Glu Glu Asn Pro Gln Val Tyr Phe Cys Cys Cys
65                  70                  75                  80
Glu Gly Asn Phe Cys Asn Glu Arg Phe Thr His Leu Pro Glu Ala Gly
                85                  90                  95
Gly Pro Glu Val Thr Tyr Glu Pro Pro
            100                 105


<210> 23
<211> 107
<212> PRT
<213> Artificial Sequence

<220>
<223> human ActRIIB soluble (extracellular), processed polypeptide
      sequence with the N-terminal 6 amino acids of the EC domain
      deleted and the C-terminal 3 amino acids of the EC domain
      deleted and with an L79D mutatioN

<400> 23
Glu Thr Arg Glu Cys Ile Tyr Tyr Asn Ala Asn Trp Glu Leu Glu Arg
1               5                   10                  15
Thr Asn Gln Ser Gly Leu Glu Arg Cys Glu Gly Glu Gln Asp Lys Arg
            20                  25                  30
Leu His Cys Tyr Ala Ser Trp Arg Asn Ser Ser Gly Thr Ile Glu Leu
        35                  40                  45
Val Lys Lys Gly Cys Trp Asp Asp Asp Phe Asn Cys Tyr Asp Arg Gln
    50                  55                  60
Glu Cys Val Ala Thr Glu Glu Asn Pro Gln Val Tyr Phe Cys Cys Cys
65                  70                  75                  80
Glu Gly Asn Phe Cys Asn Glu Arg Phe Thr His Leu Pro Glu Ala Gly
                85                  90                  95
Gly Pro Glu Val Thr Tyr Glu Pro Pro Pro Thr
            100                 105


<210> 24
<211> 360
<212> PRT
<213> Artificial Sequence

<220>
<223> Unprocessed ActRIIB-Fc fusion protein with the N-terminal 6
      amino acids of the EC domain deleted and the C-terminal 3

amino acids of the EC domain deleted and with an L79D
      mutation and with TPA leader sequence

<400> 24
Met Asp Ala Met Lys Arg Gly Leu Cys Cys Val Leu Leu Leu Cys Gly
1               5                   10                  15
Ala Val Phe Val Ser Pro Gly Ala Ala Glu Thr Arg Glu Cys Ile Tyr
            20                  25                  30
Tyr Asn Ala Asn Trp Glu Leu Glu Arg Thr Asn Gln Ser Gly Leu Glu
        35                  40                  45
Arg Cys Glu Gly Glu Gln Asp Lys Arg Leu His Cys Tyr Ala Ser Trp
    50                  55                  60
Arg Asn Ser Ser Gly Thr Ile Glu Leu Val Lys Lys Gly Cys Trp Asp
65                  70                  75                  80
Asp Asp Phe Asn Cys Tyr Asp Arg Gln Glu Cys Val Ala Thr Glu Glu
                85                  90                  95
Asn Pro Gln Val Tyr Phe Cys Cys Cys Glu Gly Asn Phe Cys Asn Glu
            100                 105                 110
Arg Phe Thr His Leu Pro Glu Ala Gly Gly Pro Glu Val Thr Tyr Glu
        115                 120                 125
Pro Pro Pro Thr Gly Gly Gly Thr His Thr Cys Pro Pro Cys Pro Ala
    130                 135                 140
Pro Glu Leu Leu Gly Gly Pro Ser Val Phe Leu Phe Pro Pro Lys Pro
145                 150                 155                 160
Lys Asp Thr Leu Met Ile Ser Arg Thr Pro Glu Val Thr Cys Val Val
                165                 170                 175
Val Asp Val Ser His Glu Asp Pro Glu Val Lys Phe Asn Trp Tyr Val
            180                 185                 190
Asp Gly Val Glu Val His Asn Ala Lys Thr Lys Pro Arg Glu Glu Gln
        195                 200                 205
Tyr Asn Ser Thr Tyr Arg Val Val Ser Val Leu Thr Val Leu His Gln
    210                 215                 220
Asp Trp Leu Asn Gly Lys Glu Tyr Lys Cys Lys Val Ser Asn Lys Ala
225                 230                 235                 240
Leu Pro Ala Pro Ile Glu Lys Thr Ile Ser Lys Ala Lys Gly Gln Pro
                245                 250                 255
Arg Glu Pro Gln Val Tyr Thr Leu Pro Pro Ser Arg Glu Glu Met Thr
            260                 265                 270
Lys Asn Gln Val Ser Leu Thr Cys Leu Val Lys Gly Phe Tyr Pro Ser
        275                 280                 285
Asp Ile Ala Val Glu Trp Glu Ser Asn Gly Gln Pro Glu Asn Asn Tyr
    290                 295                 300
Lys Thr Thr Pro Pro Val Leu Asp Ser Asp Gly Ser Phe Phe Leu Tyr
305                 310                 315                 320
Ser Lys Leu Thr Val Asp Lys Ser Arg Trp Gln Gln Gly Asn Val Phe
                325                 330                 335
Ser Cys Ser Val Met His Glu Ala Leu His Asn His Tyr Thr Gln Lys
            340                 345                 350
Ser Leu Ser Leu Ser Pro Gly Lys
        355                 360


<210> 25
<211> 335
<212> PRT
<213> Artificial Sequence

<220>
<223> Processed ActRIIB-Fc fusion protein with the N-terminal 6
      amino acids of the EC domain deleted and the C-terminal 3
      amino acids of the EC domain deleted and with an L79D mutation

<400> 25

Glu Thr Arg Glu Cys Ile Tyr Tyr Asn Ala Asn Trp Glu Leu Glu Arg
1               5                   10                  15
Thr Asn Gln Ser Gly Leu Glu Arg Cys Glu Gly Glu Gln Asp Lys Arg
            20                  25                  30
Leu His Cys Tyr Ala Ser Trp Arg Asn Ser Ser Gly Thr Ile Glu Leu
        35                  40                  45
Val Lys Lys Gly Cys Trp Asp Asp Asp Phe Asn Cys Tyr Asp Arg Gln
    50                  55                  60
Glu Cys Val Ala Thr Glu Glu Asn Pro Gln Val Tyr Phe Cys Cys Cys
65                  70                  75                  80
Glu Gly Asn Phe Cys Asn Glu Arg Phe Thr His Leu Pro Glu Ala Gly
                85                  90                  95
Gly Pro Glu Val Thr Tyr Glu Pro Pro Pro Thr Gly Gly Gly Thr His
            100                 105                 110
Thr Cys Pro Pro Cys Pro Ala Pro Glu Leu Leu Gly Gly Pro Ser Val
        115                 120                 125
Phe Leu Phe Pro Pro Lys Pro Lys Asp Thr Leu Met Ile Ser Arg Thr
    130                 135                 140
Pro Glu Val Thr Cys Val Val Val Asp Val Ser His Glu Asp Pro Glu
145                 150                 155                 160
Val Lys Phe Asn Trp Tyr Val Asp Gly Val Glu Val His Asn Ala Lys
                165                 170                 175
Thr Lys Pro Arg Glu Glu Gln Tyr Asn Ser Thr Tyr Arg Val Val Ser
            180                 185                 190
Val Leu Thr Val Leu His Gln Asp Trp Leu Asn Gly Lys Glu Tyr Lys
        195                 200                 205
Cys Lys Val Ser Asn Lys Ala Leu Pro Ala Pro Ile Glu Lys Thr Ile
    210                 215                 220
Ser Lys Ala Lys Gly Gln Pro Arg Glu Pro Gln Val Tyr Thr Leu Pro
225                 230                 235                 240
Pro Ser Arg Glu Glu Met Thr Lys Asn Gln Val Ser Leu Thr Cys Leu
                245                 250                 255
Val Lys Gly Phe Tyr Pro Ser Asp Ile Ala Val Glu Trp Glu Ser Asn
            260                 265                 270
Gly Gln Pro Glu Asn Asn Tyr Lys Thr Thr Pro Pro Val Leu Asp Ser
        275                 280                 285
Asp Gly Ser Phe Phe Leu Tyr Ser Lys Leu Thr Val Asp Lys Ser Arg
    290                 295                 300
Trp Gln Gln Gly Asn Val Phe Ser Cys Ser Val Met His Glu Ala Leu
305                 310                 315                 320
His Asn His Tyr Thr Gln Lys Ser Leu Ser Leu Ser Pro Gly Lys
                325                 330                 335

<210> 26
<211> 115
<212> PRT
<213> Homo sapiens

<220>
<223> human ActRIIB soluble (extracellular) processed polypeptide

<400> 26
Gly Arg Gly Glu Ala Glu Thr Arg Glu Cys Ile Tyr Tyr Asn Ala Asn
1               5                   10                  15
Trp Glu Leu Glu Arg Thr Asn Gln Ser Gly Leu Glu Arg Cys Glu Gly
            20                  25                  30
Glu Gln Asp Lys Arg Leu His Cys Tyr Ala Ser Trp Ala Asn Ser Ser
        35                  40                  45
Gly Thr Ile Glu Leu Val Lys Lys Gly Cys Trp Leu Asp Asp Phe Asn
    50                  55                  60
Cys Tyr Asp Arg Gln Glu Cys Val Ala Thr Glu Glu Asn Pro Gln Val
65                  70                  75                  80

Tyr Phe Cys Cys Cys Glu Gly Asn Phe Cys Asn Glu Arg Phe Thr His
                85                  90                  95
Leu Pro Glu Ala Gly Gly Pro Glu Val Thr Tyr Glu Pro Pro Pro Thr
            100                 105                 110
Ala Pro Thr
        115

<210> 27
<211> 100
<212> PRT
<213> Artificial Sequence

<220>
<223> human ActRIIB soluble (extracellular) processed polypeptide
      sequence with the C-terminal 15 amino acids deleted

<400> 27
Gly Arg Gly Glu Ala Glu Thr Arg Glu Cys Ile Tyr Tyr Asn Ala Asn
 1               5                  10                  15
Trp Glu Leu Glu Arg Thr Asn Gln Ser Gly Leu Glu Arg Cys Glu Gly
            20                  25                  30
Glu Gln Asp Lys Arg Leu His Cys Tyr Ala Ser Trp Ala Asn Ser Ser
        35                  40                  45
Gly Thr Ile Glu Leu Val Lys Lys Gly Cys Trp Leu Asp Asp Phe Asn
    50                  55                  60
Cys Tyr Asp Arg Gln Glu Cys Val Ala Thr Glu Glu Asn Pro Gln Val
65                  70                  75                  80
Tyr Phe Cys Cys Cys Glu Gly Asn Phe Cys Asn Glu Arg Phe Thr His
                85                  90                  95
Leu Pro Glu Ala
            100

<210> 28
<211> 512
<212> PRT
<213> Homo sapiens

<220>
<223> human ActRIIB precursor protein

<400> 28
Met Thr Ala Pro Trp Val Ala Leu Ala Leu Leu Trp Gly Ser Leu Trp
 1               5                  10                  15
Pro Gly Ser Gly Arg Gly Glu Ala Glu Thr Arg Glu Cys Ile Tyr Tyr
            20                  25                  30
Asn Ala Asn Trp Glu Leu Glu Arg Thr Asn Gln Ser Gly Leu Glu Arg
        35                  40                  45
Cys Glu Gly Glu Gln Asp Lys Arg Leu His Cys Tyr Ala Ser Trp Arg
    50                  55                  60
Asn Ser Ser Gly Thr Ile Glu Leu Val Lys Lys Gly Cys Trp Leu Asp
65                  70                  75                  80
Asp Phe Asn Cys Tyr Asp Arg Gln Glu Cys Val Ala Thr Glu Glu Asn
                85                  90                  95
Pro Gln Val Tyr Phe Cys Cys Cys Glu Gly Asn Phe Cys Asn Glu Arg
            100                 105                 110
Phe Thr His Leu Pro Glu Ala Gly Gly Pro Glu Val Thr Tyr Glu Pro
        115                 120                 125
Pro Pro Thr Ala Pro Thr Leu Leu Thr Val Leu Ala Tyr Ser Leu Leu
    130                 135                 140
Pro Ile Gly Gly Leu Ser Leu Ile Val Leu Leu Ala Phe Trp Met Tyr
145                 150                 155                 160

Arg His Arg Lys Pro Pro Tyr Gly His Val Asp Ile His Glu Asp Pro
                165                 170                 175
Gly Pro Pro Pro Pro Ser Pro Leu Val Gly Leu Lys Pro Leu Gln Leu
            180                 185                 190
Leu Glu Ile Lys Ala Arg Gly Arg Phe Gly Cys Val Trp Lys Ala Gln
        195                 200                 205
Leu Met Asn Asp Phe Val Ala Val Lys Ile Phe Pro Leu Gln Asp Lys
    210                 215                 220
Gln Ser Trp Gln Ser Glu Arg Glu Ile Phe Ser Thr Pro Gly Met Lys
225                 230                 235                 240
His Glu Asn Leu Leu Gln Phe Ile Ala Ala Glu Lys Arg Gly Ser Asn
                245                 250                 255
Leu Glu Val Glu Leu Trp Leu Ile Thr Ala Phe His Asp Lys Gly Ser
            260                 265                 270
Leu Thr Asp Tyr Leu Lys Gly Asn Ile Ile Thr Trp Asn Glu Leu Cys
        275                 280                 285
His Val Ala Glu Thr Met Ser Arg Gly Leu Ser Tyr Leu His Glu Asp
    290                 295                 300
Val Pro Trp Cys Arg Gly Glu Gly His Lys Pro Ser Ile Ala His Arg
305                 310                 315                 320
Asp Phe Lys Ser Lys Asn Val Leu Leu Lys Ser Asp Leu Thr Ala Val
                325                 330                 335
Leu Ala Asp Phe Gly Leu Ala Val Arg Phe Glu Pro Gly Lys Pro Pro
            340                 345                 350
Gly Asp Thr His Gly Gln Val Gly Thr Arg Arg Tyr Met Ala Pro Glu
        355                 360                 365
Val Leu Glu Gly Ala Ile Asn Phe Gln Arg Asp Ala Phe Leu Arg Ile
    370                 375                 380
Asp Met Tyr Ala Met Gly Leu Val Leu Trp Glu Leu Val Ser Arg Cys
385                 390                 395                 400
Lys Ala Ala Asp Gly Pro Val Asp Glu Tyr Met Leu Pro Phe Glu Glu
                405                 410                 415
Glu Ile Gly Gln His Pro Ser Leu Glu Glu Leu Gln Glu Val Val Val
            420                 425                 430
His Lys Lys Met Arg Pro Thr Ile Lys Asp His Trp Leu Lys His Pro
        435                 440                 445
Gly Leu Ala Gln Leu Cys Val Thr Ile Glu Glu Cys Trp Asp His Asp
    450                 455                 460
Ala Glu Ala Arg Leu Ser Ala Gly Cys Val Glu Glu Arg Val Ser Leu
465                 470                 475                 480
Ile Arg Arg Ser Val Asn Gly Thr Thr Ser Asp Cys Leu Val Ser Leu
                485                 490                 495
Val Thr Ser Val Thr Asn Val Asp Leu Pro Pro Lys Glu Ser Ser Ile
            500                 505                 510


<210> 29
<211> 116
<212> PRT
<213> Homo sapiens

<220>
<223> human ActRIIB soluble (extracellular) processed polypeptide

<400> 29
Ser Gly Arg Gly Glu Ala Glu Thr Arg Glu Cys Ile Tyr Tyr Asn Ala
1               5                   10                  15
Asn Trp Glu Leu Glu Arg Thr Asn Gln Ser Gly Leu Glu Arg Cys Glu
            20                  25                  30
Gly Glu Gln Asp Lys Arg Leu His Cys Tyr Ala Ser Trp Arg Asn Ser
        35                  40                  45
Ser Gly Thr Ile Glu Leu Val Lys Lys Gly Cys Trp Leu Asp Asp Phe
    50                  55                  60

Asn Cys Tyr Asp Arg Gln Glu Cys Val Ala Thr Glu Glu Asn Pro Gln
65                  70                  75                  80
Val Tyr Phe Cys Cys Cys Glu Gly Asn Phe Cys Asn Glu Arg Phe Thr
                85                  90                  95
His Leu Pro Glu Ala Gly Gly Pro Glu Val Thr Tyr Glu Pro Pro Pro
            100                 105                 110
Thr Ala Pro Thr
        115

<210> 30
<211> 101
<212> PRT
<213> Artificial Sequence

<220>
<223> human ActRIIB soluble (extracellular), processed polypeptide
      with the C-terminal 15 amino acids deleted

<400> 30
Ser Gly Arg Gly Glu Ala Glu Thr Arg Glu Cys Ile Tyr Tyr Asn Ala
 1               5                  10                  15
Asn Trp Glu Leu Glu Arg Thr Asn Gln Ser Gly Leu Glu Arg Cys Glu
            20                  25                  30
Gly Glu Gln Asp Lys Arg Leu His Cys Tyr Ala Ser Trp Arg Asn Ser
        35                  40                  45
Ser Gly Thr Ile Glu Leu Val Lys Lys Gly Cys Trp Leu Asp Asp Phe
    50                  55                  60
Asn Cys Tyr Asp Arg Gln Glu Cys Val Ala Thr Glu Glu Asn Pro Gln
65                  70                  75                  80
Val Tyr Phe Cys Cys Cys Glu Gly Asn Phe Cys Asn Glu Arg Phe Thr
                85                  90                  95
His Leu Pro Glu Ala
            100

<210> 31
<211> 115
<212> PRT
<213> Homo sapiens

<220>
<223> human ActRIIB soluble (extracellular), processed polypeptide

<400> 31
Gly Arg Gly Glu Ala Glu Thr Arg Glu Cys Ile Tyr Tyr Asn Ala Asn
 1               5                  10                  15
Trp Glu Leu Glu Arg Thr Asn Gln Ser Gly Leu Glu Arg Cys Glu Gly
            20                  25                  30
Glu Gln Asp Lys Arg Leu His Cys Tyr Ala Ser Trp Arg Asn Ser Ser
        35                  40                  45
Gly Thr Ile Glu Leu Val Lys Lys Gly Cys Trp Leu Asp Asp Phe Asn
    50                  55                  60
Cys Tyr Asp Arg Gln Glu Cys Val Ala Thr Glu Glu Asn Pro Gln Val
65                  70                  75                  80
Tyr Phe Cys Cys Cys Glu Gly Asn Phe Cys Asn Glu Arg Phe Thr His
                85                  90                  95
Leu Pro Glu Ala Gly Gly Pro Glu Val Thr Tyr Glu Pro Pro Pro Thr
            100                 105                 110
Ala Pro Thr
        115

<210> 32
<211> 100
<212> PRT
<213> Artificial Sequence

<220>
<223> human ActRIIB soluble (extracellular), processed polypeptide
      with the C-terminal 15 amino acids deleted

<400> 32
Gly Arg Gly Glu Ala Glu Thr Arg Glu Cys Ile Tyr Tyr Asn Ala Asn
1               5                   10                  15
Trp Glu Leu Glu Arg Thr Asn Gln Ser Gly Leu Glu Arg Cys Glu Gly
            20                  25                  30
Glu Gln Asp Lys Arg Leu His Cys Tyr Ala Ser Trp Arg Asn Ser Ser
        35                  40                  45
Gly Thr Ile Glu Leu Val Lys Lys Gly Cys Trp Leu Asp Asp Phe Asn
    50                  55                  60
Cys Tyr Asp Arg Gln Glu Cys Val Ala Thr Glu Glu Asn Pro Gln Val
65                  70                  75                  80
Tyr Phe Cys Cys Cys Glu Gly Asn Phe Cys Asn Glu Arg Phe Thr His
                85                  90                  95
Leu Pro Glu Ala
            100


<210> 33
<211> 107
<212> PRT
<213> Artificial Sequence

<220>
<223> human ActRIIB soluble (extracellular), processed polypeptide
      sequence with the N-terminal 6 amino acids of the EC domain
      deleted and the C-terminal 3 amino acids of the EC domain
      deleted and with an L79D mutation

<400> 33
Glu Thr Arg Glu Cys Ile Tyr Tyr Asn Ala Asn Trp Glu Leu Glu Arg
1               5                   10                  15
Thr Asn Gln Ser Gly Leu Glu Arg Cys Glu Gly Glu Gln Asp Lys Arg
            20                  25                  30
Leu His Cys Tyr Ala Ser Trp Ala Asn Ser Ser Gly Thr Ile Glu Leu
        35                  40                  45
Val Lys Lys Gly Cys Trp Asp Asp Asp Phe Asn Cys Tyr Asp Arg Gln
    50                  55                  60
Glu Cys Val Ala Thr Glu Glu Asn Pro Gln Val Tyr Phe Cys Cys Cys
65                  70                  75                  80
Glu Gly Asn Phe Cys Asn Glu Arg Phe Thr His Leu Pro Glu Ala Gly
                85                  90                  95
Gly Pro Glu Val Thr Tyr Glu Pro Pro Pro Thr
            100                 105


<210> 34
<211> 360
<212> PRT
<213> Artificial Sequence

<220>
<223> Unprocessed ActRIIB-Fc fusion protein with the N-terminal 6
      amino acids of the EC domain deleted and the C-terminal 3
      amino acids of the EC domain deleted and with an L79D

mutation and with TPA leader sequence

<400> 34
Met Asp Ala Met Lys Arg Gly Leu Cys Cys Val Leu Leu Leu Cys Gly
1               5                   10                  15
Ala Val Phe Val Ser Pro Gly Ala Ala Glu Thr Arg Glu Cys Ile Tyr
            20                  25                  30
Tyr Asn Ala Asn Trp Glu Leu Glu Arg Thr Asn Gln Ser Gly Leu Glu
        35                  40                  45
Arg Cys Glu Gly Glu Gln Asp Lys Arg Leu His Cys Tyr Ala Ser Trp
    50                  55                  60
Ala Asn Ser Ser Gly Thr Ile Glu Leu Val Lys Lys Gly Cys Trp Asp
65                  70                  75                  80
Asp Asp Phe Asn Cys Tyr Asp Arg Gln Glu Cys Val Ala Thr Glu Glu
                85                  90                  95
Asn Pro Gln Val Tyr Phe Cys Cys Cys Glu Gly Asn Phe Cys Asn Glu
            100                 105                 110
Arg Phe Thr His Leu Pro Glu Ala Gly Gly Pro Glu Val Thr Tyr Glu
        115                 120                 125
Pro Pro Pro Thr Gly Gly Gly Thr His Thr Cys Pro Pro Cys Pro Ala
    130                 135                 140
Pro Glu Leu Leu Gly Gly Pro Ser Val Phe Leu Phe Pro Pro Lys Pro
145                 150                 155                 160
Lys Asp Thr Leu Met Ile Ser Arg Thr Pro Glu Val Thr Cys Val Val
                165                 170                 175
Val Asp Val Ser His Glu Asp Pro Glu Val Lys Phe Asn Trp Tyr Val
            180                 185                 190
Asp Gly Val Glu Val His Asn Ala Lys Thr Lys Pro Arg Glu Glu Gln
        195                 200                 205
Tyr Asn Ser Thr Tyr Arg Val Val Ser Val Leu Thr Val Leu His Gln
    210                 215                 220
Asp Trp Leu Asn Gly Lys Glu Tyr Lys Cys Lys Val Ser Asn Lys Ala
225                 230                 235                 240
Leu Pro Ala Pro Ile Glu Lys Thr Ile Ser Lys Ala Lys Gly Gln Pro
                245                 250                 255
Arg Glu Pro Gln Val Tyr Thr Leu Pro Pro Ser Arg Glu Glu Met Thr
            260                 265                 270
Lys Asn Gln Val Ser Leu Thr Cys Leu Val Lys Gly Phe Tyr Pro Ser
        275                 280                 285
Asp Ile Ala Val Glu Trp Glu Ser Asn Gly Gln Pro Glu Asn Asn Tyr
    290                 295                 300
Lys Thr Thr Pro Pro Val Leu Asp Ser Asp Gly Ser Phe Phe Leu Tyr
305                 310                 315                 320
Ser Lys Leu Thr Val Asp Lys Ser Arg Trp Gln Gln Gly Asn Val Phe
                325                 330                 335
Ser Cys Ser Val Met His Glu Ala Leu His Asn His Tyr Thr Gln Lys
            340                 345                 350
Ser Leu Ser Leu Ser Pro Gly Lys
        355                 360


<210> 35
<211> 335
<212> PRT
<213> Artificial Sequence

<220>
<223> Processed ActRIIB-Fc fusion protein with the N-terminal 6
      amino acids of the EC domain deleted and the C-terminal 3
      amino acids of the EC domain deleted and with an L79D mutation

<400> 35
Glu Thr Arg Glu Cys Ile Tyr Tyr Asn Ala Asn Trp Glu Leu Glu Arg

1                   5                   10                  15
Thr Asn Gln Ser Gly Leu Glu Arg Cys Glu Gly Glu Gln Asp Lys Arg
                20                  25                  30
Leu His Cys Tyr Ala Ser Trp Ala Asn Ser Ser Gly Thr Ile Glu Leu
        35                  40                  45
Val Lys Lys Gly Cys Trp Asp Asp Asp Phe Asn Cys Tyr Asp Arg Gln
    50                  55                  60
Glu Cys Val Ala Thr Glu Glu Asn Pro Gln Val Tyr Phe Cys Cys Cys
65                  70                  75                  80
Glu Gly Asn Phe Cys Asn Glu Arg Phe Thr His Leu Pro Glu Ala Gly
                85                  90                  95
Gly Pro Glu Val Thr Tyr Glu Pro Pro Pro Thr Gly Gly Gly Thr His
                100                 105                 110
Thr Cys Pro Pro Cys Pro Ala Pro Glu Leu Leu Gly Gly Pro Ser Val
        115                 120                 125
Phe Leu Phe Pro Pro Lys Pro Lys Asp Thr Leu Met Ile Ser Arg Thr
    130                 135                 140
Pro Glu Val Thr Cys Val Val Val Asp Val Ser His Glu Asp Pro Glu
145                 150                 155                 160
Val Lys Phe Asn Trp Tyr Val Asp Gly Val Glu Val His Asn Ala Lys
                165                 170                 175
Thr Lys Pro Arg Glu Glu Gln Tyr Asn Ser Thr Tyr Arg Val Val Ser
                180                 185                 190
Val Leu Thr Val Leu His Gln Asp Trp Leu Asn Gly Lys Glu Tyr Lys
        195                 200                 205
Cys Lys Val Ser Asn Lys Ala Leu Pro Ala Pro Ile Glu Lys Thr Ile
    210                 215                 220
Ser Lys Ala Lys Gly Gln Pro Arg Glu Pro Gln Val Tyr Thr Leu Pro
225                 230                 235                 240
Pro Ser Arg Glu Glu Met Thr Lys Asn Gln Val Ser Leu Thr Cys Leu
                245                 250                 255
Val Lys Gly Phe Tyr Pro Ser Asp Ile Ala Val Glu Trp Glu Ser Asn
                260                 265                 270
Gly Gln Pro Glu Asn Asn Tyr Lys Thr Thr Pro Pro Val Leu Asp Ser
        275                 280                 285
Asp Gly Ser Phe Phe Leu Tyr Ser Lys Leu Thr Val Asp Lys Ser Arg
    290                 295                 300
Trp Gln Gln Gly Asn Val Phe Ser Cys Ser Val Met His Glu Ala Leu
305                 310                 315                 320
His Asn His Tyr Thr Gln Lys Ser Leu Ser Leu Ser Pro Gly Lys
                325                 330                 335

<210> 36
<211> 115
<212> PRT
<213> Artificial Sequence

<220>
<223> human ActRIIB soluble (extracellular), processed polypeptide
      sequence with L79D mutation

<400> 36
Gly Arg Gly Glu Ala Glu Thr Arg Glu Cys Ile Tyr Tyr Asn Ala Asn
1               5                   10                  15
Trp Glu Leu Glu Arg Thr Asn Gln Ser Gly Leu Glu Arg Cys Glu Gly
                20                  25                  30
Glu Gln Asp Lys Arg Leu His Cys Tyr Ala Ser Trp Arg Asn Ser Ser
        35                  40                  45
Gly Thr Ile Glu Leu Val Lys Lys Gly Cys Trp Asp Asp Asp Phe Asn
    50                  55                  60
Cys Tyr Asp Arg Gln Glu Cys Val Ala Thr Glu Glu Asn Pro Gln Val
65                  70                  75                  80

Tyr Phe Cys Cys Cys Glu Gly Asn Phe Cys Asn Glu Arg Phe Thr His
                85                  90                  95
Leu Pro Glu Ala Gly Gly Pro Glu Val Thr Tyr Glu Pro Pro Pro Thr
            100                 105                 110
Ala Pro Thr
        115

<210> 37
<211> 115
<212> PRT
<213> Artificial Sequence

<220>
<223> human ActRIIB soluble (extracellular), processed polypeptide
      sequence with L79D mutation

<400> 37
Gly Arg Gly Glu Ala Glu Thr Arg Glu Cys Ile Tyr Tyr Asn Ala Asn
 1               5                  10                  15
Trp Glu Leu Glu Arg Thr Asn Gln Ser Gly Leu Glu Arg Cys Glu Gly
            20                  25                  30
Glu Gln Asp Lys Arg Leu His Cys Tyr Ala Ser Trp Ala Asn Ser Ser
        35                  40                  45
Gly Thr Ile Glu Leu Val Lys Lys Gly Cys Trp Asp Asp Asp Phe Asn
    50                  55                  60
Cys Tyr Asp Arg Gln Glu Cys Val Ala Thr Glu Glu Asn Pro Gln Val
65                  70                  75                  80
Tyr Phe Cys Cys Cys Glu Gly Asn Phe Cys Asn Glu Arg Phe Thr His
                85                  90                  95
Leu Pro Glu Ala Gly Gly Pro Glu Val Thr Tyr Glu Pro Pro Pro Thr
            100                 105                 110
Ala Pro Thr
        115

<210> 38
<211> 343
<212> PRT
<213> Artificial Sequence

<220>
<223> human ActRIIB soluble (extracellular), processed polypeptide
      sequence with L79D mutation fused to an Fc domain with a
      GGG linker

<400> 38
Gly Arg Gly Glu Ala Glu Thr Arg Glu Cys Ile Tyr Tyr Asn Ala Asn
 1               5                  10                  15
Trp Glu Leu Glu Arg Thr Asn Gln Ser Gly Leu Glu Arg Cys Glu Gly
            20                  25                  30
Glu Gln Asp Lys Arg Leu His Cys Tyr Ala Ser Trp Arg Asn Ser Ser
        35                  40                  45
Gly Thr Ile Glu Leu Val Lys Lys Gly Cys Trp Asp Asp Asp Phe Asn
    50                  55                  60
Cys Tyr Asp Arg Gln Glu Cys Val Ala Thr Glu Glu Asn Pro Gln Val
65                  70                  75                  80
Tyr Phe Cys Cys Cys Glu Gly Asn Phe Cys Asn Glu Arg Phe Thr His
                85                  90                  95
Leu Pro Glu Ala Gly Gly Pro Glu Val Thr Tyr Glu Pro Pro Pro Thr
            100                 105                 110
Ala Pro Thr Gly Gly Gly Thr His Thr Cys Pro Pro Cys Pro Ala Pro
        115                 120                 125

Glu Leu Leu Gly Gly Pro Ser Val Phe Leu Phe Pro Pro Lys Pro Lys
    130                 135                 140
Asp Thr Leu Met Ile Ser Arg Thr Pro Glu Val Thr Cys Val Val Val
145                 150                 155                 160
Asp Val Ser His Glu Asp Pro Glu Val Lys Phe Asn Trp Tyr Val Asp
                165                 170                 175
Gly Val Glu Val His Asn Ala Lys Thr Lys Pro Arg Glu Glu Gln Tyr
            180                 185                 190
Asn Ser Thr Tyr Arg Val Val Ser Val Leu Thr Val Leu His Gln Asp
        195                 200                 205
Trp Leu Asn Gly Lys Glu Tyr Lys Cys Lys Val Ser Asn Lys Ala Leu
    210                 215                 220
Pro Ala Pro Ile Glu Lys Thr Ile Ser Lys Ala Lys Gly Gln Pro Arg
225                 230                 235                 240
Glu Pro Gln Val Tyr Thr Leu Pro Pro Ser Arg Glu Glu Met Thr Lys
                245                 250                 255
Asn Gln Val Ser Leu Thr Cys Leu Val Lys Gly Phe Tyr Pro Ser Asp
            260                 265                 270
Ile Ala Val Glu Trp Glu Ser Asn Gly Gln Pro Glu Asn Asn Tyr Lys
        275                 280                 285
Thr Thr Pro Pro Val Leu Asp Ser Asp Gly Ser Phe Phe Leu Tyr Ser
    290                 295                 300
Lys Leu Thr Val Asp Lys Ser Arg Trp Gln Gln Gly Asn Val Phe Ser
305                 310                 315                 320
Cys Ser Val Met His Glu Ala Leu His Asn His Tyr Thr Gln Lys Ser
                325                 330                 335
Leu Ser Leu Ser Pro Gly Lys
            340

<210> 39
<211> 343
<212> PRT
<213> Artificial Sequence

<220>
<223> human ActRIIB soluble (extracellular), processed polypeptide
      sequence with L79D mutation fused to an Fc domain

<400> 39
Gly Arg Gly Glu Ala Glu Thr Arg Glu Cys Ile Tyr Tyr Asn Ala Asn
 1               5                  10                  15
Trp Glu Leu Glu Arg Thr Asn Gln Ser Gly Leu Glu Arg Cys Glu Gly
            20                  25                  30
Glu Gln Asp Lys Arg Leu His Cys Tyr Ala Ser Trp Ala Asn Ser Ser
        35                  40                  45
Gly Thr Ile Glu Leu Val Lys Lys Gly Cys Trp Asp Asp Asp Phe Asn
    50                  55                  60
Cys Tyr Asp Arg Gln Glu Cys Val Ala Thr Glu Glu Asn Pro Gln Val
65                  70                  75                  80
Tyr Phe Cys Cys Cys Glu Gly Asn Phe Cys Asn Glu Arg Phe Thr His
                85                  90                  95
Leu Pro Glu Ala Gly Gly Pro Glu Val Thr Tyr Glu Pro Pro Pro Thr
            100                 105                 110
Ala Pro Thr Gly Gly Gly Thr His Thr Cys Pro Pro Cys Pro Ala Pro
        115                 120                 125
Glu Leu Leu Gly Gly Pro Ser Val Phe Leu Phe Pro Pro Lys Pro Lys
    130                 135                 140
Asp Thr Leu Met Ile Ser Arg Thr Pro Glu Val Thr Cys Val Val Val
145                 150                 155                 160
Asp Val Ser His Glu Asp Pro Glu Val Lys Phe Asn Trp Tyr Val Asp
                165                 170                 175
Gly Val Glu Val His Asn Ala Lys Thr Lys Pro Arg Glu Glu Gln Tyr

180                 185                 190
Asn Ser Thr Tyr Arg Val Val Ser Val Leu Thr Val Leu His Gln Asp
        195                 200                 205
Trp Leu Asn Gly Lys Glu Tyr Lys Cys Lys Val Ser Asn Lys Ala Leu
    210                 215                 220
Pro Ala Pro Ile Glu Lys Thr Ile Ser Lys Ala Lys Gly Gln Pro Arg
225                 230                 235                 240
Glu Pro Gln Val Tyr Thr Leu Pro Pro Ser Arg Glu Glu Met Thr Lys
                245                 250                 255
Asn Gln Val Ser Leu Thr Cys Leu Val Lys Gly Phe Tyr Pro Ser Asp
            260                 265                 270
Ile Ala Val Glu Trp Glu Ser Asn Gly Gln Pro Glu Asn Asn Tyr Lys
        275                 280                 285
Thr Thr Pro Pro Val Leu Asp Ser Asp Gly Ser Phe Phe Leu Tyr Ser
    290                 295                 300
Lys Leu Thr Val Asp Lys Ser Arg Trp Gln Gln Gly Asn Val Phe Ser
305                 310                 315                 320
Cys Ser Val Met His Glu Ala Leu His Asn His Tyr Thr Gln Lys Ser
                325                 330                 335
Leu Ser Leu Ser Pro Gly Lys
            340

<210> 40
<211> 368
<212> PRT
<213> Artificial Sequence

<220>
<223> human ActRIIB soluble (extracellular), processed polypeptide
      sequence with L79D mutation fused to an Fc domain and with
      TPA leader sequence

<400> 40
Met Asp Ala Met Lys Arg Gly Leu Cys Cys Val Leu Leu Leu Cys Gly
1               5                   10                  15
Ala Val Phe Val Ser Pro Gly Ala Ser Gly Arg Gly Glu Ala Glu Thr
            20                  25                  30
Arg Glu Cys Ile Tyr Tyr Asn Ala Asn Trp Glu Leu Glu Arg Thr Asn
        35                  40                  45
Gln Ser Gly Leu Glu Arg Cys Glu Gly Glu Gln Asp Lys Arg Leu His
    50                  55                  60
Cys Tyr Ala Ser Trp Arg Asn Ser Ser Gly Thr Ile Glu Leu Val Lys
65                  70                  75                  80
Lys Gly Cys Trp Asp Asp Asp Phe Asn Cys Tyr Asp Arg Gln Glu Cys
                85                  90                  95
Val Ala Thr Glu Glu Asn Pro Gln Val Tyr Phe Cys Cys Cys Glu Gly
            100                 105                 110
Asn Phe Cys Asn Glu Arg Phe Thr His Leu Pro Glu Ala Gly Gly Pro
        115                 120                 125
Glu Val Thr Tyr Glu Pro Pro Pro Thr Ala Pro Thr Gly Gly Gly Thr
    130                 135                 140
His Thr Cys Pro Pro Cys Pro Ala Pro Glu Leu Leu Gly Gly Pro Ser
145                 150                 155                 160
Val Phe Leu Phe Pro Pro Lys Pro Lys Asp Thr Leu Met Ile Ser Arg
                165                 170                 175
Thr Pro Glu Val Thr Cys Val Val Val Asp Val Ser His Glu Asp Pro
            180                 185                 190
Glu Val Lys Phe Asn Trp Tyr Val Asp Gly Val Glu Val His Asn Ala
        195                 200                 205
Lys Thr Lys Pro Arg Glu Glu Gln Tyr Asn Ser Thr Tyr Arg Val Val
    210                 215                 220
Ser Val Leu Thr Val Leu His Gln Asp Trp Leu Asn Gly Lys Glu Tyr

225                 230                 235                 240
Lys Cys Lys Val Ser Asn Lys Ala Leu Pro Ala Pro Ile Glu Lys Thr
                245                 250                 255
Ile Ser Lys Ala Lys Gly Gln Pro Arg Glu Pro Gln Val Tyr Thr Leu
            260                 265                 270
Pro Pro Ser Arg Glu Glu Met Thr Lys Asn Gln Val Ser Leu Thr Cys
        275                 280                 285
Leu Val Lys Gly Phe Tyr Pro Ser Asp Ile Ala Val Glu Trp Glu Ser
    290                 295                 300
Asn Gly Gln Pro Glu Asn Asn Tyr Lys Thr Thr Pro Pro Val Leu Asp
305                 310                 315                 320
Ser Asp Gly Ser Phe Phe Leu Tyr Ser Lys Leu Thr Val Asp Lys Ser
                325                 330                 335
Arg Trp Gln Gln Gly Asn Val Phe Ser Cys Ser Val Met His Glu Ala
            340                 345                 350
Leu His Asn His Tyr Thr Gln Lys Ser Leu Ser Leu Ser Pro Gly Lys
        355                 360                 365

<210> 41
<211> 368
<212> PRT
<213> Artificial Sequence

<220>
<223> human ActRIIB soluble (extracellular), processed polypeptide
      sequence with L79D mutation fused to an Fc domain and with
      TPA leader sequence

<400> 41
Met Asp Ala Met Lys Arg Gly Leu Cys Cys Val Leu Leu Leu Cys Gly
1               5                   10                  15
Ala Val Phe Val Ser Pro Gly Ala Ser Gly Arg Gly Glu Ala Glu Thr
            20                  25                  30
Arg Glu Cys Ile Tyr Tyr Asn Ala Asn Trp Glu Leu Glu Arg Thr Asn
        35                  40                  45
Gln Ser Gly Leu Glu Arg Cys Glu Gly Glu Gln Asp Lys Arg Leu His
    50                  55                  60
Cys Tyr Ala Ser Trp Ala Asn Ser Ser Gly Thr Ile Glu Leu Val Lys
65                  70                  75                  80
Lys Gly Cys Trp Asp Asp Asp Phe Asn Cys Tyr Asp Arg Gln Glu Cys
                85                  90                  95
Val Ala Thr Glu Glu Asn Pro Gln Val Tyr Phe Cys Cys Cys Glu Gly
            100                 105                 110
Asn Phe Cys Asn Glu Arg Phe Thr His Leu Pro Glu Ala Gly Gly Pro
        115                 120                 125
Glu Val Thr Tyr Glu Pro Pro Pro Thr Ala Pro Thr Gly Gly Gly Thr
    130                 135                 140
His Thr Cys Pro Pro Cys Pro Ala Pro Glu Leu Leu Gly Gly Pro Ser
145                 150                 155                 160
Val Phe Leu Phe Pro Pro Lys Pro Lys Asp Thr Leu Met Ile Ser Arg
                165                 170                 175
Thr Pro Glu Val Thr Cys Val Val Val Asp Val Ser His Glu Asp Pro
            180                 185                 190
Glu Val Lys Phe Asn Trp Tyr Val Asp Gly Val Glu Val His Asn Ala
        195                 200                 205
Lys Thr Lys Pro Arg Glu Glu Gln Tyr Asn Ser Thr Tyr Arg Val Val
    210                 215                 220
Ser Val Leu Thr Val Leu His Gln Asp Trp Leu Asn Gly Lys Glu Tyr
225                 230                 235                 240
Lys Cys Lys Val Ser Asn Lys Ala Leu Pro Ala Pro Ile Glu Lys Thr
                245                 250                 255
Ile Ser Lys Ala Lys Gly Gln Pro Arg Glu Pro Gln Val Tyr Thr Leu

260                 265                 270
Pro Pro Ser Arg Glu Glu Met Thr Lys Asn Gln Val Ser Leu Thr Cys
        275                 280                 285
Leu Val Lys Gly Phe Tyr Pro Ser Asp Ile Ala Val Glu Trp Glu Ser
    290                 295                 300
Asn Gly Gln Pro Glu Asn Asn Tyr Lys Thr Thr Pro Pro Val Leu Asp
305                 310                 315                 320
Ser Asp Gly Ser Phe Phe Leu Tyr Ser Lys Leu Thr Val Asp Lys Ser
                325                 330                 335
Arg Trp Gln Gln Gly Asn Val Phe Ser Cys Ser Val Met His Glu Ala
            340                 345                 350
Leu His Asn His Tyr Thr Gln Lys Ser Leu Ser Leu Ser Pro Gly Lys
        355                 360                 365

<210> 42
<211> 141
<212> PRT
<213> Artificial Sequence

<220>
<223> human ActRIIB soluble (extracellular), processed polypeptide
      having a variant C-terminal sequence

<400> 42
Gly Arg Gly Glu Ala Glu Thr Arg Glu Cys Ile Tyr Tyr Asn Ala Asn
1               5                   10                  15
Trp Glu Leu Glu Arg Thr Asn Gln Ser Gly Leu Glu Arg Cys Glu Gly
            20                  25                  30
Glu Gln Asp Lys Arg Leu His Cys Tyr Ala Ser Trp Arg Asn Ser Ser
        35                  40                  45
Gly Thr Ile Glu Leu Val Lys Lys Gly Cys Trp Leu Asp Asp Phe Asn
    50                  55                  60
Cys Tyr Asp Arg Gln Glu Cys Val Ala Thr Glu Glu Asn Pro Gln Val
65                  70                  75                  80
Tyr Phe Cys Cys Cys Glu Gly Asn Phe Cys Asn Glu Arg Phe Thr His
                85                  90                  95
Leu Pro Glu Ala Gly Gly Pro Glu Gly Pro Trp Ala Ser Thr Thr Ile
            100                 105                 110
Pro Ser Gly Gly Pro Glu Ala Thr Ala Ala Ala Gly Asp Gln Gly Ser
        115                 120                 125
Gly Ala Leu Trp Leu Cys Leu Glu Gly Pro Ala His Glu
    130                 135                 140

<210> 43
<211> 141
<212> PRT
<213> Artificial Sequence

<220>
<223> human ActRIIB soluble (extracellular), processed polypeptide
      having a variant C-terminal sequence having an L79D mutation

<400> 43
Gly Arg Gly Glu Ala Glu Thr Arg Glu Cys Ile Tyr Tyr Asn Ala Asn
1               5                   10                  15
Trp Glu Leu Glu Arg Thr Asn Gln Ser Gly Leu Glu Arg Cys Glu Gly
            20                  25                  30
Glu Gln Asp Lys Arg Leu His Cys Tyr Ala Ser Trp Arg Asn Ser Ser
        35                  40                  45
Gly Thr Ile Glu Leu Val Lys Lys Gly Cys Trp Asp Asp Asp Phe Asn
    50                  55                  60

Cys Tyr Asp Arg Gln Glu Cys Val Ala Thr Glu Glu Asn Pro Gln Val
65                  70                  75                  80
Tyr Phe Cys Cys Cys Glu Gly Asn Phe Cys Asn Glu Arg Phe Thr His
                85                  90                  95
Leu Pro Glu Ala Gly Gly Pro Glu Gly Pro Trp Ala Ser Thr Thr Ile
            100                 105                 110
Pro Ser Gly Gly Pro Glu Ala Thr Ala Ala Ala Gly Asp Gln Gly Ser
        115                 120                 125
Gly Ala Leu Trp Leu Cys Leu Glu Gly Pro Ala His Glu
    130                 135                 140

<210> 44
<211> 370
<212> PRT
<213> Artificial Sequence

<220>
<223> human ActRIIB soluble (extracellular), processed polypeptide
      sequence having a variant C-terminal sequence having an L79D
      mutation fused to an Fc domain with a TGGG linker

<400> 44
Gly Arg Gly Glu Ala Glu Thr Arg Glu Cys Ile Tyr Tyr Asn Ala Asn
 1               5                  10                  15
Trp Glu Leu Glu Arg Thr Asn Gln Ser Gly Leu Glu Arg Cys Glu Gly
            20                  25                  30
Glu Gln Asp Lys Arg Leu His Cys Tyr Ala Ser Trp Arg Asn Ser Ser
        35                  40                  45
Gly Thr Ile Glu Leu Val Lys Lys Gly Cys Trp Asp Asp Asp Phe Asn
    50                  55                  60
Cys Tyr Asp Arg Gln Glu Cys Val Ala Thr Glu Glu Asn Pro Gln Val
65                  70                  75                  80
Tyr Phe Cys Cys Cys Glu Gly Asn Phe Cys Asn Glu Arg Phe Thr His
                85                  90                  95
Leu Pro Glu Ala Gly Gly Pro Glu Gly Pro Trp Ala Ser Thr Thr Ile
            100                 105                 110
Pro Ser Gly Gly Pro Glu Ala Thr Ala Ala Ala Gly Asp Gln Gly Ser
        115                 120                 125
Gly Ala Leu Trp Leu Cys Leu Glu Gly Pro Ala His Glu Thr Gly Gly
    130                 135                 140
Gly Thr His Thr Cys Pro Pro Cys Pro Ala Pro Glu Leu Leu Gly Gly
145                 150                 155                 160
Pro Ser Val Phe Leu Phe Pro Pro Lys Pro Lys Asp Thr Leu Met Ile
                165                 170                 175
Ser Arg Thr Pro Glu Val Thr Cys Val Val Val Asp Val Ser His Glu
            180                 185                 190
Asp Pro Glu Val Lys Phe Asn Trp Tyr Val Asp Gly Val Glu Val His
        195                 200                 205
Asn Ala Lys Thr Lys Pro Arg Glu Glu Gln Tyr Asn Ser Thr Tyr Arg
    210                 215                 220
Val Val Ser Val Leu Thr Val Leu His Gln Asp Trp Leu Asn Gly Lys
225                 230                 235                 240
Glu Tyr Lys Cys Lys Val Ser Asn Lys Ala Leu Pro Ala Pro Ile Glu
                245                 250                 255
Lys Thr Ile Ser Lys Ala Lys Gly Gln Pro Arg Glu Pro Gln Val Tyr
            260                 265                 270
Thr Leu Pro Pro Ser Arg Glu Glu Met Thr Lys Asn Gln Val Ser Leu
        275                 280                 285
Thr Cys Leu Val Lys Gly Phe Tyr Pro Ser Asp Ile Ala Val Glu Trp
    290                 295                 300
Glu Ser Asn Gly Gln Pro Glu Asn Asn Tyr Lys Thr Thr Pro Pro Val
305                 310                 315                 320

Leu Asp Ser Asp Gly Ser Phe Phe Leu Tyr Ser Lys Leu Thr Val Asp
                325                 330                 335
Lys Ser Arg Trp Gln Gln Gly Asn Val Phe Ser Cys Ser Val Met His
            340                 345                 350
Glu Ala Leu His Asn His Tyr Thr Gln Lys Ser Leu Ser Leu Ser Pro
        355                 360                 365
Gly Lys
    370

<210> 45
<211> 1083
<212> DNA
<213> Artificial Sequence

<220>
<223> Unprocessed ActRIIB-Fc fusion protein with the N-terminal 6
      amino acids of the EC domain deleted and the C-terminal 3
      amino acids of the EC domain deleted and with an L79D
      mutation and with TPA leader sequence

<400> 45
atggatgcaa tgaagagagg gctctgctgt gtgctgctgc tgtgtggagc agtcttcgtt 60
tcgcccggcg ccgccgaaac ccgcgaatgt atttattaca atgctaattg ggaactcgaa 120
cggacgaacc aatccgggct cgaacggtgt gagggggaac aggataaacg cctccattgc 180
tatgcgtcgt ggaggaactc ctccgggacg attgaactgg tcaagaaagg gtgctgggac 240
gacgatttca attgttatga ccgccaggaa tgtgtcgcga ccgaagagaa tccgcaggtc 300
tatttctgtt gttgcgaggg gaatttctgt aatgaacggt ttacccacct ccccgaagcc 360
ggcgggcccg aggtgaccta tgaacccccg cccaccggtg gtggaactca cacatgccca 420
ccgtgcccag cacctgaact cctgggggga ccgtcagtct tcctcttccc cccaaaaccc 480
aaggacaccc tcatgatctc ccggacccct gaggtcacat gcgtggtggt ggacgtgagc 540
cacgaagacc ctgaggtcaa gttcaactgg tacgtggacg gcgtggaggt gcataatgcc 600
aagacaaagc cgcgggagga gcagtacaac agcacgtacc gtgtggtcag cgtcctcacc 660
gtcctgcacc aggactggct gaatggcaag gagtacaagt gcaaggtctc caacaaagcc 720
ctcccagccc ccatcgagaa aaccatctcc aaagccaaag ggcagccccg agaaccacag 780
gtgtacaccc tgcccccatc ccgggaggag atgaccaaga accaggtcag cctgacctgc 840
ctggtcaaag gcttctatcc cagcgacatc gccgtggagt gggagagcaa tgggcagccg 900
gagaacaact acaagaccac gcctcccgtg ctggactccg acggctcctt cttcctctat 960
agcaagctca ccgtggacaa gagcaggtgg cagcagggga acgtcttctc atgctccgtg 1020
atgcatgagg ctctgcacaa ccactacacg cagaagagcc tctccctgtc cccgggtaaa 1080
tga                                                               1083

<210> 46
<211> 344
<212> PRT
<213> Artificial Sequence

<220>
<223> fusion protein comprising a soluble extracellular domain of
      ActRIIB fused to an Fc domain

<400> 46
Ser Gly Arg Gly Glu Ala Glu Thr Arg Glu Cys Ile Tyr Tyr Asn Ala
1               5                   10                  15
Asn Trp Glu Leu Glu Arg Thr Asn Gln Ser Gly Leu Glu Arg Cys Glu
            20                  25                  30
Gly Glu Gln Asp Lys Arg Leu His Cys Tyr Ala Ser Trp Arg Asn Ser
        35                  40                  45
Ser Gly Thr Ile Glu Leu Val Lys Lys Gly Cys Trp Leu Asp Asp Phe
    50                  55                  60
Asn Cys Tyr Asp Arg Gln Glu Cys Val Ala Thr Glu Glu Asn Pro Gln
65                  70                  75                  80
Val Tyr Phe Cys Cys Cys Glu Gly Asn Phe Cys Asn Glu Arg Phe Thr

85                  90                  95
His Leu Pro Glu Ala Gly Gly Pro Glu Val Thr Tyr Glu Pro Pro Pro
            100                 105                 110
Thr Ala Pro Thr Gly Gly Gly Thr His Thr Cys Pro Pro Cys Pro Ala
        115                 120                 125
Pro Glu Leu Leu Gly Gly Pro Ser Val Phe Leu Phe Pro Pro Lys Pro
    130                 135                 140
Lys Asp Thr Leu Met Ile Ser Arg Thr Pro Glu Val Thr Cys Val Val
145                 150                 155                 160
Val Asp Val Ser His Glu Asp Pro Glu Val Lys Phe Asn Trp Tyr Val
                165                 170                 175
Asp Gly Val Glu Val His Asn Ala Lys Thr Lys Pro Arg Glu Glu Gln
            180                 185                 190
Tyr Asn Ser Thr Tyr Arg Val Val Ser Val Leu Thr Val Leu His Gln
        195                 200                 205
Asp Trp Leu Asn Gly Lys Glu Tyr Lys Cys Lys Val Ser Asn Lys Ala
    210                 215                 220
Leu Pro Val Pro Ile Glu Lys Thr Ile Ser Lys Ala Lys Gly Gln Pro
225                 230                 235                 240
Arg Glu Pro Gln Val Tyr Thr Leu Pro Pro Ser Arg Glu Glu Met Thr
                245                 250                 255
Lys Asn Gln Val Ser Leu Thr Cys Leu Val Lys Gly Phe Tyr Pro Ser
            260                 265                 270
Asp Ile Ala Val Glu Trp Glu Ser Asn Gly Gln Pro Glu Asn Asn Tyr
        275                 280                 285
Lys Thr Thr Pro Pro Val Leu Asp Ser Asp Gly Ser Phe Phe Leu Tyr
    290                 295                 300
Ser Lys Leu Thr Val Asp Lys Ser Arg Trp Gln Gln Gly Asn Val Phe
305                 310                 315                 320
Ser Cys Ser Val Met His Glu Ala Leu His Asn His Tyr Thr Gln Lys
                325                 330                 335
Ser Leu Ser Leu Ser Pro Gly Lys
            340

<210> 47
<211> 329
<212> PRT
<213> Artificial Sequence

<220>
<223> fusion protein comprising a soluble extracellular domain of
      ActRIIB (R64) with the C-terminal 15 amino acids deleted fused
      to an Fc domain

<400> 47
Ser Gly Arg Gly Glu Ala Glu Thr Arg Glu Cys Ile Tyr Tyr Asn Ala
1               5                   10                  15
Asn Trp Glu Leu Glu Arg Thr Asn Gln Ser Gly Leu Glu Arg Cys Glu
            20                  25                  30
Gly Glu Gln Asp Lys Arg Leu His Cys Tyr Ala Ser Trp Arg Asn Ser
        35                  40                  45
Ser Gly Thr Ile Glu Leu Val Lys Lys Gly Cys Trp Leu Asp Asp Phe
    50                  55                  60
Asn Cys Tyr Asp Arg Gln Glu Cys Val Ala Thr Glu Glu Asn Pro Gln
65                  70                  75                  80
Val Tyr Phe Cys Cys Cys Glu Gly Asn Phe Cys Asn Glu Arg Phe Thr
                85                  90                  95
His Leu Pro Glu Ala Gly Gly Gly Thr His Thr Cys Pro Pro Cys Pro
            100                 105                 110
Ala Pro Glu Leu Leu Gly Gly Pro Ser Val Phe Leu Phe Pro Pro Lys
        115                 120                 125
Pro Lys Asp Thr Leu Met Ile Ser Arg Thr Pro Glu Val Thr Cys Val

130                 135                 140
Val Val Asp Val Ser His Glu Asp Pro Glu Val Lys Phe Asn Trp Tyr
145                 150                 155                 160
Val Asp Gly Val Glu Val His Asn Ala Lys Thr Lys Pro Arg Glu Glu
                165                 170                 175
Gln Tyr Asn Ser Thr Tyr Arg Val Val Ser Val Leu Thr Val Leu His
            180                 185                 190
Gln Asp Trp Leu Asn Gly Lys Glu Tyr Lys Cys Lys Val Ser Asn Lys
        195                 200                 205
Ala Leu Pro Val Pro Ile Glu Lys Thr Ile Ser Lys Ala Lys Gly Gln
    210                 215                 220
Pro Arg Glu Pro Gln Val Tyr Thr Leu Pro Pro Ser Arg Glu Glu Met
225                 230                 235                 240
Thr Lys Asn Gln Val Ser Leu Thr Cys Leu Val Lys Gly Phe Tyr Pro
                245                 250                 255
Ser Asp Ile Ala Val Glu Trp Glu Ser Asn Gly Gln Pro Glu Asn Asn
            260                 265                 270
Tyr Lys Thr Thr Pro Pro Val Leu Asp Ser Asp Gly Ser Phe Phe Leu
        275                 280                 285
Tyr Ser Lys Leu Thr Val Asp Lys Ser Arg Trp Gln Gln Gly Asn Val
    290                 295                 300
Phe Ser Cys Ser Val Met His Glu Ala Leu His Asn His Tyr Thr Gln
305                 310                 315                 320
Lys Ser Leu Ser Leu Ser Pro Gly Lys
                325


<210> 48
<211> 407
<212> PRT
<213> Unknown

<220>
<223> full-length, unprocessed precursor protein GDF11
      (GDF11 preproprotein)

<400> 48
Met Val Leu Ala Ala Pro Leu Leu Leu Gly Phe Leu Leu Leu Ala Leu
1               5                   10                  15
Glu Leu Arg Pro Arg Gly Glu Ala Ala Glu Gly Pro Ala Ala Ala Ala
            20                  25                  30
Ala Ala Ala Ala Ala Ala Ala Ala Ala Gly Val Gly Gly Glu Arg Ser
        35                  40                  45
Ser Arg Pro Ala Pro Ser Val Ala Pro Glu Pro Asp Gly Cys Pro Val
    50                  55                  60
Cys Val Trp Arg Gln His Ser Arg Glu Leu Arg Leu Glu Ser Ile Lys
65                  70                  75                  80
Ser Gln Ile Leu Ser Lys Leu Arg Leu Lys Glu Ala Pro Asn Ile Ser
                85                  90                  95
Arg Glu Val Val Lys Gln Leu Leu Pro Lys Ala Pro Pro Leu Gln Gln
            100                 105                 110
Ile Leu Asp Leu His Asp Phe Gln Gly Asp Ala Leu Gln Pro Glu Asp
        115                 120                 125
Phe Leu Glu Glu Asp Glu Tyr His Ala Thr Thr Glu Thr Val Ile Ser
    130                 135                 140
Met Ala Gln Glu Thr Asp Pro Ala Val Gln Thr Asp Gly Ser Pro Leu
145                 150                 155                 160
Cys Cys His Phe His Phe Ser Pro Lys Val Met Phe Thr Lys Val Leu
                165                 170                 175
Lys Ala Gln Leu Trp Val Tyr Leu Arg Pro Val Pro Arg Pro Ala Thr
            180                 185                 190
Val Tyr Leu Gln Ile Leu Arg Leu Lys Pro Leu Thr Gly Glu Gly Thr
        195                 200                 205

Ala Gly Gly Gly Gly Gly Gly Arg Arg His Ile Arg Ile Arg Ser Leu
    210                 215                 220
Lys Ile Glu Leu His Ser Arg Ser Gly His Trp Gln Ser Ile Asp Phe
225                 230                 235                 240
Lys Gln Val Leu His Ser Trp Phe Arg Gln Pro Gln Ser Asn Trp Gly
                245                 250                 255
Ile Glu Ile Asn Ala Phe Asp Pro Ser Gly Thr Asp Leu Ala Val Thr
            260                 265                 270
Ser Leu Gly Pro Gly Ala Glu Gly Leu His Pro Phe Met Glu Leu Arg
        275                 280                 285
Val Leu Glu Asn Thr Lys Arg Ser Arg Arg Asn Leu Gly Leu Asp Cys
    290                 295                 300
Asp Glu His Ser Ser Glu Ser Arg Cys Cys Arg Tyr Pro Leu Thr Val
305                 310                 315                 320
Asp Phe Glu Ala Phe Gly Trp Asp Trp Ile Ile Ala Pro Lys Arg Tyr
                325                 330                 335
Lys Ala Asn Tyr Cys Ser Gly Gln Cys Glu Tyr Met Phe Met Gln Lys
            340                 345                 350
Tyr Pro His Thr His Leu Val Gln Gln Ala Asn Pro Arg Gly Ser Ala
        355                 360                 365
Gly Pro Cys Cys Thr Pro Thr Lys Met Ser Pro Ile Asn Met Leu Tyr
    370                 375                 380
Phe Asn Asp Lys Gln Gln Ile Ile Tyr Gly Lys Ile Pro Gly Met Val
385                 390                 395                 400
Val Asp Arg Cys Gly Cys Ser
                405

<210> 49
<211> 1221
<212> DNA
<213> Unknown

<220>
<223> full-length, unprocessed precursor protein GDF11
      (GDF11 preproprotein)

<400> 49
   atggtgctcg cggccccgct gctgctgggc ttcctgctcc tcgccctgga gctgcggccc 60
   cggggggagg cggccgaggg ccccgcggcg gcggcggcgg cggcggcggc ggcggcagcg 120
   gcgggggtcg ggggggagcg ctccagccgg ccagccccgt ccgtggcgcc cgagccggac 180
   ggctgccccg tgtgcgtttg gcggcagcac agccgcgagc tgcgcctaga gagcatcaag 240
   tcgcagatct tgagcaaact gcggctcaag gaggcgccca acatcagccg cgaggtggtg 300
   aagcagctgc tgcccaaggc gccgccgctg cagcagatcc tggacctaca cgacttccag 360
   ggcgacgcgc tgcagcccga ggacttcctg gaggaggacg agtaccacgc caccaccgag 420
   accgtcatta gcatggccca ggagacggac ccagcagtac agacagatgg cagccctctc 480
   tgctgccatt ttcacttcag ccccaaggtg atgttcacaa aggtactgaa ggcccagctg 540
   tgggtgtacc tacggcctgt accccgccca gccacagtct acctgcagat cttgcgacta 600
   aaacccctaa ctggggaagg gaccgcaggg ggagggggcg gaggccggcg tcacatccgt 660
   atccgctcac tgaagattga gctgcactca cgctcaggcc attggcagag catcgacttc 720
   aagcaagtgc tacacagctg gttccgccag ccacagagca actggggcat cgagatcaac 780
   gcctttgatc ccagtggcac agacctggct gtcacctccc tggggccggg agccgagggg 840
   ctgcatccat tcatggagct tcgagtccta gagaacacaa aacgttcccg gcggaacctg 900
   ggtctggact gcgacgagca ctcaagcgag tcccgctgct gccgatatcc cctcacagtg 960
   gactttgagg ctttcggctg ggactggatc atcgcaccta agcgctacaa ggccaactac 1020
   tgctccggcc agtgcgagta catgttcatg caaaaatatc cgcataccca tttggtgcag 1080
   caggccaatc caagaggctc tgctgggccc tgttgtaccc ccaccaagat gtccccaatc 1140
   aacatgctct acttcaatga caagcagcag attatctacg gcaagatccc tggcatggtg 1200
   gtggatcgct gtggctgctc t                                           1221

<210> 50
<211> 274
<212> PRT

<213> Homo sapiens

<220>
<223> GDF11 propeptide of human GDF11 protein

<400> 50
Ala Glu Gly Pro Ala Ala Ala Ala Ala Ala Ala Ala Ala Ala Ala Ala
1               5                   10                  15
Ala Gly Val Gly Gly Glu Arg Ser Ser Arg Pro Ala Pro Ser Val Ala
            20                  25                  30
Pro Glu Pro Asp Gly Cys Pro Val Cys Val Trp Arg Gln His Ser Arg
        35                  40                  45
Glu Leu Arg Leu Glu Ser Ile Lys Ser Gln Ile Leu Ser Lys Leu Arg
    50                  55                  60
Leu Lys Glu Ala Pro Asn Ile Ser Arg Glu Val Val Lys Gln Leu Leu
65                  70                  75                  80
Pro Lys Ala Pro Pro Leu Gln Gln Ile Leu Asp Leu His Asp Phe Gln
                85                  90                  95
Gly Asp Ala Leu Gln Pro Glu Asp Phe Leu Glu Glu Asp Glu Tyr His
            100                 105                 110
Ala Thr Thr Glu Thr Val Ile Ser Met Ala Gln Glu Thr Asp Pro Ala
        115                 120                 125
Val Gln Thr Asp Gly Ser Pro Leu Cys Cys His Phe His Phe Ser Pro
    130                 135                 140
Lys Val Met Phe Thr Lys Val Leu Lys Ala Gln Leu Trp Val Tyr Leu
145                 150                 155                 160
Arg Pro Val Pro Arg Pro Ala Thr Val Tyr Leu Gln Ile Leu Arg Leu
                165                 170                 175
Lys Pro Leu Thr Gly Glu Gly Thr Ala Gly Gly Gly Gly Gly Gly Arg
            180                 185                 190
Arg His Ile Arg Ile Arg Ser Leu Lys Ile Glu Leu His Ser Arg Ser
        195                 200                 205
Gly His Trp Gln Ser Ile Asp Phe Lys Gln Val Leu His Ser Trp Phe
    210                 215                 220
Arg Gln Pro Gln Ser Asn Trp Gly Ile Glu Ile Asn Ala Phe Asp Pro
225                 230                 235                 240
Ser Gly Thr Asp Leu Ala Val Thr Ser Leu Gly Pro Gly Ala Glu Gly
                245                 250                 255
Leu His Pro Phe Met Glu Leu Arg Val Leu Glu Asn Thr Lys Arg Ser
            260                 265                 270
Arg Arg

<210> 51
<211> 822
<212> DNA
<213> Homo sapiens

<220>
<223> GDF11 propeptide of human GDF11 protein

<400> 51
gccgagggcc ccgcggcggc ggcggcggcg gcggcggcgg cggcagcggc gggggtcggg 60
ggggagcgct ccagccggcc agccccgtcc gtggcgcccg agccggacgg ctgccccgtg 120
tgcgtttggc ggcagcacag ccgcgagctg cgcctagaga gcatcaagtc gcagatcttg 180
agcaaactgc ggctcaagga ggcgcccaac atcagccgcg aggtggtgaa gcagctgctg 240
cccaaggcgc cgccgctgca gcagatcctg gacctacacg acttccaggg cgacgcgctg 300
cagcccgagg acttcctgga ggaggacgag taccacgcca ccaccgagac cgtcattagc 360
atggcccagg agacggaccc agcagtacag acagatggca gccctctctg ctgccatttt 420
cacttcagcc ccaaggtgat gttcacaaag gtactgaagg cccagctgtg ggtgtaccta 480
cggcctgtac cccgcccagc cacagtctac ctgcagatct tgcgactaaa acccctaact 540
ggggaaggga ccgcaggggg agggggcgga ggccggcgtc acatccgtat ccgctcactg 600

aagattgagc tgcactcacg ctcaggccat tggcagagca tcgacttcaa gcaagtgcta 660
cacagctggt tccgccagcc acagagcaac tggggcatcg agatcaacgc ctttgatccc 720
agtggcacag acctggctgt cacctccctg gggccgggag ccgaggggct gcatccattc 780
atggagcttc gagtcctaga gaacacaaaa cgttcccggc gg 822

<210> 52
<211> 109
<212> PRT
<213> Homo sapiens

<220>
<223> Mature human GDF11 protein

<400> 52
Asn Leu Gly Leu Asp Cys Asp Glu His Ser Ser Glu Ser Arg Cys Cys
1               5                   10                  15
Arg Tyr Pro Leu Thr Val Asp Phe Glu Ala Phe Gly Trp Asp Trp Ile
            20                  25                  30
Ile Ala Pro Lys Arg Tyr Lys Ala Asn Tyr Cys Ser Gly Gln Cys Glu
        35                  40                  45
Tyr Met Phe Met Gln Lys Tyr Pro His Thr His Leu Val Gln Gln Ala
    50                  55                  60
Asn Pro Arg Gly Ser Ala Gly Pro Cys Cys Thr Pro Thr Lys Met Ser
65                  70                  75                  80
Pro Ile Asn Met Leu Tyr Phe Asn Asp Lys Gln Gln Ile Ile Tyr Gly
                85                  90                  95
Lys Ile Pro Gly Met Val Val Asp Arg Cys Gly Cys Ser
            100                 105

<210> 53
<211> 327
<212> DNA
<213> Homo sapiens

<220>
<223> GDF11 propeptide of human GDF11 protein

<400> 53
aacctgggtc tggactgcga cgagcactca agcgagtccc gctgctgccg atatcccctc 60
acagtggact ttgaggcttt cggctgggac tggatcatcg cacctaagcg ctacaaggcc 120
aactactgct ccggccagtg cgagtacatg ttcatgcaaa aatatccgca tacccatttg 180
gtgcagcagg ccaatccaag aggctctgct gggccctgtt gtacccccac caagatgtcc 240
ccaatcaaca tgctctactt caatgacaag cagcagatta tctacggcaa gatccctggc 300
atggtggtgg atcgctgtgg ctgctct 327